(19) Europäisches Patentamt
European Patent Office
Office européen des brevets

(11) Veröffentlichungsnummer: **0 216 127**
**B1**

(12) # EUROPÄISCHE PATENTSCHRIFT

(45) Veröffentlichungstag der Patentschrift: **26.09.90**

(51) Int. Cl.[5]: **C07D 309/30**, C07D 309/10, A61K 31/365, C07F 7/18

(21) Anmeldenummer: **86111383.5**

(22) Anmeldetag: **18.08.86**

(54) **6-Phenoxymethyl-4-hydroxytetrahydropyran-2-one, Verfahren zu ihrer Herstellung, ihre Verwendung als Arzneimittel, pharmazeutische Präparate und Zwischenprodukte.**

(30) Priorität: **29.08.85 DE 3530798**
**07.12.85 DE 3543336**

(43) Veröffentlichungstag der Anmeldung:
**01.04.87 Patentblatt 87/14**

(45) Bekanntmachung des Hinweises auf die Patenterteilung:
**26.09.90 Patentblatt 90/39**

(84) Benannte Vertragsstaaten:
**AT BE CH DE FR GB IT LI LU NL SE**

(56) Entgegenhaltungen:
**US-A- 4 255 444**

**TETRAHEDRON LETTERS, Band 23, Nr. 42, 1982, Seiten 4305-4308, Pergamon Press Ltd, Oxford, GB; Y.-L. YANG et al.: "Mevinic acids and analogues: preparation of a key chiral intermediate"**
**J. Med. Chem., 1985, Vol. 28, Nr. 3, Seiten 347-358.**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80(DE)**

(72) Erfinder: **Wess, Günther, Dr., Hainstrasse 35, D-6455 Erlensee(DE)**
Erfinder: **Granzer, Ernold, Dr. Dr., Falkensteiner Strasse 24, D-6233 Kelkheim (Taunus)(DE)**
Erfinder: **Beck, Gerhard, Dr., Gustav-Freytag-Strasse 24, D-6000 Frankfurt am Main 1(DE)**
Erfinder: **Lau, Hans-Hermann, Dr., Kastanienhain 29, D-6232 Bad Soden am Taunus(DE)**

**EP 0 216 127 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Das Enzym 3-Hydroxy-3-methylglutaryl-Coenzym-A-Reduktase (HMG–CoA-Reduktase) katalysiert die Bildung von Mevalonsäure aus 3-Hydroxy-3-methylglutaryl-Coenzym A (HMG–CoA). Diese Reaktion spielt eine zentrale Rolle bei der Biosynthese des Cholesterins. Derivate der 3-Hydroxy-3-methylglutarsäure (HMG) und der Mevalonsäure sind als Hemmer der Cholesterinbiosynthese beschrieben worden (M.R. Boots et al., J. Pharm. Sci. 69, 306 (1980), F.M. Singer et al., Proc. Soc. Exper. Biol. Med. 102, 370 (1959), H. Feres, Tetrahedron Lett. 24, 3769 (1983)). 3-Hydroxy-3-methyl-glutarsäure selbst zeigt an der Ratte und im Humanversuch signifikante cholesterinsenkende Wirkung (Z. Beg, Experientia 23, 380 (1967), ibid 24, 15 (1968), P.J. Lupien et al., Lancet 1978, 1, 283). Die in der US-Patentschrift 4 255 444 beschriebenen 4-Hydroxy-tetrahydropyran-2-on-Derivate, die in 6-Stellung einen über eine Alkylenbrücke gebundenen Phenoxy-Rest aufweisen, der gegebenenfalls mit Chlor substituiert ist, weisen eine schwache Hemmwirkung auf die HMG-CoA-Reduktase auf. Stokker et al. beschreiben im Journal of Medicinal Chemistry, Vol. 28, Seiten 347–358 (1985), daß ein Wirkungsabfall bzw. Wirkungsverlust dann auftritt, wenn der Hydroxytetrahydropyran-2-on-Ring über eine Oxymethylengruppe mit dem aromatischen Rest, wie z.B. einem gegebenenfalls mit Chlor disubstituierten Phenylrest, verbunden ist. Es wurde nun gefunden, daß 6-Phenoxymethyl-4-hydroxytetrahydropyran-2-one der allgemeinen Formel I bzw. die ihnen entsprechenden Dihydroxycarbonsäuren, deren Salze und Ester Hemmstoffe der HMG-CoA-Reduktase sind. Die Erfindung betrifft daher neue 6-Phenoxymethyl-4-hydroxytetrahydropyran-2-one der allgemeinen Formel I

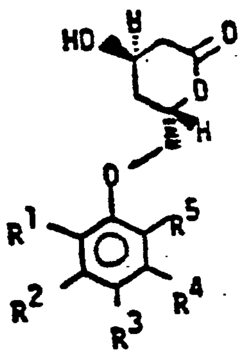

I

worin
$R^1$ und $R^5$ gleich oder verschieden sind und a) Halogen, b) Cycloalkyl mit 4–8 C-Atomen oder einen Phenylrest, der im Kern 1- bis 3-fach substituiert sein kann mit Halogen, Trifluormethyl und/oder Alkyl oder Alkoxy mit je 1–4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1–3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,
β) Halogen, Hydroxy, Cycloalkyl mit 3–7 C-Atomen, unsubstituierten Phenyl-, α- oder β-Thienylresten, oder Phenyl-, α- oder β-Thienylresten, welche ihrerseits im Kern 1- bis 3-fach substituiert sind mit Halogen, Trifluormethyl und/oder Alkyl oder Alkoxy mit 1–4 C-Atomen,
γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,
δ) der Gruppe

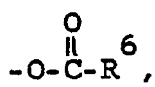

wobei $R^6$ bedeutet: einen geradkettigen oder verzweigten Alkyl- oder Alkenylrest mit bis zu 8 Kohlenstoffatomen, oder einen Cycloalkyl oder Cycloalkenylrest mit jeweils 3–8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1–4 C-Atomen, oder einen 3-Pyridylrest,

$R^2$ und $R^4$ Wasserstoff bedeuten, und
$R^3$ Alkyl oder Alkenyl mit bis zu 4 C-Atomen,

Halogen oder Alkoxy mit 1–4 C-Atomen ist, sowie die entsprechenden offenkettigen Dihydroxycarbonsäuren, deren pharmakologisch verträgliche Salze mit Basen und deren pharmakologisch verträglichen Ester.

Die Substituenten haben bevorzugt folgende Bedeutungen:

$R^1$ und $R^5$, beide gleich oder verschieden:

a) Halogen,

b) Cycloalkyl mit 5 bis 6 C-Atomen, Phenyl, das im Kern 1–3-fach substituiert sein kann mit Halogen, Trifluormethyl und/oder Alkyl oder Alkoxy mit je 1–4 C-Atomen, oder

c) 1. geradkettiges oder verzweigtes Alkyl oder Alkenyl mit bis zu 12 Kohlenstoffatomen, wobei der Alkyl- bzw. Alkenylrest seinerseits 1–2-fach substituiert sein kann mit Phenylresten, die ihrerseits im Kern 1- bis 3-fach substituiert sein können mit Halogen, Trifluormethyl und/oder Alkyl oder Alkoxy mit 1–4 C-Atomen,

2. geradkettiges, mit

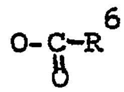

substituiertes Alkyl der Formel

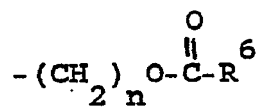

worin n = 1 bis 3 und $R^6$ einen geradkettigen oder verzweigten Alkyl- oder Alkenylrest mit bis zu 8 C-Atomen, einen Cycloalkylrest mit 5 bis 6 C-Atomen oder einen Phenylrest, welcher seinerseits im Kern 1–3-fach substituiert sein kann mit Halogen, Trifluormethyl und/oder Alkyl oder Alkoxy mit 1 bis 4 C-Atomen, bedeutet,

3. geradkettiges mit $OR^7$ substituiertes Alkyl der Formel

$$-(CH_2)_nOR^7,$$

worin n = 1 bis 3 und $R^7$ Wasserstoff oder einen geradkettigen oder verzweigten Alkyl- oder Alkenylrest mit bis zu 8 C-Atomen, einen Cycloalkylrest mit 5 bis 6 C-Atomen, oder einen Phenylrest oder Benzylrest, welche ihrerseits im aromatischen Kern 1–3-fach substituiert sein können mit Halogen, Trifluormethyl und/oder Alkyl oder Alkoxy mit 1 bis 4 C-Atomen, bedeutet,

$R^2$ und $R^4$ Wasserstoff,

$R^3$ Methyl, Ethyl, Propyl, 1-Propenyl, Allyl, Fluor oder Chlor.

Besonders bevorzugt bedeuten die Substituenten:

$R^1$ und $R^5$, wobei $R^1$ und $R^5$ gleich oder verschieden sind:

1) Methyl, Ethyl, Propyl, Allyl, 1-Propenyl, Isopropenyl, Isopropyl, Cyclopentyl, Cyclohexyl, oder

2) die Gruppe

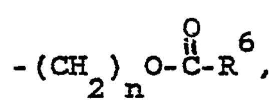

mit n = 1–3, wobei $R^6$ bedeutet: Methyl, Ethyl, Propyl, i-Propyl, n-Butyl, i-Butyl, t-Butyl, Phenyl oder im Kern 1- bis 3-fach substituiertes Phenyl mit Halogen, Methyl oder Methoxy, oder

3) die Gruppe $-(CH_2)_nOR^7$, mit n = 1 bis 3, wobei $R^7$ bedeutet: Wasserstoff, Methyl, geradkettiges oder verzweigtes Alkyl oder Alkenyl mit 3–5 Kohlenstoffatomen, Cyclopentyl, Cyclohexyl; Phenyl oder Benzyl, wobei die aromatischen Kerne 1–3-fach substituiert sein können mit Fluor, Chlor, Methyl, Methoxy, oder

4) eine Alkylgruppe der Formel

$$-(CH_2)_nCHR^8R^9$$

worin n = 0 bis 2 ist und $R^8$ und $R^9$ gleich oder verschieden sind und Wasserstoff, Methyl, Ethyl, Propyl, Allyl, i-Propyl, n-Butyl, i-Butyl, t-Butyl, Cyclohexyl, Cyclopentyl, Benzyl oder Phenyl, wobei die aromatischen Kerne 1- bis 3-fach substituiert sein können mit Fluor, Chlor, Methyl oder Methoxy, bedeuten.

$R^2$ und $R^4$ Wasserstoff
$R^3$ Methyl, Chlor, Fluor.

Als pharmakologisch verträgliche Salze der Dihydroxycarbonsäuren, welche die Formel VII

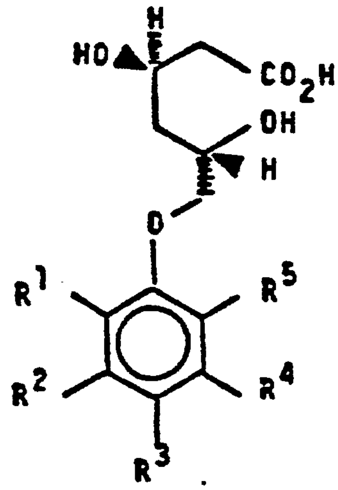

VII

worin $R^1$ bis $R^5$ die zu Formel I angegebenen Bedeutungen haben, seien Alkalimetallsalze oder Ammoniumsalze genannt; pharmazeutisch annehmbare Ester sind z.B. Alkylester mit 1 bis 4 C-Atomen, Phenylester, Benzylester oder auch 2,3-Dihydroxypropylester.

Die Erfindung betrifft die Enantiomeren mit der in der allgemeinen Formel I angegebenen absoluten Konfiguration 4R, 6S, wobei die offenkettigen Dihydroxycarbonsäuren VII, deren Ester und Salze die in der allgemeinen Formel VII angegebene absolute Konfiguration 3R, 5S besitzen.

Die Erfindung betrifft ferner ein Verfahren zur Herstellung der Verbindungen der allgemeinen Formel I, das dadurch gekennzeichnet ist, daß man:

a) entsprechend substituierte Phenolen der Formel II,

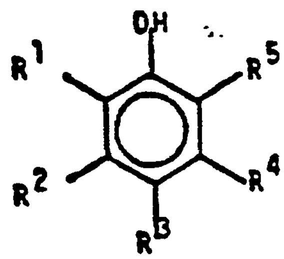

II

worin $R^1$, $R^2$, $R^3$, $R^4$ und $R^5$ die angegebenen Bedeutungen haben, mit dem chiralen Jodid der Formel III

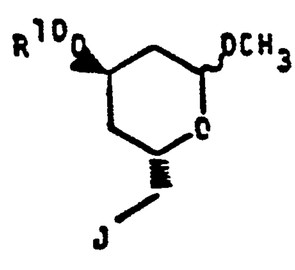

III

worin $R^{10}$ eine gegen Basen und schwache Säuren stabile Schutzgruppe bedeutet, z.B. die t-Butyldiphenylsilyl-Gruppe (t–$BuPh_2Si$),
in die Ether der Formel IV

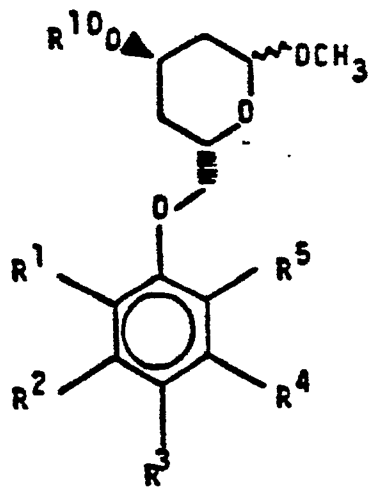

IV

worin $R^1$ bis $R^5$ die zu Formel I und $R^{10}$ die zu Formel III angegebenen Bedeutungen haben, überführt,

b) die Ether der Formel IV zu den entsprechenden Halbacetalen der Formel V

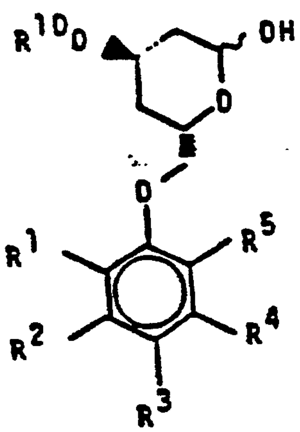

V

worin $R^1$ bis $R^5$ und $R^{10}$ die zu Formel I bzw. III angegebenen Bedeutungen haben, hydrolysiert,

c) die Halbacetale der Formel V zu den entsprechenden Lactonen der Formel VI

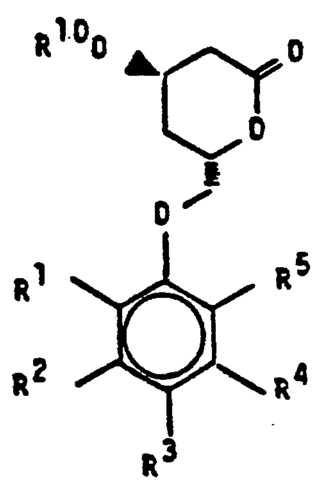

VI

worin $R^1$ bis $R^5$ die zu Formel I und $R^{10}$ die zu Formel III angegebenen Bedeutungen haben, oxidiert und

d) die geschützten Hydroxylaktone der Formel VI in die 6-Phenoxymethyl-4-hydroxytetrahydropyran-2-one der Formel I

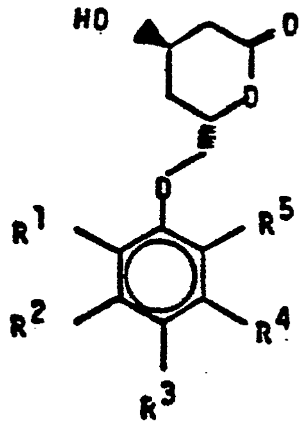

I

überführt, gegebenenfalls die erhaltenen Verbindungen der Formel I in die entsprechenden offenkettigen Dihydroxycarbonsäuren, deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

Die Veretherung von Verbindungen der Formel II mit Verbindungen der Formel III zu Verbindungen der allgemeinen Formel IV erfolgt z.B. in einem Lösungsmittel in Anwesenheit einer Base, vorzugsweise in Dimethylsulfoxid unter Verwendung von Kaliumcarbonat bei 50–80°C.

Die Hydrolyse der Acetale IV zu den Halbacetalen V wird zweckmäßig unter wäßrig sauren Bedingungen vorgenommen, vorzugsweise mit Essigsäure in Tetrahydrofuran/Wasser-Gemischen, oder auch mit Salzsäure in THF/Wasser oder Trifluoressigsäure bei 0–80°C.

Die Überführung der Halbacetale V in die Laktone VI wird mit Hilfe eines Oxidationsmittels durchgeführt, vorzugsweise mit Chromtrioxid in Pyridin/Methylenchlorid.

Die Abspaltung der t-Butyldiphenylsilylgruppe zu den freien Hydroxylaktonen I wird vorzugsweise in Acetonitril mit wäßrigem Fluorwasserstoff bei 40–60°C oder mit Tetrabutylammoniumfluorid in Eisessig/THF bei Raumtemperatur oder leicht erhöhter Temperatur durchgeführt.

Die Hydroxylaktone I werden mit wäßrigem Alkali gegebenenfalls unter Zuhilfenahme eines Lösungsmittels beispielsweise Alkohol oder Tetrahydrofuran in üblicher Weise in die Alkalisalze überführt, aus denen sich nach Ansäuren die freien Säuren erhalten lassen. Die Ammoniumsalze werden mit entsprechenden Aminen aus den freien Säuren in allgemein bekannter Weise erhalten. Die Ester der freien Säuren können aus diesen selbst, aus den entsprechenden Alkalisalzen oder aus den Laktonen I nach üblichen bekannten Verfahren erhalten werden.

Sofern die einzelnen Reaktionsprodukte nicht bereits in genügend reiner Form anfallen, so daß sie für den folgenden Reaktionsschritt eingesetzt werden können, eignet sich eine Reinigung mittels Kristallisation, Säulen- oder Hochdruckflüssigkeitschromatographie.

Falls das Jodid der Formel III nicht als reines Enantiomeres vorliegt, können auch Mischungen der enantiomeren Endprodukte entstehen, die nach allgemein üblichen Verfahren aufgetrennt werden können.

Das chirale Jodid III kann beispielsweise nach einem bekannten Verfahren (J.R. Falck, Tetrahedron Lett. 23, 4305 (1982)) aus tri-O-Acetyl-D-glucal hergestellt werden.

Soweit die als Ausgangsmaterial eingesetzten Phenole II nicht in der Literatur beschrieben sind, lassen sie sich in Analogie zu bekannten Verfahren herstellen (Houben-Weyl, Methoden der organischen Chemie, Band VI/1 c, 1976). Ein bevorzugtes Verfahren zur Herstellung von Phenolen II, welche die im Formelschema 1 angegebenen allgemeinen Formeln XVII, XVIII und XIX aufweisen, wobei die Reste $R^1$–$R^4$ die oben angegebene Bedeutung haben, geht von entsprechend substituierten Phenolen X aus (Formelschema 1). Diese werden in die entsprechenden Allylether XI überführt, vorzugsweise mit Allylbromid in Aceton oder Dimethylformamid bei erhöhter Temperatur unter Verwendung einer geeigneten Base, z.B. Kaliumcarbonat. Die Allylether XI werden bei erhöhter Temperatur in die entsprechenden Allylphenole XII umgelagert, in vielen Fällen ohne Lösungsmittel aber auch gegebenenfalls unter Verwendung von Diethylanilin als Lösungsmittel. Die so erhaltenen Phenole XII werden an der OH-Gruppe geschützt. Als Schutzgruppe (Z) eignet sich die Benzylgruppe, aber auch andere wie z.B. THP können verwendet werden. Die geschützten Allylphenole XIII werden vorzugsweise mit Diboran oder 9-Borabicyclo (3.3.1) nonan (9-BBN) in Tetrahydrofuran zu den Alkoholen XV (n = 3) hydroboriert. Die Aldehyde XIV werden aus den geschützten Phenolen XIII mit Oxidationsmitteln wie z.B. $NaIO_4/OsO_4$ oder

Ozon entweder direkt erhalten oder durch Glykolspaltung der aus XIII erhaltbaren 1,2-Diole gewonnen. Reduktion der Aldehyde XIV, vorzugsweise mit Natriumborhydrid führt zu den Alkoholen XV (n = 2). Die Alkohole der Formel XV (n = 2 oder 3) werden gegebenenfalls in ein reaktionsfähiges Derivat, vorzugsweise Tosylat, Mesylat oder Jodid überführt und mit entsprechenden Phenolen oder Alkoholen in Gegenwart einer Base, vorzugsweise Kaliumcarbonat, in geeigneten Lösungsmitteln, z.B. Dimethylsulfoxid oder Dimethylformamid verethert (Verbindungen der Formel XVII gemäß Formelschema, jedoch geschützt). Alternativ können die Alkohole XV (n = 2, 3) mit geeigneten Alkylhalogeniden umgesetzt werden, beispielsweise in Dimethylformamid unter Verwendung von Natriumhydrid als Base zu den geschützten Ethern entsprechend XVII. Nach Abspalten der Schutzgruppe Z werden die Phenole XVII erhalten. Acylierung der Alkohole XV mit einem reaktionsfähigen Derivat einer Carbonsäure nach allgemein üblichen Verfahren führt nach Abspaltung der Schutzgruppe Z zu Phenolen der Formel XVIII. Einwirkung von geeigneten Grignard Reagenzien auf die Aldehyde XIV führt zu den Alkoholen XVI. Diese Alkohole werden nach allgemein bekannten Verfahren in die Phenole der Formel XIX ($R^9$ = H) überführt. Die Alkohole XVI können mit Oxidationsmitteln in die entsprechenden Ketone überführt werden und dann durch erneutes Einwirken eines Grignard Reagenzes und entsprechend allgemein bekannten Verfahren in die Phenole XIX ($R^8$, $R^9 \neq$ H) überführt werden. Für Phenole XIX mit n = 0 geht man zweckmäßigerweise von entsprechenden Benzaldehyden, Benzoesäureestern oder aromatischen Ketonen aus.

Formelschema 1

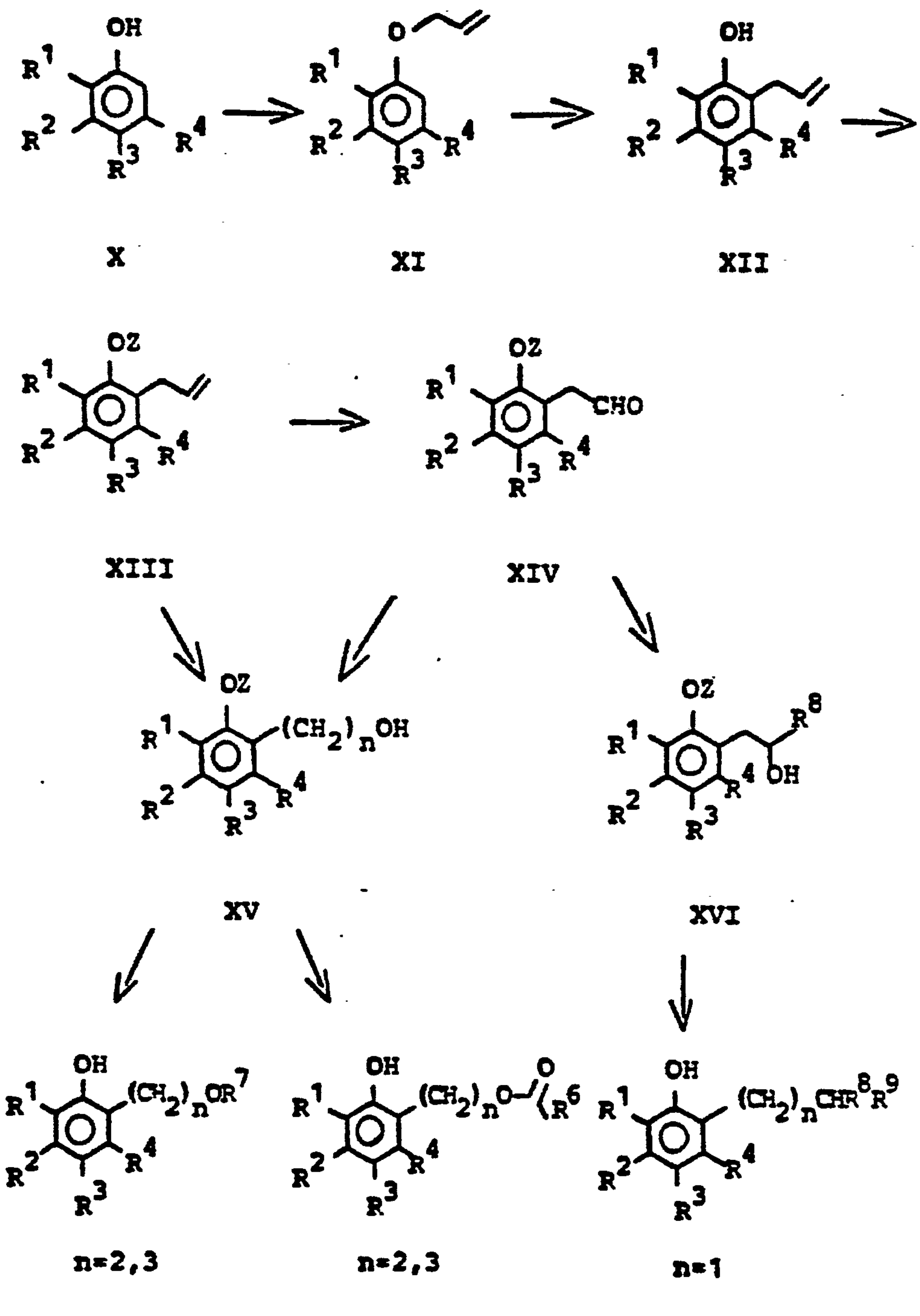

X

XI

XII

XIII

XIV

XV

XVI

n=2,3

n=2,3

n=1

XVII

XVIII

XIX

Außer den in den Beispielen beschriebenen Verbindungen lassen sich nach den erfindungsgemäßen Verfahren die folgenden Verbindungen herstellen:
6(S)-{2-[3-(4-Fluorphenoxy)propyl]-4-methyl-6-propylphenoxymethyl}-3,4,5,6-tetrahydro-4(R)-hydroxy-2H-pyran-2-on
6(S)-{2-[3-(4-Fluorphenoxy)propyl]-4-fluor-6-propylphenoxymethyl}-3,4,5,6-tetrahydro-4(R)-hydroxy-2H-pyran-2-on
6(S)-{2-[3-(4-Chlorphenoxy)propyl]-4-fluor-6-propylphenoxymethyl}-3,4,5,6-tetrahydro-4(R)-hydroxy-2H-pyran-2-on
6(S)-{2-[3-(4-Methylphenoxy)propyl]-4-flucr-6-propylphenoxymethyl}-3,4,5,6-tetrahydro-4(R)-hydroxy-2H-pyran-2-on
6(S)-{2-[3-(4-Fluorphenoxy)propyl]-4-chlor-6-propylphenoxymethyl}-3,4,5,6-tetrahydro-4(R)-hydroxy-2H-pyran-2-on
6(S)-{2,6-Bis-[3-(4-fluorphenoxy)propyl]-4-methylphenoxymethyl}-3,4,5,6-tetrahydro-4(R)-hydroxy-2H-pyran-2-on
6(S)-{2,6-Bis-[3-(4-methylphenoxy)propyl]-4-methylphenoxymethyl}-3,4,5,6-tetrahydro-4(R)-hydroxy-2H-pyran-2-on
6(S)-{2-[3-(2,4,6-trimethylphenoxy)propyl]-6-allyl-4-methylphenoxymethyl}-3,4,5,6-tetrahydro-4(R)-hydroxy-2H-pyran-2-on
6(S)-{2-[3-(2,4,6-trimethylphenoxy)propyl]-6-propyl-4-methylphenoxymethyl}-3,4,5,6-tetrahydro-4(R)-hydroxy-2H-pyran-2-on
6(S)-{2-[3-(4-Fluorphenoxy)propyl]-4-chlor-6-cyclopentylphenoxymethyl}-3,4,5,6-tetrahydro-4(R)-hydroxy-2H-pyran-2-on
6(S)-{2-[3-(4-Methylphenoxy)propyl]-4-chlor-6-cyclopentylphenoxymethyl}-3,4,5,6-tetrahydro-4(R)-hydroxy-2H-pyran-2-on
6(S)-{2-[3-(4-Fluorphenoxy)propyl]-4-methyl-6-cyclopentylphenoxymethyl}-3,4,5,6-tetrahydro-4(R)-hydroxy-2H-pyran-2-on
6(S)-{2-[2-(4-Fluorphenyl)ethyl]-4-methyl-6-cyclopentylphenoxymethyl}-3,4,5,6-tetrahydro-4(R)-hydroxy-2H-pyran-2-on
6(S)-{2-[2-(4-Methylphenyl)ethyl]-4-methyl-6-cyclopentylphenoxymethyl}-3,4,5,6-tetrahydro-4(R)-hydroxy-2H-pyran-2-on
6(S)-{2-[2-(4-Chlorphenyl)ethyl]-4,6-dimethylphenoxymethyl}-3,4,5,6-tetrahydro-4(R)-hydroxy-2H-pyran-2-on
6(S)-{2-[2-(4-Fluorphenyl)ethyl]-4-methyl-6-propylphenoxymethyl}-3,4,5,6-tetrahydro-4(R)-hydroxy-2H-pyran-2-on
6(S)-{2-[2-(4-Fluorphenoxy)ethyl]-4,6-dimethylphenoxymethyl}-3,4,5,6-tetrahydro-4(R)-hydroxy-2H-pyran-2-on
6(S)-{2-[2-(4-Methylphenoxy)ethyl]-4,6-dimethylphenoxymethyl}-3,4,5,6-tetrahydro-4(R)-hydroxy-2H-pyran-2-on
6(S)-{2-[2,2-Bis-(4-methylphenyl)ethyl]-4,6-dimethylphenoxymethyl}-3,4,5,6-tetrahydro-4(R)-hydroxy-2H-pyran-2-on
6(S)-[2-(4-Methylbenzyl)-4,6-dimethylphenoxymethyl]-3,4,5,6-tetrahydro-4(R)-hydroxy-2H-pyran-2-on
6(S)-[2-Bis-(4-methylphenyl)methyl-4,6-dimethylphenoxymethyl]-3,4,5,6-tetrahydro-4(R)-hydroxy-2H-pyran-2-on
6(S)-[2-Bis-(4-methylphenyl)methyl-6-methylphenoxymethyl]-3,4,5,6-tetrahydro-4(R)-hydroxy-2H-pyran-2-on
6(S)-[2-Bis-(4-fluorphenyl)methyl-6-methylphenoxymethyl]-3,4,5,6-tetrahydro-4(R)-hydroxy-2H-pyran-2-on
6(S)-{2-[1-(4-Fluorphenyl)-3-methylbutyl]-4,6-dimethylphenoxymethyl}-3,4,5,6-tetrahydro-4(R)-hydroxy-2H-pyran-2-on
6(S)-{2-[1-(4-Fluorphenyl)-2-methylpropyl]-4,6-dimethylphenoxymethyl}-3,4,5,6-tetrahydro-4(R)-hydroxy-2H-pyran-2-on
6(S)-[2-(1-Cyclohexyl-1-methyl)ethyl-4,6-dimethylphenoxymethyl]-3,4,5,6-tetrahydro-4(R)-hydroxy-2H-pyran-2-on
6(S)-{2-[1-(4-Fluorphenyl)-1-methyl]-ethyl-4,6-dimethylphenoxymethyl}-3,4,5,6-tetrahydro-4(R)-hydroxy-2H-pyran-2-on
6(S)-[2-(2-Cyclohexylethyl)-4,6-dimethylphenoxymethyl]-3,4,5,6-tetrahydro-4(R)-hydroxy-2H-pyran-2-on
6(S)-[2-(3,4,5-Trimethoxybenzyl)-4,6-dimethylphenoxymethyl]-3,4,5,6-tetrahydro-4(R)-hydroxy-2H-pyran-2-on
6(S)-{2-[1-(4-Fluorphenyl)-3-(4-fluorphenoxy)-propyl]-4-chlor-6-methyl-phenoxymethyl}-3,4,5,6-tetrahydro-4(R)-hydroxy-2H-pyran-2-on
6(S)-{2-[1-(4-Fluorphenyl)-3-(4-fluorphenoxy)-propyl]-4-fluor-6-methyl-phenoxymethyl}-3,4,5,6-tetrahydro-4(R)-hydroxy-2H-pyran-2-on
6(S)-{2-[1-Cyclohexyl-3-(4-fluorphenoxy)-propyl]-4,6-dichlor-phenoxymethyl}-3,4,5,6-tetrahydro-4(R)-hydroxy-2H-pyran-2-on
6(S)-{2-[1-Cyclohexyl-3-(4-fluorphenoxy)-propyl]-4-chlor-6-methyl-phenoxymethyl}-3,4,5,6-tetrahydro-4(R)-hydroxy-2H-pyran-2-on
6(S)-{2-[1-Cyclohexyl-3-(4-fluorphenoxy)-propyl]-4-fluor-6-methyl-phenoxymethyl}-3,4,5,6-tetrahydro-4(R)-hydroxy-2H-pyran-2-on
6(S)-{2-[1-Cyclohexyl-3-(4-fluorphenoxy)-propyl]-6-methyl-phenoxymethyl}-3,4,5,6-tetrahydro-4(R)-hydroxy-2H-pyran-2-on
6(S)-{2-[Bis-(4-fluorphenyl)-methyl]-4-fluor-6-methyl-phenoxymethyl}-3,4,5,6-tetrahydro-4(R)-hydroxy-2H-pyran-2-on
6(S)-{2-[Cyclohexyl-(4-fluorphenyl)-methyl]-4-chlor-6-methyl-phenoxymethyl}-3,4,5,6-tetrahydro-4(R)-hydroxy-2H-pyran-2-on
6(S)-{2-[Cyclohexyl-(4-fluorphenyl)-methyl]-4-methyl-6-chlor-phenoxymethyl}-3,4,5,6-tetrahydro-4(R)-hydroxy-2H-pyran-2-on
6(S)-{2-[Cyclohexyl-(4-fluorphenyl)-methyl]-4,6-dichlor-phenoxymethyl}-3,4,5,6-tetrahydro-4(R)-hydroxy-2H-pyran-2-on
6(S)-{2-[Cyclohexyl-(4-chlorphenyl)-methyl]-4-chlor-6-methyl-phenoxymethyl}-3,4,5,6-tetrahydro-4(R)-hydroxy-2H-pyran-2-on
6(S)-{2-[Cyclohexyl-(4-chlorphenyl)-methyl]-4-methyl-6-chlor-phenoxymethyl}-3,4,5,6-tetrahydro-4(R)-hydroxy-2H-pyran-2-on
6(S)-{2-[Cyclohexyl-(4-chlorphenyl)-methyl]-4,6-dichlor-phenoxymethyl}-3,4,5,6-tetrahydro-4(R)-hydroxy-2H-pyran-2-on
6(S)-{2-[Bis-(4-chlorphenyl)-methyl]-4,6-dimethyl-phenoxymethyl}-3,4,5,6-tetrahydro-4(R)-hydroxy-2H-pyran-2-on
6(S)-{2-[Bis-(4-chlorphenyl)-methyl]-4-chlor-methyl-phenoxymethyl}-3,4,5,6-tetrahydro-4(R)-hydroxy-2H-pyran-2-on
6(S)-{2-[Bis-(4-chlorphenyl)-methyl]-4-methyl-6-chlor-phenoxymethyl}-3,4,5,6-tetrahydro-4(R)-hydroxy-2H-pyran-2-on
6(S)-{2-[Bis-(4-chlorphenyl)-methyl]-4,6-dichlor-phenoxymethyl}-3,4,5,6-tetrahydro-4(R)-hydroxy-2H-pyran-2-on
6(S)-{2-[Bis-(4-chlor-3-methyl-phenyl)-methyl]-4,6-dimethyl-phenoxymethyl}-3,4,5,6-tetrahydro-4(R)-hydroxy-2H-pyran-2-on
6(S)-{2-[Bis-(4-chlor-3-methyl-phenyl)-methyl]-4-chlor-6-methyl-phenoxymethyl}-3,4,5,6-tetrahydro-4(R)-hydroxy-2H-pyran-2-on
6(S)-{2-[Bis-(4-chlor-3-methyl-phenyl)-methyl]-4-methyl-6-chlor-phenoxymethyl}-3,4,5,6-tetrahydro-4(R)-hydroxy-2H-pyran-2-on
6(S)-{2-[Bis-(4-chlor-3-methyl-phenyl)-methyl]-4,6-dichlor-phenoxymethyl}-3,4,5,6-tetrahydro-4(R)-hydroxy-2H-pyran-2-on
6(S)-{2-[Bis-(3-fluor-4-methyl-phenyl)-methyl]-4,6-dimethyl-phenoxymethyl}-3,4,5,6-tetrahydro-4(R)-hydroxy-2H-pyran-2-on
6(S)-{2-[Bis-(3-fluor-4-methyl-phenyl)-methyl]-4-chlor-6-methyl-phenoxymethyl}-3,4,5,6-tetrahydro-4(R)-hydroxy-2H-pyran-2-on
6(S)-{2-[Bis-(3-fluor-4-methyl-phenyl)-methyl]-4-methyl-6-chlor-phenoxymethyl}-3,4,5,6-tetrahydro-4(R)-hydroxy-2H-pyran-2-on
6(S)-{2-[Bis-(3-fluor-4-methyl-phenyl]-4,6-dichlor-phenoxymethyl}-3,4,5,6-tetrahydro-4(R)-hydroxy-2H-pyran-2-on
6(S)-{2-[Bis-(3-chlor-4-methyl-phenyl)-methyl]-4,6-dimethyl-phenoxymethyl}-3,4,5,6-tetrahydro-4(R)-hydroxy-2H-pyran-2-on
6(S)-{2-[Bis-(3-chlor-4-methyl-phenyl)-methyl]-4-chlor-6-methyl-phenoxymethyl}-3,4,5,6-tetrahydro-4(R)-hydroxy-2H-pyran-2-on
6(S)-{2-[Bis-(3-chlor-4-methyl-phenyl)-methyl]-4-methyl-6-chlor-phenoxymethyl}-3,4,5,6-tetrahydro-4(R)-hydroxy-2H-pyran-2-on
6(S)-{2-[Bis-(3-chlor-4-methyl-phenyl)-methyl]-4,6-dichlor-phenoxymethyl}-3,4,5,6-tetrahydro-4(R)-hydroxy-2H-pyran-2-on

Das Enzym HMG–CoA-Reduktase ist in der Natur weit verbreitet. Es katalysiert die Bildung von Mevalonsäure aus HMG–CoA. Diese Reaktion ist ein zentraler Schritt der Cholesterin-Biosynthese (I.R. Sabine, 3-Hydroxy-3-methylglutaryl Coenzym A Reductase, CRC Press, 1983). Hohe Cholesterinspiegel werden mit einer Reihe von Erkrankungen wie z.B. koronarer Herzkrankheit oder Atherosklerose in Verbindung gebracht. Daher ist die Senkung erhöhter Cholesterinspiegel zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG–CoA-Reduktase blockieren die Cholesterin-Biosynthese auf einer frühen Stufe. Sie eignen sich daher zur Vorbeugung und Behandlung von Erkrankungen, die durch einen erhöhten Cholesterinspiegel verursacht werden. Eine Reduktion bzw. Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäuren-bindender Stoffe wie Anionenaustauscher erzielen. Die erhöhte Gallensäurenausscheidung führt zu einer

verstärkten Neusynthese und damit zu einem erhöhten Cholesterinabbau (M.S. Brown, P.T. Kovanen, J.L. Goldstein, Science 212, 628 (1981); M.S. Brown, J.L. Goldstein Spektrum der Wissenschaft 1985 (1), 96). Die erfindungsgemäßen Verbindungen sind Hemmstoffe der HMG–CoA-Reduktase. Sie eignen sich daher zur Hemmung bzw. Verminderung der Cholesterin-Biosynthese und damit zur Vorbeugung oder Behandlung von Erkrankungen, die durch erhöhte Cholesterinspiegel verursacht werden, insbesondere koronare Herzkrankheit, Atherosklerose, Hypercholesterinämie, Hyperlipoproteinämie und ähnliche Erkrankungen.

Die Erfindung betrifft daher auch pharmazeutische Präparate auf Basis der Verbindung der Formel I bzw. der entsprechenden Dihydroxycarbonsäuren, deren Salzen und Ester, sowie die Verwendung dieser Verbindungen als Arzneimittel, insbesondere zur Behandlung der Hypercholesterinämie.

Die Verbindungen der Formel I bzw. die entsprechenden Säuren, Salze oder Ester werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 2500 mg, vorzugsweise im Dosisbereich 10–500 mg.

Die erfindungsgemäßen Verbindungen können als Laktone der allgemeinen Formel I, in Form der freien Säuren VII oder in Form pharmazeutisch annehmbarer Salze oder Ester zur Anwendung kommen, und zwar gelöst oder suspendiert in pharmakologisch unbedenklichen organischen Lösungsmitteln wie einoder mehrwertigen Alkoholen wie z.B. Ethanol oder Glycerin, in Triacetin, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder anderen pharmazeutisch annehmbaren Zusatzstoffen wie Stärke, Cyclodextrin oder Polysacchariden. Ferner können die erfindungsgemäßen Verbindungen kombiniert werden mit Zusatzstoffen, die Gallensäuren binden, insbesondere nicht toxische, basische Anionenaustauscherharze, die Gallensäuren in einer nichtresorbierbaren Form im Gastrointestinaltrakt binden. Die Salze der Dihydroxycarbonsäuren können auch als wäßrige Lösungen verabreicht werden.

Die Verbindungen der Formeln IV–VI sind neu und z.B. wertvolle Zwischenprodukte für die Herstellung von Verbindungen der Formel I. Die Erfindung betrifft daher auch die Verbindungen der Formeln IV, V und VI sowie Verfahren zu deren Herstellung.

Die HMG–CoA-Reduktase-Aktivität wurde in folgenden Testsystemen bestimmt:

1) Inhibierung der HMG–CoA-Reduktase-Aktivität an solubilisierten Enzympräparationen aus Ratten-Lebermikrosomen

Die HMG–CoA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden. Als Substrat diente (S,R)$^{14}$C–HMG–CoA, die Konzentration von NADPH wurde während der Inkubation durch ein regenerierendes System aufrechterhalten. Die Abtrennung von $^{14}$C-Mevalonat von Substrat und anderen Produkten (z.B. $^{14}$C–HMG) erfolgte über Säulenelution, wobei das Elutionsprofil jeder Einzelprobe ermittelt wurde. Auf die ständige Mitführung von $^{3}$H-Mevalonat wurde verzichtet, weil es bei der Bestimmung um die Relativangabe der Hemmwirkung handelt. In eine Versuchsreihe wurden jeweils die enzymfreie Kontrolle, der enzymhaltige Normalansatz (= 100%) und solche mit Präparatezusätzen zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt. Nach der oben beschriebenen Methode wurden von den erfindungsgemäßen Verbindungen z.B. folgende Hemmwerte auf die HMG–CoA-Reduktase ermittelt ($IC_{50}$-Wert M = molare Konzentration der Verbindung, die für eine 50%ige Hemmung erforderlich ist):

| Beispiel | $IC_{50}$-Wert M | Beispiel | $IC_{50}$-Wert M |
|---|---|---|---|
| 44 a | 3,6 x $10^{-7}$ | 44 w | 9,0 x $10^{-8}$ |
| 44 i | 7,5 x $10^{-8}$ | 44 x | 5,0 x $10^{-8}$ |
| 44 j | 4,6 x $10^{-7}$ | 44 y | 7,5 x $10^{-8}$ |
| | | 44 z | 1,0 x $10^{-7}$ |
| 44 l | 7,0 x $10^{-6}$ | 44 aa | 9,5 x $10^{-8}$ |
| 44 m | 5,3 x $10^{-7}$ | 44 bb | 1,0 x $10^{-7}$ |
| 44 q | 7,0 x $10^{-8}$ | 44 dd | 1,5 x $10^{-7}$ |
| 44 r | 1,6 x $10^{-7}$ | 44 hh | 5,0 x $10^{-8}$ |
| 44 s | 4,0 x $10^{-7}$ | 44 kk | 2,5 x $10^{-8}$ |
| 44 t | 5,0 x $10^{-7}$ | 44 mm | 1,9 x $10^{-7}$ |
| 44 u | 1,4 x $10^{-7}$ | 44 nn | 1,9 x $10^{-7}$ |
| 44 v | 1,0 x $10^{-7}$ | | |

2. Supprimierung bzw. Inhibierung der HMG–CoA-Reduktase in Zellkulturen von Fibroblasten

Monolayers von Fibroblasten (L-Zellen) in lipoproteinfreiem Nährmedium wurden mit entsprechenden Konzentrationen der Prüfsubstanzen eine bestimmte Zeit (z.B. 3 Stunden) inkubiert, dann wurde die HMG–CoA-Reduktaseaktivität der Zellen, modifiziert nach Chang et al. (J. Biol. Chem. 256, 6174 (1981)) bestimmt. Dazu wurden die Zellextrakte mit D, L [$^3$H]–HMG–CoA inkubiert und das unter der Einwirkung der vorhandenen HMG–CoA-Reduktaseaktivität aus den Zellen gebildete Produkt [$^3$H]-Mevalonat nach Cyclisierung zu [$^3$H]-Mevalonolacton dünnschichtchromatographisch vom Ausgangsprodukt getrennt und unter Verwendung eines internen Standards von $^{14}$C-Mevalonat die Menge des in der Zeiteinheit gebildeten [$^3$H]-Mevalonats, bezogen auf Zellproteinmenge bestimmt. Die unter Zusatz einer bestimmten Konzentration eines Prüfpräparates gefundene HMG–CoA-Reduktaseaktivität der Zellkultur wurde prozentual auf jene einer gleichbehandelten ohne Prüfpräparat, jedoch gleicher Lösungsmittelkonzentration, bezogen.

Biologische Prüfergebnisse:

Unter der Bedingung einer dreistündigen Inkubationszeit konfluenter L-Zellen im lipoproteinfreien Nährmedium mit den untersuchten Verbindungen wurden folgende für eine 50%ige Hemmung der HMG–CoA-Reduktaseaktivität ($IC_{50}$) erforderliche molare Konzentration der Prüfpräparate ermittelt:

| Verbindung gemäß Beispiel Nr.: | $IC_{50}$ |
|---|---|
| 44 a | $2x10^{-4}$ M |
| 44 i | $1x10^{-5}$ M |
| 44 j | $1x10^{-4}$ M |
| 44 q | $5x10^{-5}$ M |
| 44 v | $4x10^{-5}$ M |
| 44 w | $8x10^{-5}$ M |
| 44 x | $1.4x10^{-5}$ M |
| 44 y | $1.5x10^{-5}$ M |

Herstellung der Ausgangsverbindungen der Formel II

Beispiel 1:

2-Allyl-4,6-dimethylphenol (1) (Formelschema 1, XII)

150 g (1,23 Mol) 2,4-Dimethylphenol, 330 g (2,4 Mol) Kaliumcarbonat und 180 g (1,5 Mol) Allylbromid wurden in 900 ml Aceton 20 h unter Rückfluß gerührt. Nach dem Abkühlen wurde der Niederschlag abgesaugt und mit Aceton nachgewaschen. Das Filtrat wurde im Vakuum eingeengt und der Rückstand zwischen Petrolether und 2n Natronlauge verteilt. Die organische Phase wurde dreimal mit 2n Natronlauge und zweimal mit Wasser gewaschen. Trocknen mit Magnesiumsulfat, Einengen im Vakuum und Destillation ergab 178,7 g (1,08 Mol, 88%) 2,4-Dimethylphenylallylether, Kp. 86°C/7 Torr. Der Allylether wurde unter Rühren ohne Lösungsmittel 3 h auf 210°C erhitzt. Destillation ergab 165,4 g (1,02 Mol, 94%) 2-Allyl-4,6-dimethylphenol (1) Kp. 96–100°C/8 Torr.

$^1$H-NMR ($CDCl_3$, 60 MHz): $\delta$ = 2,2 (S, 6H, Methyl-H), 3,3 (d, 2H, $-CH_2-$), 4,6 (s, 1H, –OH), 4,8–6,2 (m, 3H, olefinische H), 6,7 (m, 2H, aromatische H).

Beispiel 2:

2-Allyl-4,6-dimethylphenylbenzylether (2) (Formelschema 1, XIII)

76,15 g (0,47 Mol) 2-Allyl-4,6-dimethylphenol, 130 g (0,94 Mol) Kaliumcarbonat und 65,5 g (0,52 Mol) Benzylchlorid wurden in 1 l Aceton 20 h unter Rückfluß erhitzt. Nach 20 h wurden nochmals 26 g Kaliumcarbonat und 13 g Benzylchlorid zugegeben. Es wurde abgesaugt und das Filtrat im Vakuum eingeengt und in Toluol aufgenommen. Die organische Phase wurde zweimal mit 2n Natronlauge und zweimal mit Wasser gewaschen. Trocknen mit Magnesiumsulfat, Abziehen des Lösungsmittels und Destillation ergab 109,8 g (0,44 Mol, 85%) Benzylether 2, KP.: 147–153°C/0,2 Torr.

$^1$H-NMR ($CDCl_3$, 60 MHz): $\delta$ = 2,2 (s, 6H, Methyl-H), 3,4 (d, 2H, allyl.-$CH_2-$), 4,7 (s, 2H, benzyl.-$CH_2-$), 4,8–6,2 (m, 3H, olefinische H), 6,8 (s, 2H, aromatische H), 7,0–7,4 (m, 5H, aromatische H).

In Analogie zu Beispiel 1 wurden die Allylphenole 4, 7 und 9 gewonnen und in Analogie zu Beispiel 2 als Benzylether 5, 8 und 10 geschützt.

Tabelle 1
(die Formeln entsprechen den Formeln X, XII und XIII des Formelschemas 1)

| 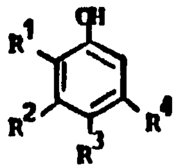 X | beschrieben in | 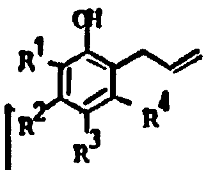 XII | charakteristische $^1H$-NMR-Daten ($CDCl_3$, 60 MHz) $\delta$ = | 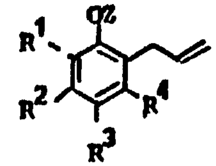 XIII<br>Z = $CH_2C_6H_5$ |
|---|---|---|---|---|
| 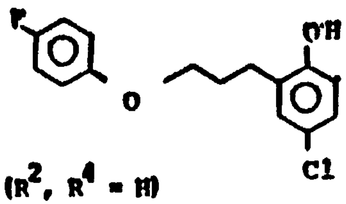<br>($R^2$, $R^4$ = H) | Beispiel 3 | Beispiel: 4<br>($R^2$, $R^4$ = H) | 2,0 (m, 2H, $-CH_2-$)<br>3,0 (t, 2H, Ar-$CH_2$-)<br>3,3 (d, 2H, Ar-$CH_2$-)<br>3,9 (t, 2H, $-OCH_2-$)<br>4,8-6,2 (m, 3H, olefin.H) | Beispiel 5<br>($R^2$, $R^4$ = H) |
| 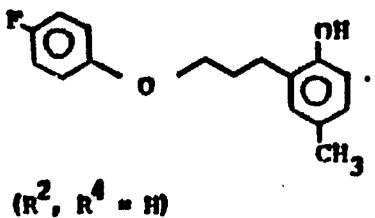<br>($R^2$, $R^4$ = H) | Beispiel 6 | Beispiel 7<br>($R^2$, $R^4$ = H) | 2,0 (m, 2H, $-CH_2-$)<br>2,2 (s, 3H, $-CH_3$)<br>3,2 (d, 2H, Ar-$CH_2$-)<br>3,8 (t, 2H, $-OCH_2-$)<br>4,8-6,2 (m, 3H, olefin.H) | Beispiel 8<br>($R^2$, $R^4$ = H) |
| 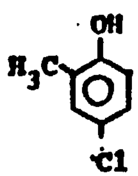<br>($R^2$, $R^4$ = H) | Beil 6,359 | Beispiel 9<br>($R^2$, $R^4$ = H) | 2,2 (s, 3H, $-CH_3$)<br>4,9-6,1 (m, 3H, olefin. H) | Beispiel 10<br>($R^2$, $R^4$ = H) |

Beispiel 11:

3-(2-Benzyloxy-3,5-dimethylphenyl)propan-1-ol (11) (Formelschema 1, XV)

108,5 g (0,43 Mol) 2-Allyl-4,6-dimethylphenylbenzylether (Beispiel 2) wurden in 250 ml trockenem Tetrahydrofuran unter Argonatmosphäre mit 9,76 g (0,258 Mol) Natriumborhydrid versetzt. Innerhalb 5 min wurden unter Rühren 32,5 g (0,258 Mol) Dimethylsulfat zugetropft. Das Reaktionsgemisch erwärmte sich auf ca. 40°C. Man ließ 4 h bei 35–40°C rühren. Anschließend wurden bei 5°C vorsichtig 43 ml Wasser und anschließend 320 ml 14 n wäßrige Natronlauge zugetropft. Schließlich wurden unter Kühlen 65 ml 35 proz. (0,75 Mol) Wasserstoffperoxidlösung zugetropft und 30 min nachgerührt. Die Reaktionsmischung wurde in Toluol gegossen und viermal mit Eiswasser und zweimal mit gesättigter wäßriger Natriumchloridlösung gewaschen. Trocknen mit Magnesiumsulfat und Einengen im Vakuum ergaben 110,4 g eines hellgelben Öls. Präp. HPLC auf Kieselgel (Cyclohexan/Essigester = 5:1) ergab 89,15 g (0,33 Mol, 76%) Alkohol als hellgelbes Öl).
$^1$H-NMR ($CDCl_3$, 60 MHz): δ = 1,8 (m, 2H, $-CH_2-$), 2,2 (d, 6H, Methyl-H), 2,7 (t, 2H, $Ar-CH_2-$), 3,4 (t, 2H, $-CO_2O-$), 4,8 (s, 2H, benzyl. $-CH_2-$), 6,8 (s, 2H, aromatische H), 7,0–7,4 (m, 5H, aromatische H).

Beispiel 12:

3-{2-Benzyloxy-3-[3-(4-Fluorphenoxy)propyl]-4-chlorphenyl}propan-1-01 (12) (Formelschema 1, XV)

72,6 ml einer 0,5 m 9-BBN-Lösung (35 mmol) in Tetrahydrofuran wurden in 50 ml trockenem Tetrahydrofuran vorgelegt. Bei Raumtemp. wurden 9,5 g (23,1 mmol) Allylverbindung aus Beispiel 5 zugetropft und 30 min gerührt. Anschließend wurde 1 h unter Rückfluß erhitzt. Nach dem Erkalten wurden nacheinander vorsichtig 14 ml Ethanol, 23 ml 2n wäßrige NaOH (46 mmol) und schließlich 6,1 ml (59 mmol) 30 proz. Wasserstoffperoxidlösung. Man ließ 1 h unter Rückfluß kochen. Das Reaktionsgemisch wurde in Toluol gegossen. Die organische Phase wurde dreimal mit Eiswasser und einmal mit gesättigter wäßriger Natriumchloridlösung gewaschen. Trocknen mit Magnesiumsulfat, Einengen im Vakuum und Chromatographie auf Kieselgel (Cyclohexan/Essigester = 5:1) ergab 9,0 g (21 mmol, 91%) Alkohol 12.
$^1$H-NMR ($CDCl_3$, 60 MHz): δ = 1,4–2,5 (m, 4H, $-CH_2-$), 2,5–3,0 (m, 4H, $Ar-CH_2-$), 3,5 und 3,8 (jeweils t, jeweils 2H, $-OCH_2-$), 4,8 (s, 2H, benzyl.-$CH_2-$), 6,6–7,5 (m, 11H, aromatische H).
In Analogie zu Beispiel 11 und 12 wurden die in Tabelle 2 aufgeführten Alkohole hergestellt.

**Tabelle 2:** (vgl. Formelschema 1, XV)

| Beispiel | Hydroborierungsreagenz | Produkt: $Z^1 = CH_2C_6H_5$ 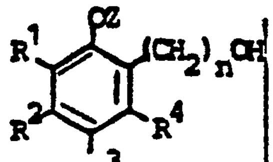 XV | Edukt |
|---|---|---|---|
| 13 | 9-BBN | 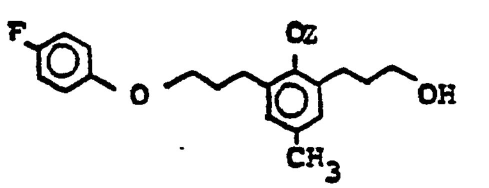 | 8 |
| 14 | $BH_3$<br>9-BBN | 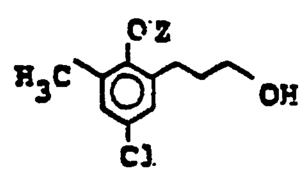 | 10 |

Beispiel 15:

2-[3-(4-Fluorphenoxy)propyl]-4,6-dimethylphenol (15) (Formelschema 1, XVII)

1. 43,5 g (162 mmol) 11 wurden in 160 ml Methylenchlorid und 160 ml Pyridin gelöst und unter Kühlen portionsweise mit 37 g (194 mmol) p-Toluolsulfonsäurechlorid versetzt. Man ließ auf Raumtemp. kommen. Nach 18 h wurde das Lösungsmittel im Vakuum abgezogen und der Rückstand mit Toluol aufgenommen und zweimal mit Wasser und zweimal mit gesättigter wäßriger Natriumhydrogencarbonatlösung, schließlich mit gesättigter wäßriger Natriumchloridlösung gewaschen. Trocknen mit Magnesiumsulfat, Abziehen des Lösungsmittels und Chromatographie auf Kieselgel ($CH_2Cl_2$) ergab 51 g (120 mmol, 74%) Tosylat 11a als farbloses Öl.

2. 51 g (120 mmol) Tosylat (11a) wurden mit 90 g (600 mmol) Natriumjodid in 900 ml Aceton 4 h unter Rückfluß erhitzt. Man saugte vom unlöslichen ab. Das Filtrat wurde im Vakuum eingeengt und der Rückstand in Toluol aufgenommen. Die organische Phase wurde einmal mit Wasser, zweimal mit 2 proz. wäßriger Natriumbisulfitlösung und einmal mit gesättigter wäßriger Natriumhydrogencarbonatlösung gewaschen. Trocknen mit Magnesiumsulfat, Abziehen des Lösungsmittels im Vakuum und Chromatographie auf Kieselgel (Cyclohexan/Essigester = 92:8) ergaben 40,6 g (107 mmol, 89%) Jodid 11b als farbloses Öl.

3. 9,62 g (86 mmol) 4-Fluorphenol wurden mit 23,7 g (172 mmol) Kaliumcarbonat in 75 ml Dimethylsulfoxid vorgelegt. Es wurden 21,8 g (57,3 mmol) Jodid 11b zugegeben und 4 h auf 50°C erhitzt. Das Reaktionsgemisch wurde zwischen Ether und Wasser verteilt. Die wäßrige Phase noch einmal mit Ether extrahiert. Die vereinten Etherphasen wurden einmal mit Wasser gewaschen und über Magnesiumsulfat getrocknet. Abziehen des Lösungsmittels im Vakuum und Chromatographie auf Kieselgel (Cyclohexan/Essigester = 9:1) ergab 20,5 g (56,3 mmol, 98%) 4-Fluorphenylether.

4. 20,5 g (56,3 mmol) 4-Fluorphenylether wurden in 200 ml Methanol mit 1 g Pd/C (10%) bei Raumtemp. und Normaldruck hydriert. Nach Chromatographie erhielt man 13,3 g (48,5 mmol, 86%) 2-[3-(4-Fluorphenoxy)propyl]-4,6-dimethylphenol (15).

MS: 274 ($M^+$)

$^1$H-NMR ($CDCl_3$, 60 MHz): $\delta$ = 1,8–2,3 (s und m, 8H, Methyl-H, $-CH_2-$), 2,8 (t, 2H, Ar–$CH_2$–), 3,9 (t, 2H, $OCH_2$–), 5,1 (s, 1H, OH), 6,7–7,2 (m, 6H, aromatische H).

Beispiel 16:

2-[2-(4-Fluorphenoxyethyl)]-4,6-dimethylphenol (16)

In Analogie zu Beispiel 15 wurden aus Beispiel 31 das Tosylat gemäß Reaktion 15.1 gewonnen und direkt Dimethylformamid unter Verwendung von 1.05 Äquivalenten Kaliumcarbonat bei 150°C, mit 4-Fluorphenyl umgesetzt. Abspaltung der Schutzgruppe gemäß 24.4 ergab Phenol 16.

MS: 260 ($M^+$)

$^1$H-NMR ($CDCl_3$, 60 MHz): $\delta$ = 225 (s, 6H, $-CH_3$), 3,06 (t, 2H, Ar–$CH_2$–), 4,20 (t, 2H, $-CH_2O$), 6,50 (s, 1H, OH), 6,67–7,00 (m, 6H, arom. H).

In Analogie zu Beispiel 15 wurden die in Tabelle 3 aufgeführten Phenole hergestellt. Die Abspaltung der Benzylgruppe wurde bei Chloraromaten unter Zusatz von Essigsäure durchgeführt.

Tabelle 3: (vgl. Formelschema 1, XVII)

| Beispiel | Formel | MS ($M^+$) | Ausgangs-material |
|---|---|---|---|
| 17 | 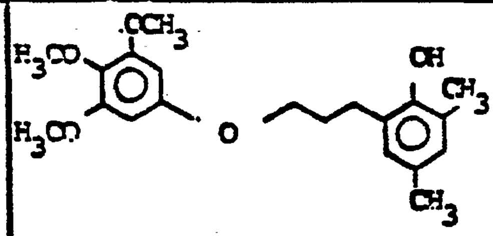 | $C_{20}H_{26}O_5$ (346) | 11 |
| 18 | 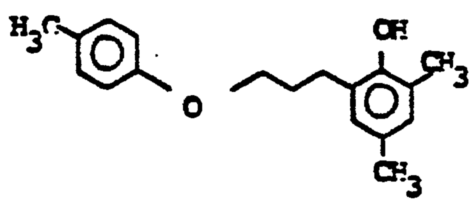 | $C_{18}H_{22}O_2$ (270) | 11 |
| 19 | 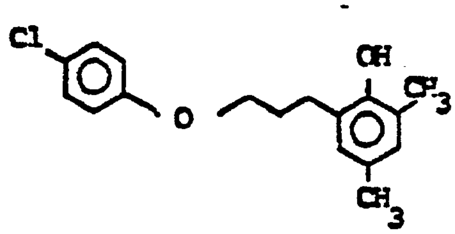 | $C_{17}H_{19}ClO_2$ (290) | 11 |
| 20 | 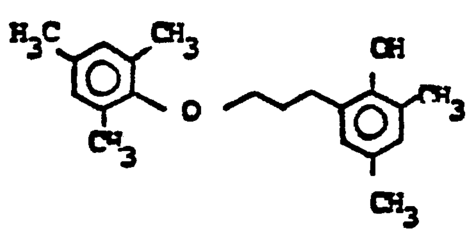 | $C_{20}H_{26}O_2$ (298) | 11 |

Tabelle 3 Fortsetzung

| Beispiel | Formel | MS($M^+$) | Ausgangs-material |
|---|---|---|---|
| 21 | 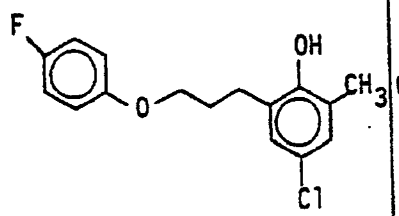 | $C_{16}H_{16}ClFO_2$(294) | 14 |
| 22 | 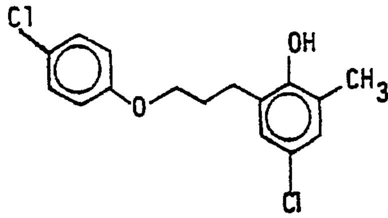 | $C_{16}H_{16}Cl_2O_2$(310) | 14 |
| 23 | 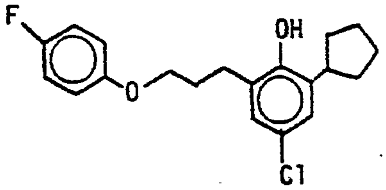 | $C_{20}H_{22}ClFO_2$(348) | |
| 24 | 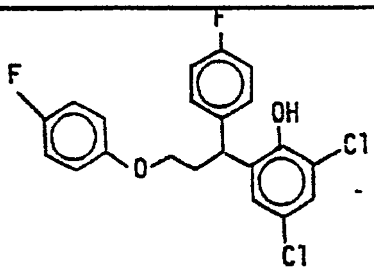 | $C_{21}H_{16}Cl_2F_2O_2$(408) | 1-(2,4-Dichlor-phenyl)-3-(4-Fluor-phenoxy)-propen W.N. White, W.K. Fife J.Org. Chem. 31,147 (1966) |

Beispiel 25:

2-[3-(4-Fluorphenoxy)propyl]-4-chlor-6-(3-pivaloyloxypropyl)phenylbenzylether (25) (vgl. Formelschema 1, XVIII, geschützt)

9 g (21 mmol) Alkohol 12 wurden in 90 ml Methylenchlorid und 90 ml Triethylamin gelöst und mit 3,4 g (28 mmol) Pivaloylchlorid über Nacht gerührt. Die Mischung wurde auf Eis/konz. Salzsäure gegossen und die organische Phase einmal mit 2 n Salzsäure und einmal mit gesättigter wäßriger Natriumhydrogencarbonatlösung gewaschen. Trocknen mit Magnesiumsulfat, Abziehen des Lösungsmittels und Chromatographie auf Kieselgel (Cyclohexan/Essigester = 5:1) ergab 5,12 g (10 mmol, 48%) Pivaloylester 25.

$^1$H-NMR ($CDCl_3$, 60 MHz): δ = 1,2 (s, 9H, Methyl-H), 1,7–2,3 (m, 4H, $-CH_2-$), 2,5–3,0 (m, 4H, Ar–$CH_2$–), 3,7–4,3 (m, 4H, $-OCH_2-$), 4,8 (s, 2H, benzyl.-$CH_2$–), 6,5–6,6 (m, 11H, aromatische H).

Beispiel 26:

2-[3-(4-Fluorphenoxy)propyl]-4-chlor-6-(3-pivaloyloxypropyl)phenol (26) (vgl. Formelschema 1, XVIII)

5,1 g (10 mmol) Pivaloylester wurden in 200 ml Essigsäureethylester und 20 ml Eisessig mit 500 mg Palladium auf Kohle (10%) bei Raumtemperatur und Normaldruck hydriert. Nach Chromatographie auf Kieselgel erhielt man 3,7 g (8,8 mmol, 87%) Phenol 26.
Schmp.: 78°C
MS: 422 ($M^+$), 320
In Analogie zu Beispiel 25 und 26 wurden die Beispiele 27–29 hergestellt.

Beispiel 27:

2-[3-(4-Fluorphenoxy)propyl]-4-methyl-6-(3-pivaloyloxypropyl)phenol, MS: 402 ($M^+$), 300, ausgehend von 13.

Beispiel 28:

4,6-Dimethyl-2-(3-pivaloyloxypropyl)phenol, MS: 264 ($M^+$) 162, ausgehend von 11.

Beispiel 29:

4,6-Dimethyl-2-[2(R,S)-pivaloyloxypropyl]phenol, MS: 264 ($M^+$), 162, ausgehend von 4,6-Dimethyl-2-[2(R,S)-pivaloyloxypropyl]phenylbenzylether, der bei der Darstellung von 11 anfällt.

Beispiel 30:

2-(2-Benzyloxy-3,5-dimethylphenyl)acetaldehyd (30) (vgl. Formelschema 1, XIV)

1. 205 g (0,81 Mol) 2-Allyl-4,6-dimethylphenylbenzylether (Beispiel 2) und 147 g (1,25 Mol) N-Methylmorpholin-N-oxid wurden in 1000 ml Aceton und 750 ml Wasser vorgelegt und mit 200 mg (0.79 mmol) Osmiumtetroxid versetzt. Man ließ 20 h bei Raumtemperatur rühren, gab 5 g Natriumpyrosulfit zu und sättigte mit Natriumchlorid. Das Aceton wurde im Vakuum weitgehend entfernt und der Rückstand mit Essigester extrahiert. Die vereinigten Essigesterphasen wurden einmal mit wäßriger NaCl/HCl- und einmal mit wäßriger NaCl/$KHCO_3$-Lösung gewaschen. Trocknen mit Magnesiumsulfat, Abziehen des Lösungsmittels im Vakuum und Kugelrohrdestillation (170–180°C, 0.1 Torr) ergab 114.8 g (0.40 Mol, 50%) Diol. Rf (Kieselgel, Essigester/Methylenchlorid = 1:1):0,35.

2. 8.3 g (29.0 mmol) Diol und 14.5 g (67.8 mmol) Natriumperjodat wurden in 50 ml Tetrahydrofuran und 10 ml Wasser bei 20–25°C unter Kühlen 30 min gerührt. Man verdünnte mit Wasser und extrahierte mit Ether. Die vereinigten Etherphasen wurden einmal mit Wasser und einmal mit Kaliumhydrogencarbonatlösung gewaschen. Trocknen mit Magnesiumsulfat und Abziehen des Lösungsmittels ergab nach Versetzen mit Toluol und erneutem Abrotieren und Trocknen an der Ölpumpe 7.1 g (28 mmol, 96%) 2-(2-Benzyloxy-3,5-dimethylphenyl)acetaldehyd (30).

$^1$H-NMR ($CCl_4$, 60 MHz): $\delta$ = 2,1 (s, 6H, $-CH_3$), 3,4 (d, 2H, $-CH_2-$), 4,6 (s, 2H, $-CH_2-$), 6,6 und 6,8 (jeweils s, jeweils 1H, arom. H), 7,2 ("s", 5H, arom. H), 9,2 (t, 1H, $-CHO$).

Beispiel 31:

2-(2-Benzyloxy-3,5-dimethylphenyl)ethanol (31) (vgl. Formelschema 1, XV)

7,7 g (30,3 mmol) Aldehyd 30 wurden in 50 ml Methanol gelöst vorgelegt und 0,9 g (23,8 mmol) Natriumborhydrid in Eiswasser gelöst bei 15°C zugetropft. Nach 30 min wurde unter Kühlen mit verdünnter Essigsäure angesäuert, mit 100 ml gesättigter wäßriger Natriumchloridlösung verrührt und mit Ether extrahiert. Die vereinigten Etherphasen wurden einmal mit gesättigter wäßriger Natriumhydrogencarbonatlösung gewaschen. Trocknen mit Magnesiumsulfat, Abziehen des Lösungsmittels im Vakuum und Kugelrohrdestillation (140°C/0.05 Torr) ergab 6.9 g (27.0 mmol, 89%) Alkohol 31.

$^1$H-NMR ($CCl_4$, 60 MHz): $\delta$ = 2.2 (s, 6H, $-CH_3$), 2.7 (t, 2H, $-CH_2-$), 3.6 (t, 2H, $-CH_2-$), 4.7 (s, 2H, Benzyl-$CH_2$), 6.7 (s, 2H, arom. H), 7.2 (m, 5H, arom. H).

Beispiel 32:

4,6-Dimethyl-2-[2-(4-fluorphenyl)ethyl]phenol (32) (vgl. Formelschema 1, XIX)

1. Zu einer Grignard-Lösung in Ether aus 2.7 g (111 mmol) Magnesium und 19 g (108 mmol) 4-Fluorbrombenzol wurden bei Raumtemperatur 18.4 g (72 mmol) Aldehyd 30 in Ether zugetropft und 2 h bei Raumtemperatur gerührt. Unter Eiskühlung wurde mit 4n Essigsäure hydrolysiert, und mit Ether extrahiert. Die vereinigten Etherphasen wurden einmal mit Wasser und einmal mit gesättigter Kaliumhydrogencarbonatlösung gewaschen. Trocknen mit Magnesiumsulfat und Abziehen des Lösungsmittels im Vakuum ergab nach Kristallisation aus Cyclohexan 18.4 g (52.6 mmol, 73%) 2-(2-Benzyloxy-3,5-dimethyl-phenyl)-1-(4-fluorphenyl)ethanol.
Schmp.: 74°C

2. 7 g (20 mmol) Alkohol gemäß Stufe 1., 2,55 g (25 mmol) Acetanhydrid und 2,4 g (30,3 mmol) Pyridin wurden 2 h auf dem Dampfbad erhitzt. Es wurde Toluol zugegeben und bei 50°C im Vakuum abdestilliert. Der Rückstand wurde in Ether aufgenommen und zweimal mit 2n Salzsäure und einmal mit gesättigter wäßriger Kaliumhydrogencarbonatlösung gewaschen. Trocknen mit Magnesiumsulfat und Abziehen des Lösungsmittels im Vakuum ergab 7.7 g (19.6 mmol, 98%) 1-Acetoxy-2-(2-benzyloxy-3,5-dimethylphenyl)-1-(4-fluorphenyl)ethan.

3. 18,4 g (46,9 mmol) Acetat gemäß Stufe 2 wurden in Methanol mit 3 g Palladium auf Kohle (10%) unter Zusatz von 4 g Natriumacetat 6 h bei Raumtemperatur und Normaldruck hydriert. Nach beendeter Wasserstoffaufnahme wurde abgesaugt, das Filtrat eingeengt, der Rückstand in Ether aufgenommen und die etherische Lösung einmal mit Wasser gewaschen. Trocknen mit Magnesiumsulfat, Einengen im Vakuum ergab nach Kristallisation aus Cyclohexan 6.6 g (27 mmol, 57%) Phenol 32.
Schmp.: 70–72°C
$^1$H-NMR ($CCl_4$, 60 MHz): $\delta$ = 2,2 ("s", 6H, $-CH_3$), 2,8 ("s", 4H, $-CH_2-$), 4.1 (s, 1H, OH), 6.5–7.2 (m, 6H, arom. H).
MS: 244 ($M^+$)

Beispiel 33:

4.6-Dimethyl-2-[2-(4-methylphenyl)ethyl]phenol (33)

In Analogie zu Beispiel 32 wurde Beispiel 33 erhalten.

Beispiel 34:

4,6-Dimethyl-2-[2,2-bis-(4-fluorphenyl)ethyl]phenol (34) (vgl. Formelschema 1, XIX)

1. Zu 4.75 g (37.4 mmol) Oxalylchlorid in 10 ml Methylenchlorid tropfte man bei −65°C 5.8 g (74,2 mmol) Dimethylsulfoxid in 10 ml Methylenchlorid. Man ließ 30 min bei dieser Temperatur rühren und tropfte dann 9.5 g (27.1 mmol) Alkohol gemäß Beispiel 32, Stufe 1 in 20 ml Methylenchlorid zu und ließ 30 min bei −65°C rühren. Anschließend wurden 15 g (148 mmol) Triethylamin zugetropft. Es wurde zweimal mit Wasser gewaschen, mit Magnesiumsulfat getrocknet und das Lösungsmittel im Vakuum abgezogen. Der Rückstand wurde mit Cyclohexan umkristallisiert. Man erhielt 8 g (23 mmol, 85%) 2-(2-Benzyloxy-3,5-dimethylphenyl)-1-(4-fluorphenyl)ethan-1-on.
Schmp.: 99°C
$^1$H-NMR ($CCl_4$, 60 MHz): $\delta$ = 2.2 ("d", 6H, $-CH_3$), 4.0 (s, 2H, $-CH_2-$), 4.7 (s, 2H, $OCH_2-$), 6.6–7.8 (m, 11H, aromat. H).

2. 6.4 g (34 mmol) 1,2-Dibromethan wurden zu 0.85 g (35 mmol) Magnesium in Diethylether getropft und 1 h unter Rückfluß erhitzt. Mit einer Spritze wurde die Lösung zu einer Grignardlösung aus 0.85 g (35 mmol) Magnesium und 6 g (34,3 mmol) 4-Fluorbrombenzol in Diethylether getropft. Anschließend wurden 6.8 g (19.5 mmol) Keton gemäß Stufe 1 in Ether zugetropft und 4 h unter Rückfluß erhitzt. Man hydrolysierte mit Eis/HCl, extrahierte dreimal mit Ether, wusch die vereinigten Etherphasen einmal mit gesättigter wäßriger Natriumhydrogencarbonatlösung und trocknete mit Magnesiumsulfat. Abziehen des Lösungsmittels im Vakuum und Umkristallisation aus Cyclohexan ergab 7.3 g (16.4 mmol, 84%) Alkohol.
Schmp.: 119–120°C

3. 7,0 Alkohol gemäß Stufe 2 wurde in Toluol mit einer katalytischen Menge p-Toluolsulfonsäure 1 h am Wasserabscheider gekocht. Anschließend wurde die Lösung einmal mit gesättigter wäßriger Natriumhydrogencarbonatlösung gewaschen und einrotiert. Der Rückstand wurde mit Palladium auf Kohle (10%) in Methanol bei Raumtemperatur und Normaldruck hydriert. Übliche Aufarbeitung. Kugelrohrdestillation: 150–160°C, 0.05 Torr.
$^1$H-NMR ($CDCl_3$, 60 MHz): $\delta$ = 2.16 ("d", 6H, $-CH_3$), 3.25 (d, 2H, $-CH_2-$), 4.17 (s, 1H, $-OH$), 4.30 (t, 1H, Methin-H), 6.42–7.33 (m, 10H, arom. H).
MS: 338 ($M^+$)

Beispiel 35:

4,6-Dimethyl-2-[2-(4-fluorphenyl)-2-isobutylethyl]phenol (35)

In Analogie zu Beispiel 34 wurde Beispiel 35 erhalten. An Stelle der Grignard-Verbindung wurde i-Butyllithium mit Keton gemäß Beispiel 34, Stufe 1 umgesetzt.

$^1$H-NMR ($CDCl_3$, 60 MHz): δ = 0.66–1.0 (m, 6H, $-CH_3$), 1.2–1.8 (m, 3H, $-CH_2-$ und Methin-H), 2.17 ("s", 6H, $-CH_3$), 2.6–3.1 (m, 3H, $-CH_2-$ und Methin-H), 4.1 (s, 1H, OH), 6.4–7.2 (m, 6H, arom. H).
MS: 300 ($M^+$)

Beispiel 36–38:

In Analogie zu Beispiel 34 und 35 wurden ausgehend von 4.6-Dimethyl-2-(4-fluorbenzoyl)phenol (vergl. Houben-Weyl, Methoden der Organischen Chemie, Band 7/2a, 1973) die Beispiele 36, 36a, 36b, 37 und 37a gewonnen. Umsetzung des vorstehend genannten Phenols mit Cyclohexylmagnesiumbromid und anschließender Hydrogenolyse ergab das Beispiel 37b. 38 erhielt man durch Reduktion des vorstehend genannten Phenols mit Natriumborhydrid und anschließender Hydrogenolyse. Bei Verwendung von 2-Methyl-, 4-Chlor-phenol bzw. von 2-Chlor-4-methyl-phenol (G. Mazzara, M. Lamberti-Zanardi, Gazz. Chim. Ital., 399 (1986)) als Startmaterial erhielt man 36c bzw. 36d analog zu 36, bei 2,4-Dichlorphenol als Startmaterial erhielt man 36e analog zu 36.

Aus den Friedel-Crafts-Acylierungsprodukten von 2,4-Dimethylphenol, 2-Methyl-4-Chlor-phenol, 2,4-Dichlorphenol bzw. 2-Chlor-4-methyl-phenol mit Cyclohexancarbonsäurechlorid erhielt man 38a, 38b, 38c bzw. 38d analog 38. Die Beispiele 36f, 36g, 36h und 36i wurden in Analogie zu Beispiel 36 ausgehend von den oben genannten Phenolen durch Friedel-Crafts-Acylierung mit 4-Fluor-3-methyl-benzoylchlorid, Umsetzung der entstehenden Ketone mit 4-Fluor-3-methyl-phenylmagnesiumbromid und anschließender Hydrogenolyse der entstehenden Alkohole erhalten. 2-Fluor-5-bromtoluol wird bei der Umsetzung von 2-Fluortoluol mit Brom erhalten (Varma, Raman, Nilkantiah, H. Ind. Chem. Soc. 21, 112 (1944)). 4-Fluor-3-methylphenylmagnesiumbromid erhält man daraus in Analogie zu Beispiel 34-2. Umsetzung dieser Grignard-Verbindung mit einem großen Überschuß fein gepulvertem Trockeneis ergibt nach dem Umkristallisieren die reine 4-Fluor-methyl-benzoesäure, Schmp. 168°C. Bei der Reaktion dieser Säure mit Oxalkylchlorid in Toluol erhält man 4-Fluor-3-methyl-benzoylchlorid.

2-Phenyl-4,6-dichlorphenol 38e erhält man beim Einleiten von Chlor in eine Eisessig-Lösung von kommerziellem 2-Hydroxybiphenyl (I. Leupold, H. Musso, Liebigs Ann. Chem. 746, 144 (1971)).

36: 4,6-Dimethyl-2-[bis-(4-fluorphenyl)methyl]phenol
36a: 4,6-Dimethyl-2-[(4-fluorphenyl,4-methoxyphenyl)methyl]phenol
36b: 4,6-Dimethyl-2-[(4-fluorphenyl,3-trifluormethylphenyl)methyl]phenol
36c: 4-Chlor-6-methyl-2-[bis-(4-fluorphenyl)methyl]phenol
36d: 6-Chlor-4-methyl-3-[bis-(4-fluorphenyl)methyl]phenol
36e: 4,6-Dichlor-2-[bis-(4-fluorphenyl)methyl]phenol
36f: 4,6-Dimethyl-2-[bis-(4-fluor-3-methyl-phenyl)methyl]phenol
36g: 4-Chlor-6-methyl-2-[bis-(4-fluor-3-methyl-phenyl)methyl]phenol
36h: 4,6-Dichlor-2-[bis-(4-fluor-3-methyl-phenyl)methyl]phenol
36i: 6-Chlor-4-methyl-2-[bis-(4-fluor-3-methyl-phenyl)methyl]phenol
37: 4,6-Dimethyl-2-[1-(4-fluorphenyl)ethyl]phenol
37a: 4,6-Dimethyl-2-[(4-fluorphenyl,isobutyl)methyl]phenol
37b: 4,6-Dimethyl-2-[(4-fluorphenyl,cyclohexyl)methyl]phenol
38: 4,6-Dimethyl-2-(4-fluorbenzyl)phenol
38a: 4,6-Dimethyl-2-(cyclohexylmethyl)-phenol
38b: 4-Chlor-6-methyl-2-(cyclohexylmethyl)-phenol
38c: 4,6-Dichlor-2-[cyclohexyl-methyl]phenol
38d: 6-Chlor-4-methyl-2-[cyclohexyl-methyl]phenol
38e: 2-Phenyl-4,6-dichlorphenol

Herstellung der Ausgangsverbindung der Formel III

Beispiel 39:

4(R)-(t-Butyldiphenylsilyloxy)-2(R,S)-methoxy-6(S)-(4-methylphenylsulfonyloxymethyl)-3,4,5,6-tetrahydro-2H-pyran (39)
(Tetrahedron Lett. 23, 4305 (1982))

60 g (150 mmol) Alkohol 4(R)-(t-Butyldiphenylsilyloxy)-2(R,S)-methoxy-6(S)-hydroxymethyl-3,4,5,6-tetrahydro-2H-pyran (Tetrahedron Lett. 23, 4305 (1982)) wurden in 240 ml trockenem Methylenchlorid und 240 ml trockenem Pyridin gelöst und bei Raumtemperatur mit 56 g (293 mmol) p-Toluolsulfonsäurechlorid portionsweise versetzt. Man ließ 3 h bei Raumtemperatur rühren. Das Lösungsmittel wurde im Vakuum weitgehend abgezogen und der Rückstand zwischen Toluol und Wasser verteilt. Die organische Phase wurde zweimal mit Wasser und zweimal mit gesättigter wäßriger Natriumhydrogencarbonatlösung und einmal mit gesättigter wäßriger Natriumchloridlösung gewaschen. Trocknen mit Magnesiumsulfat, Abziehen des Lösungsmittels im Vakuum (zur vollständigen Entfernung des Pyridins wurde der Rückstand noch zweimal mit Toluol versetzt und erneut einrotiert) und Chromatographie aus Kieselgel ($CH_2Cl_2$) ergab 79 g (142 mmol, 95%) Tosylat 39 als farbloses Öl.
(DC: Chloroform/Essigester = 9:1, Rf = 0.63)

Beispiel 40 (III):

4(R)-(t-Butyldiphenylsilyloxy)-6(S)-jodmethyl-2(R,S)-methoxy-3,4,5,6-tetrahydro-2H-pyran (40) (Tetrahedron Lett. 23, 4305 (1982))

79 g (142 mmol) Tosylat 39 wurden in trockenem Aceton mit 43 g (286 mmol) Natriumjodid unter Rückfluß erhitzt. Bis zur vollständigen Reaktion wurde noch zusätzlich 107 g (713 mmol) Natriumjodid zugegeben, DC-Kontrolle (Chloroform).

Das Lösungsmittel wurde im Vakuum weitgehend entfernt und der Rückstand zwischen Wasser und Ether verteilt. Die Etherphase wurde nacheinander zweimal mit Wasser, einmal mit wenig Natriumsulfitlösung und einmal mit gesättigter Natriumhydrogencarbonatlösung gewaschen. Trocknen mit Magnesiumsulfat. Abziehen des Lösungsmittels im Vakuum und Chromatographie auf Kieselgel (Toluol) ergab 68 g (134 mmol, 94%) Jodid 40 (III) als farbloses Öl.

Herstellung der Endprodukte

Umsetzung von Verbindungen der Formeln II + III zu Verbindungen der Formel IV

Beispiel 41a:

6(S)-{4,6-Dimethyl-2-[3-(4-fluorphenoxy)propyl] phenoxymethyl}-3,4,5,6-tetrahydro-2(R,S)-methoxy-4(R)-(t-butyldiphenylsilyloxy)-2H-pyran (41a)

14,5 g (53 mmol) Phenol 15 und 14,6 g (105 mmol) Kaliumcarbonat wurden in 100 ml Dimethylsulfoxid vorgelegt und mit 18 g (35 mmol) Jodid 40 in 200 ml Dimethylsulfoxid versetzt. Nach 10 h bei 60°C wurde mit Wasser und Ether versetzt und die wäßrige Phase noch zweimal mit Ether extrahiert. Die vereinigten Etherphasen wurden einmal mit Wasser gewaschen. Trocknen mit Magnesiumsulfat und Abziehen des Lösungsmittels ergab nach Chromatographie auf Kieselgel (Cyclohexan/Essigester = 9:1) 18,3 g (28 mmol, 80%) Alkylierungsprodukt 41a.

MS: $C_{40}H_{49}FD_5Si$, 656 ($M^+$).

$^1$H-NMR ($CDCl_3$, 60 MHz): δ = 1,1 (s, 9H, t-Bu), 2,3 ("d", 6H, $-CH_3$), 3,5 (s, 3H, $OCH_3$), 3,7–5,1 (m, 7H, Methin-H und $-OCH_2-$), 6,7–7,8 (m, 16H, arom. H).

In Analogie zu Beispiel 41a wurden aus entsprechenden Phenolen und dem Jodid 40 die in Tabelle 4 aufgeführten Beispiele 41b–41nn erhalten.

Tabelle 4

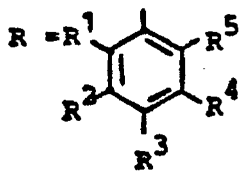

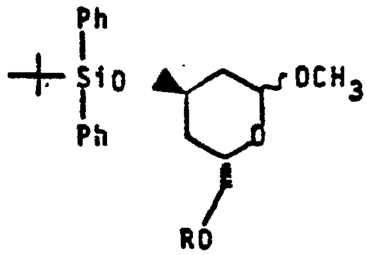

IV

| Beispiel | R | MS | Phenol (beschrieben in) |
|---|---|---|---|
| 41b | 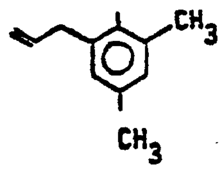 | $C_{33}H_{44}O_4Si$ 544, 512 | Beispiel 1 |
| 41c | 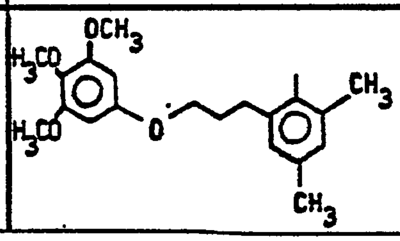 | $C_{43}H_{55}O_8Si$ 728 | Beispiel 17 |
| 41d | 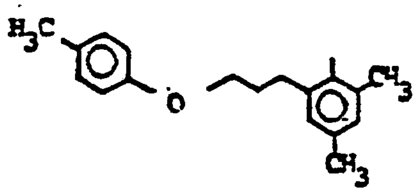 | $C_{41}H_{52}O_5Si$ 652 | Beispiel 18 |
| 41e | 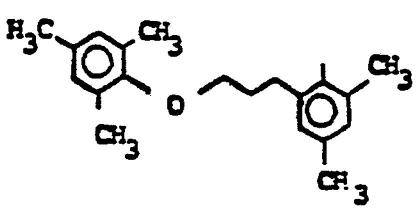 | $C_{43}H_{56}O_5Si$ 680 | Beispiel 20 |
| 41f | 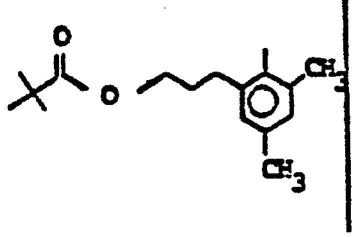 | $C_{39}H_{54}O_6Si$ 646, | Beispiel 28 |

| | | | |
|---|---|---|---|
| 41g | 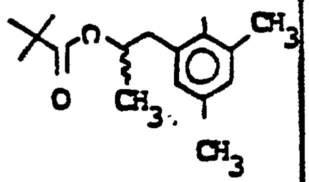 | $C_{99}H_{54}O_6Si$ 645, 614, 589 | Beispiel 29 |
| 41h | 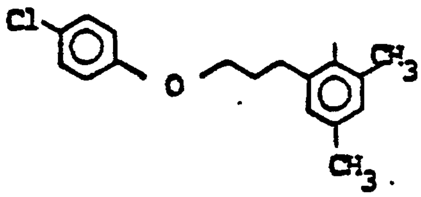 | $C_{40}H_{49}ClO_5Si$ 672 | Beispiel 19 |
| 41i | 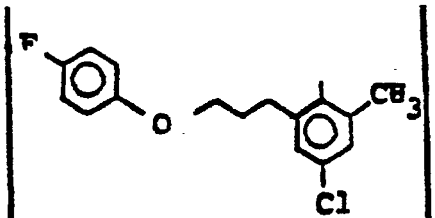 | $C_{39}H_{46}ClFO_5Si$ 676 | Beispiel 21 |
| 41j | 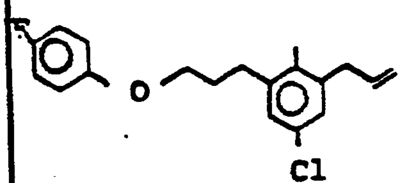 | $C_{41}H_{48}ClFO_5Si$ 702, 645 | Beispiel 4 |
| 41k | 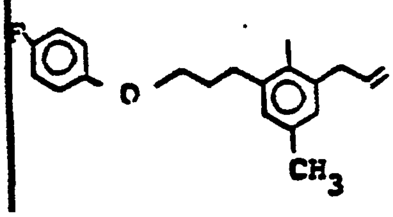 | $C_{42}H_{51}FO_5Si$ 682 | Beispiel 7 |

| | | | |
|---|---|---|---|
| 41l | 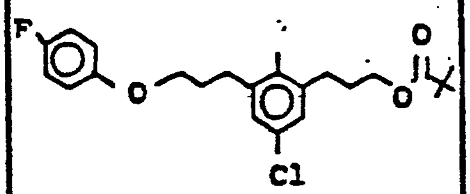 | $C_{46}H_{58}ClFO_7Si$ 747 ($M^+-C_4H_9$) | Beispiel 26 |
| 41m | 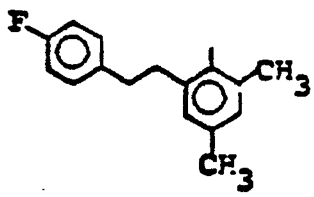 | $C_{39}H_{47}FO_4Si$ 626 | Beispiel 32 |
| 41n | 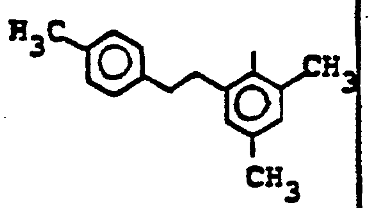 | $C_{40}H_{50}O_4Si$ 622 | Beispiel 33 |
| 41o | 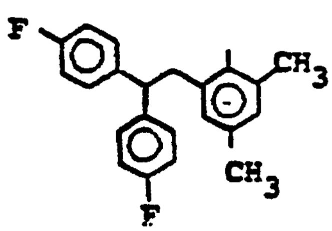 | $C_{45}H_{50}F_2O_4Si$ 720 | Beispiel 34 |
| 41p | 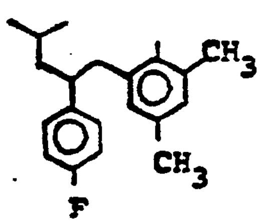 | $C_{43}H_{55}FO_4Si$ 682 | Beispiel 35 |

| | | | |
|---|---|---|---|
| 41q | 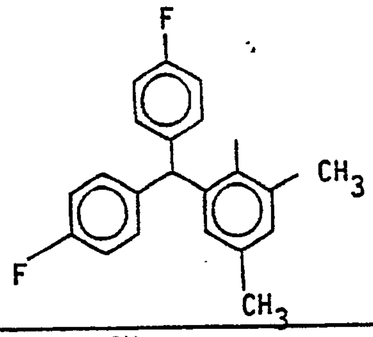 | $C_{44}H_{48}F_2O_4Si$<br>706 ($M^+$)<br>674 ($M^+$-$CH_3OH$)<br>649 ($M^+$-t-Bu)<br>617, 591, 570 | Beispiel 36 |
| 41r | 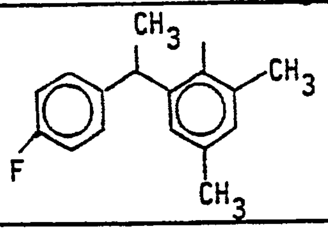 | $C_{39}H_{47}FO_4Si$<br>626 ($M^+$)<br>594 ($M^+$-$CH_3OH$)<br>569 ($M^+$-t-Bu) | Beispiel 37 |
| 41s | 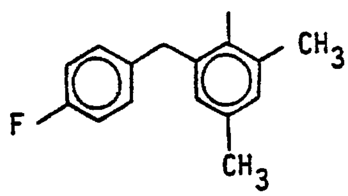 | $C_{38}H_{45}FO_4Si$<br>612 ($M^+$)<br>580 ($M^+$-$CH_3OH$)<br>555 ($M^+$-t-Bu) | Beispiel 38 |
| 41t | 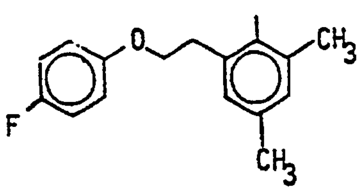 | $C_{39}H_{47}FO_5Si$<br>642 ($M^+$)<br>610 ($M^+$-$CH_3OH$)<br>585 ($M^+$-t-Bu) | Beispiel 16 |
| 41u | 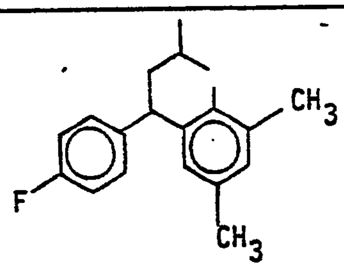 | $C_{42}H_{53}FO_4Si$<br>668 ($M^+$)<br>636 ($M^+$-$CH_3OH$)<br>611 ($M^+$-t-Bu) | Beispiel 37a |
| 41v | 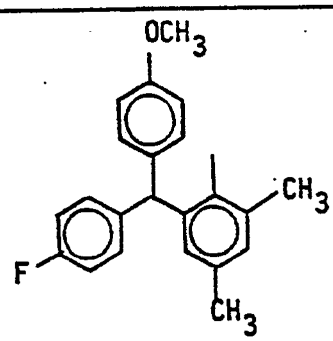 | $C_{45}H_{51}FO_5Si$<br>686 ($M^+$-$CH_3OH$)<br>661 ($M^+$-t-Bu)<br>629 | Beispiel 36a |

| | | | |
|---|---|---|---|
| 41w | 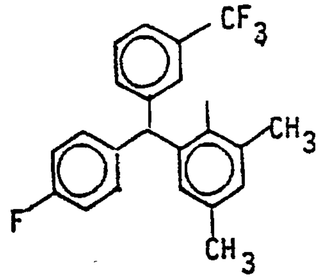 | $C_{45}H_{48}F_4O_4Si$<br>756 ($M^+$)<br>724 ($M^+$-$CH_3OH$)<br>699 ($M^+$-t-Bu) | Beispiel 36b |
| 41x | 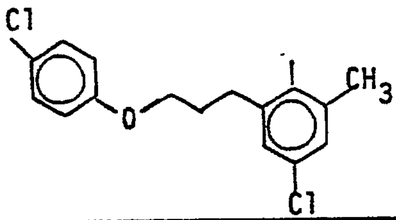 | $C_{39}H_{46}Cl_2O_5Si$<br>635 ($M^+$-t-Bu)<br>603<br>577 | Beispiel 22 |
| 41y | 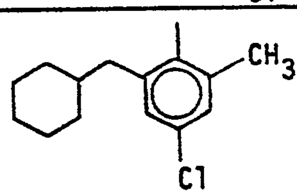 | $C_{37}H_{49}ClO_4Si$<br>620 ($M^+$)<br>563 ($M^+$-t-Bu) | Beispiel 38b |
| 41z | 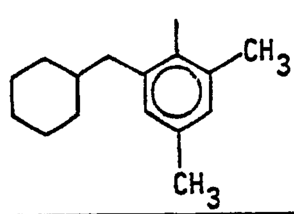 | $C_{38}H_{52}O_4Si$<br>600 ($M^+$)<br>543 ($M^+$-t-Bu) | Beispiel 38a |
| 41aa | 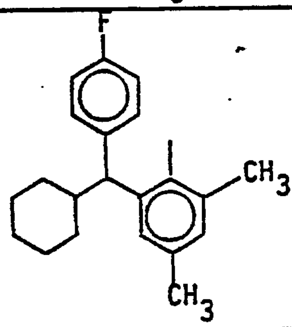 | $C_{44}H_{55}FO_4Si$<br>694 ($M^+$)<br>637 ($M^+$-t-Bu) | Beispiel 37b |
| 41bb | 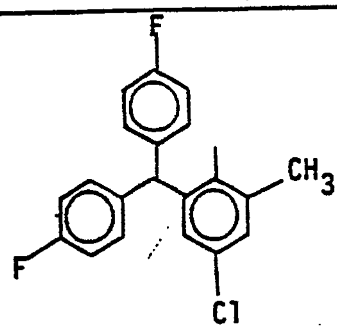 | $C_{43}H_{45}ClF_2O_4Si$<br>669 ($M^+$-t-Bu) | Beispiel 36c |

| | | | |
|---|---|---|---|
| 41cc | 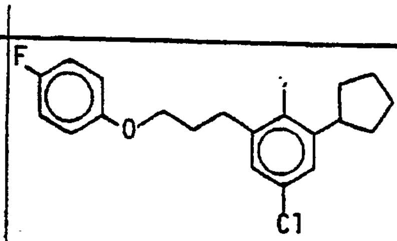 | $C_{43}H_{52}ClFO_5Si$<br>730 ($M^+$),<br>673 ($M^+$-t-Bu) | Beispiel 23 |
| 41dd | 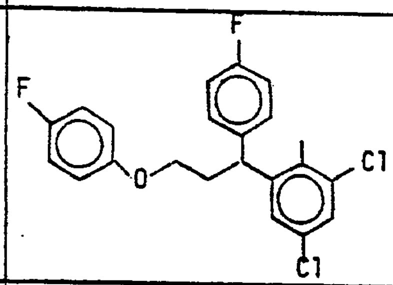 | $C_{44}H_{46}Cl_2F_2SiO_5$<br>733 ($M^+$-t-Bu),<br>675 | Beispiel 24 |
| 41ee | 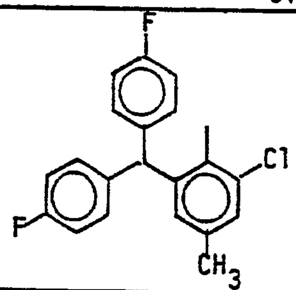 | $C_{43}H_{45}ClF_2O_4Si$<br>726 ($M^+$)<br>694 ($M^+$-$CH_3OH$)<br>689 ($M^+$-t-Bu) | Beispiel 36d |
| 41ff | 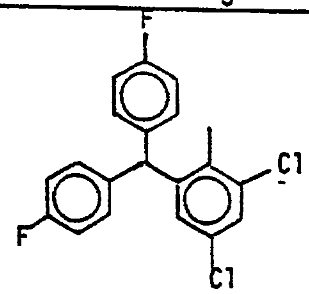 | $C_{42}H_{42}Cl_2F_2O_4Si$<br>714 ($M^+$-$CH_3OH$)<br>689 ($M^+$-t-Bu)<br>631, 610, 553, 527 | Beispiel 36e |
| 41gg | 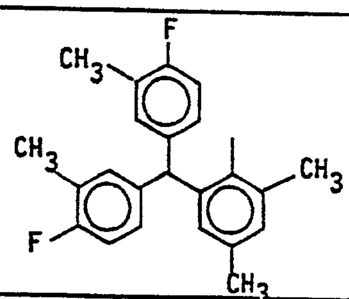 | $C_{46}H_{52}F_2O_4Si$<br>734 ($M^+$)<br>702 ($M^+$-$CH_3OH$)<br>677 ($M^+$-t-Bu) | Beispiel 36f |
| 41hh | 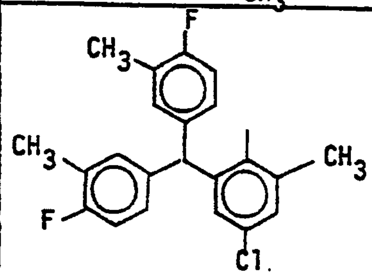 | $C_{45}H_{49}ClF_2O_4Si$<br>754 ($M^+$)<br>697 ($M^+$-t-Bu) | Beispiel 36g |

| | | | |
|---|---|---|---|
| 41 ii | 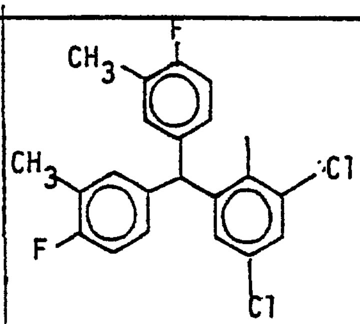 | $C_{44}H_{46}Cl_2F_2O_4Si$<br>774 ($M^+$)<br>742 ($M^+$-$CH_3OH$)<br>717 ($M^+$-t-Bu) | Beispiel 36h |
| 41jj | 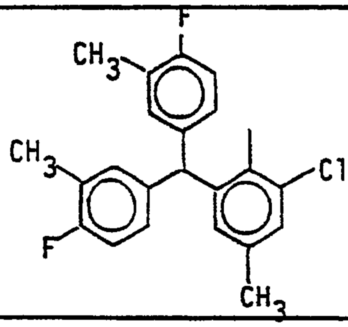 | $C_{45}H_{49}ClF_2O_4Si$<br>754 ($M^+$)<br>697 ($M^+$-t-Bu) | Beispiel 36i |
| 41kk | 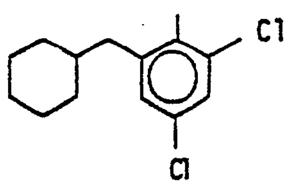 | $C_{36}H_{46}Cl_2O_4Si$<br>583 ($M^+$-t-Bu)<br>525 ($M^+$-$CH_3OH$– 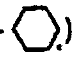) | Beispiel 38c |
| 41ll | 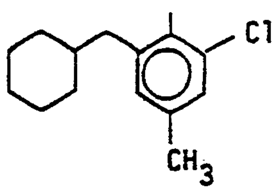 | $C_{37}H_{49}ClO_4Si$<br>620 ($M^+$)<br>543 ($M^+$-t-Bu) | Beispiel 38d |
| 41mm | 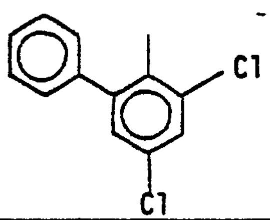 | $C_{35}H_{38}Cl_2O_4Si$<br>563 ($M^+$-t-Bu)<br>505 | Beispiel 38e |
| 41nn | 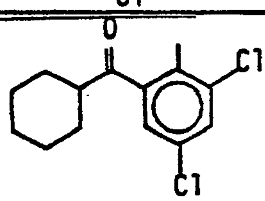 | $C_{36}H_{44}Cl_2O_5Si$<br>597 ($M^+$-t-Bu)<br>565 ($M^+$-t-Bu-$CH_3OH$)<br>537 (565-CO) | Beispiel 38c |

Umsetzung von Verbindungen der Formel IV zu Verbindungen der Formel V.

Beispiel 42a:

6(S)-{4,6-Dimethyl-2-[3-(4-fluorphenoxy)propyl]phenoxymethyl}-3,4,5,6-tetrahydro-2(R,S)-hydroxy-4(R)-(t-butyldiphenylsilyloxy)-2H-pyran (42a)

1,4 g (2,13 mmol) Alkylierungsprodukt 41a wurden in 75 ml Tetrahydrofuran, 75 ml Wasser und 105 ml Eisessig 18 h zwischen 70 und 80°C gehalten. Das Lösungsmittel wurde im Vakuum abgezogen und der Rückstand zur Entfernung noch vorhandener Essigsäurereste mit Toluol versetzt und erneut einrotiert. Chromatographie auf Kieselgel (Cyclohexan/Essigester = 8:2) ergab 950 mg (1,48 mmol, 70%) Hydrolyseprodukt 42a.
MS: $C_{39}H_{47}FO_4Si$, 642 ($M^+$), 624 ($M^+-H_2O$)
DC: Rf = 0,18 (Cyclohexan/Essigester = 5:1).
In Analogie zu Beispiel 42a wurden aus den Beispielen 41b–42nn die in Tabelle 5 aufgeführten Beispiele 42b–42nn erhalten.

Umsetzung von Verbindungen der Formel V zu Verbindungen der Formel VI

Beispiel 43a:

(6(S)-{4,6-Dimethyl-2-[3-(4-fluorphenoxy)propyl] phenoxymethyl}-3,4,5,6-tetrahydro-4(R)-(t-butyldiphenylsilyloxy)-2H-on (43a)

9,66 g (96,6 mmol) Chromtrioxid wurden in 250 ml trockenem Methylenchlorid vorgelegt und bei Raumtemp. 15,3 g (193.3 mmol) Pyridin zugetropft. Man ließ 20 min bei Raumtemp. rühren und tropfte dann bei Raumtemp. 6.2 g (9.66 mmol) Hydrolyseprodukt 42a in 250 ml Methylenchlorid zu. Nach 30 min bei Raumtemp. wurde mit Methylenchlorid verdünnt, über Celite abgesaugt und der Rückstand gründlich mit Methylenchlorid ausgewaschen. Das Filtrat wurde im Vakuum eingeengt und der Rückstand mit Cyclohexan/Essigester = 1:1 aufgenommen und erneut über Celite abgesaugt. Einengen im Vakuum und Chromatographie auf Kieselgel (Cyclohexan/Essigester = 8:2) ergab 5,34 g (8,34 mmol, 86%) Lakton 43a.
MS: $C_{39}H_{45}FO_5Si$, 640 ($M^+$)
$^1$H-NMR ($CDCl_3$, 60 MHz): $\delta$ = 1,1 (s, 9H, t-Bu), 1,8–2,9 (m, 14H, $-CH_3$ und $-CH_2-$), 3,9 (m, 4H, $-OCH_2-$), 4,4 und 5,1 (jeweils m, je 1H, Methin-H), 6,7–7,8 (m, 16H, arom. H).
In Analogie zu Beispiel 43a wurden aus den Beispielen 42b–42nn die in Tabelle 5 aufgeführten Beispiele 43b–43nn hergestellt.

Umsetzung von Verbindungen der Formel VI zu Verbindungen der Formel I

Beispiel 44a:

6(S)-{4,6-Dimethyl-2-[3-(4-fluorphenoxy)propyl]phenoxymethyl}-3,4,5,6-tetrahydro-4(R)-hydroxy-2H-pyran-2-on (44a)

950 mg (1,48 mmol) Silylverbindung 43a wurden bei Raumtemp. in 60 ml Tetrahydrofuran vorgelegt und mit 355 mg (5,92 mmol) Eisessig versetzt. Anschließend wurden 1,4 g (4,44 mmol) Tetrabutylammoniumfluorid-Trihydrat zugegeben. Nach 16 h Raumtemp. wurde das Lösungsmittel weitgehend entfernt und der Rückstand zwischen Ether und Wasser verteilt. Die wäßrige Phase wurde noch einmal mit Ether extrahiert. Die vereinigten Etherphasen wusch man einmal mit Wasser. Trocknen mit Magnesiumsulfat, Abziehen des Lösungsmittels im Vakuum und Chromatographie auf Kieselgel (Cyclohexan/Essigester = 1:1) ergab 470 mg (1,17 mmol, 79%) Hydroxylakton 44a.
MS: $C_{23}H_{27}FO_5$, 402 ($M^+$), 384 ($M^+-H_2O$).
$^1$H-NMR ($CDCl_3$, 270 MHz): $\delta$ = 1,82 (breit, 1H, OH), 2,00–2,20 (m, 4H, Methylen-H), 2,25 und 2,27 (jeweils s, je 3H, $-CH_3$), 2,68–2,83 (m, 4H, Ar–$CH_2$ und $-CH_2CO$), 3,88–4,01 (m, 4H, $-OCH_2$), 4,50 und 5,00 (jeweils m, je 1H, Methin-H), 6,80–7,10 (m, 6H, arom. H).
In Analogie zu Beispiel 44a wurden aus 43b–43nn die in Tabelle 5 aufgeführten Beispiele 44b–44nn erhalten.

**Tabelle 5:** charakteristische Daten ($^1$H-NMR in $CDCl_3$, $\delta$ ; DC auf Kieselgel, Rf)

| | 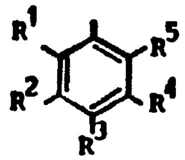 = R | 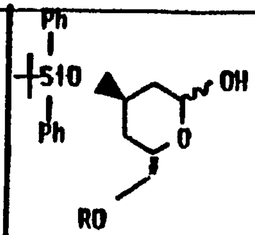 42 | 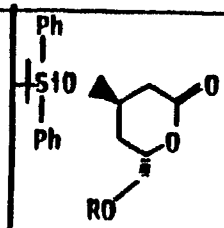 43 | 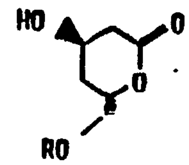 44 |
|---|---|---|---|---|
| b | 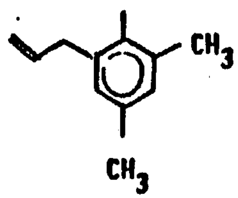 | DC : 0,40 (1) | MS : $C_{33}H_{40}O_4Si$ 528,471 | MS : $C_{17}H_{22}O_4$. 290, 272<br>$^1$H-NMR (270 MHz) : 2,0-2,3 (m, 8H, $-CH_3$,$-CH_2-$), 2,65-2,85 (ABX, 2H, $-CH_2-$), 3,38 (d, 2H,$-CH_2-$), 3,87-4,0 (ABX, 2H, $-OCH_2-$), 4,52 (m, 1H, Methin-H), 4,95 - 5,10 (m, 3H, Methin-H und allyl-H), 6,88-6,01 (m, 1H, Methin-H), 6,82 und 6,85 (jeweils s, 2H, arom. H) |
| c | 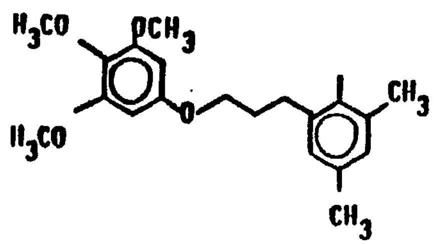 | DC : 0,29 (1)<br>MS : $C_{42}H_{54}O_8Si$ 714, 696 | MS : $C_{42}H_{52}O_8Si$ 712 | MS : $C_{26}H_{34}O_8$, 474, 456<br>$^1$H-NMR (270 MHz) : 2,0-2,2 (m, 4H, $-CH_2-$), 2,28 ("s", 6H, $-CH_3$), 2,6-2,8 (m, 4H, Ar-$CH_2$-), 3,80-4,01 (m, 13H, $OCH_3$, $-OCH_2-$),4,5 (m, 1H, Methin-H), 5,0 (m, 1H, Methin-H), 6,20 und 6,88 (jeweils s, je 2H, arom. H) |

| | | | | |
|---|---|---|---|---|
| d | 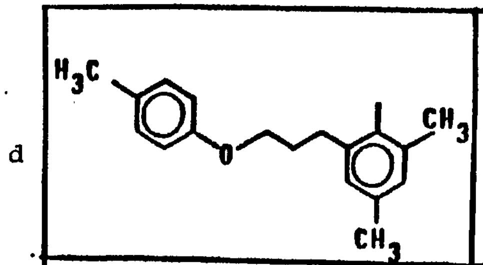 | DC: 0.40 (1) | MS: $C_{40}H_{48}O_5Si$ 636 | MS: $C_{24}H_{30}O_5$ 398, 380 |
| e | 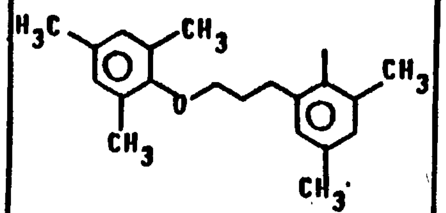 | DC: 0.35 (2) | MS: $C_{42}H_{52}O_5Si$ 664 | MS: $C_{26}H_{34}O_5$ 426, 408 |
| f | 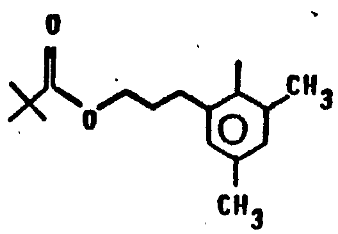 | DC : 0,49 (1) MS : $C_{38}H_{52}O_6Si$ 632 | MS : $C_{38}H_{50}O_6Si$ 630 | MS : $C_{22}H_{32}O_6$, 392 $^1H$-NMR (60MHz) : 1,5 (s, 9H, t-Bu), 1,5-2,8 (m, 14H, $-CH_3$, $-CH_2$), 3,8-4,3 (m, 4H, $-OCH_2-$), 4,5 und 5,0 (jeweils m, je 1H, Methin-H), 6,8 ("s", 2H, arom. H) |

| | | | | |
|---|---|---|---|---|
| g | 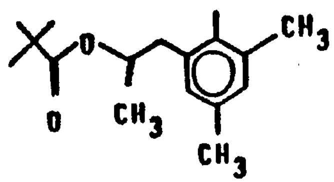 | DC: 0,51 (1) | MS: $C_{38}H_{50}O_6Si$<br>630,573 | MS: $C_{22}H_{32}O_6$, 392<br>$^1$H-NMR (60MHz) : 1,0-3,0(m, 24H, t-Bu,-$CH_3$,-$CH_2$), 4,0 (m, 2H, -$OCH_2$-) 4,5 (m, 1H, Methin-H), 5,1 (m, 2H, Methin-H), 6,8 ("s", 2H, arom. H) |
| f | 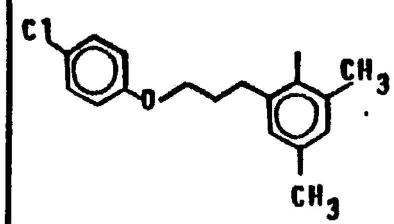 | DC: 0.42 (1) | MS: $C_{39}H_{45}ClO_5Si$<br>656 | MS: $C_{23}H_{27}ClO_5$<br>418, 400 |

| | | | | |
|---|---|---|---|---|
| i | 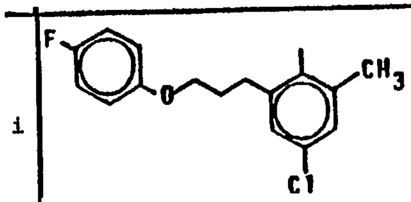 | DC: 0.34 (3)<br>MS: $C_{38}H_{44}ClFO_5Si$<br>635 ($M^+$-t.-Bu)<br>309,267 | DC: 0.38 (1)<br>MS: $C_{38}H_{42}ClFO_5Si$<br>660,<br>603 ($M^+$-t.-Bu),<br>309,293,267 | MS: $C_{22}H_{24}ClFO_5$, 422, 404, 311, 293<br>$^1$H-NMR (270 MHz): 1,7-2,0 (s,br.,1H,OH), 2,03-2,20 (m,4H,Methylen-H), 2,28 (s, 3H,$CH_3$), 2,69-2,82 (m,4H,Methylen-H), 3,88-4,02 (m,4H,Methylen-H), 4,50 (qui, 1H,Methin-H), 5,00 (m,1H,Methin-H), 6,80-6,87 (m,2H,arom.H), 6,93-7,05 (m, 4H,arom.H) |
| j | 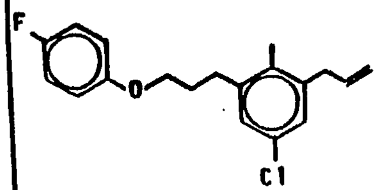 | DC: 0,38 (2) | MS: $C_{40}H_{44}ClFO_5Si$<br>686, 629 | MS : $C_{24}H_{26}ClFO_5$: 448<br>$^1$HMR (270MHz) : 2,00-2,2 (m, 4H, $-CH_2-$), 2,65-2,84 (m, 4H, $-CH_2-$), 3,40 (d, 2H, Ar-$CH_2$), 3,88-4,00 (m, 4H, $-OCH_2-$), 4,5 (m, 1H, Methin-H), 4,95-5,16 (m, 3H, allyl.H u. Methin-H), 5,83-6,01 (m, 1H, allyl.H), 6,80-7,09 (m, 6H, arom. H) |
| k | 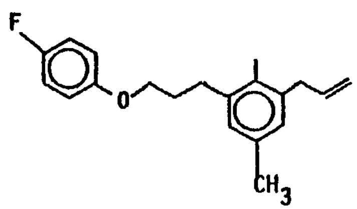 | DC: 0,22 (3)<br>MS: $C_{41}H_{49}FO_5Si$<br>(668), 650 ($M^+$-$H_2O$),<br>611 ($M^+$-t-Bu),<br>593 ($M^+$-t-Bu-$H_2O$) | MS: $C_{41}H_{47}FO_5Si$<br>666 ($M^+$)<br>609 ($M^+$-t-Bu)<br>225, 199 | MS: $C_{25}H_{29}FO_5$, 428<br>$^1$H-NMR (270 MHz): 2.0-2.20 (m, 4H, Methylen-H); 2,25 (s, 3H, $-CH_3$); 2,65 - 2,82 (m, 4H, Methylen-H); 3,39 (d, 2H, allyl. Methylen-H); 3,85 - 4,00 (m, 4H, $-OCH_2-$); 4,5 (m, 1H, Methin-H); 4,93 - 5,11 (m, 3H, Methin-H und olefin.H); 5,95 (m, 1H, olefin H); 6,80 - 7,00 (m, 6H, arom. H) |

| | | | | |
|---|---|---|---|---|
| l | 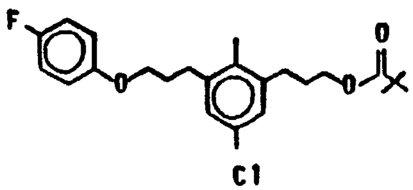 | DC: 0,46 (2) | MS: $C_{45}H_{54}ClFO_7Si$ 731, 629 | MS: $C_{29}H_{36}ClFO_7$ : 550, 532, 448 $^1$H-NMR(270 MHz) : 1,2 (s, 9H, t-Bu), 1,90-2,18 (m, 6H, $-CH_2$), 2,60-2,81 (m, 6H, $-CH_2-$), 3,91-4,16 (m, 6H, $-OCH_2-$), 4,50 und 5,0 jeweils m, je 1H, Methin-H), 6,80-7,08 (m, 6H, arom. H). |
| m | 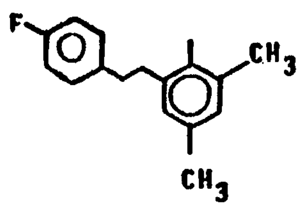 | DC: 0.18 (4) MS: $C_{38}H_{45}FO_4Si$ 612 ($M^+$) 594 | MS: $C_{38}H_{43}FO_4Si$ 610,553 | MS: $C_{22}H_{25}FO_4$: 372,354 $^1$H-NMR(270 MHz,$CDCl_3$):2,00-2,25 (m,2H,$-CH_2-$),2,25("d",6H,$-CH_3$),2.61-2.85(ABX,2H,$-CH_2-$),2,85("s",4H,$-CH_2CH_2-$), 3,84 und 3,94(jeweils dd,je1H,$-OCH_2-$), 4.52(m,1H,Methin H),5,00(m,1H,Methin-H) 6,81 und 6,86(jeweils s,je 1H,arom.H), 6,90-7,20(m,4H,arom.H) |
| n | 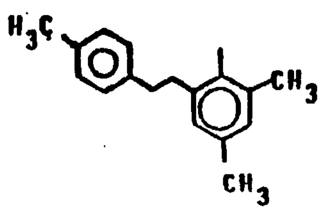 | DC: 0.22 (4) | MS: $C_{39}H_{46}O_4Si$ 606 | MS: $C_{23}H_{28}O_4$ 368, 350 |

| | | | |
|---|---|---|---|
| 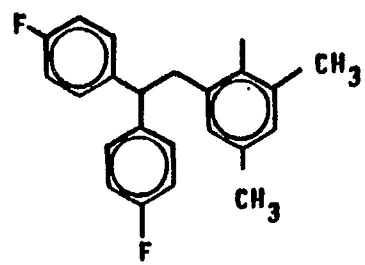 | DC: 0.15 (4) | MS: $C_{44}H_{46}F_2O_4Si$ 704, 702 647 | : $C_{28}H_{28}F_2O_4$, 466, 448 $^1$H-NMR (270 MHz): 1.90-2.10 (m, 2H, $-CH_2-$), 2.11 und 2.22 (jeweils s, je 3H, $-CH_3$), 2.65 (d, 2H, $ArCH_2-$), 3.15-3.32 (m, 2H, $-CH_2-$), 3.80 (ABX, 2H, $OCH_2$), 4.25 (t, 1H, Methin-H), 4.40 (m, 1H, Methin-H), 4.92 (m, 1H, Methin-H), 6.52 und 6.80 (jeweils s, 1H, arom.H), 6.85-7.20 (m, 8H, arom.H) |
| 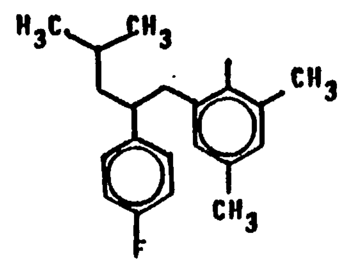 | DC: 0.18 (4) | MS: $C_{42}H_{51}FO_4Si$ 666, 609 | MS: $C_{26}H_{33}FO_4$, 428, 410 $^1$H-NMR (270 MHz): 0,75-0.90 (dd, 6H, $-CH_3$), 1.20-1.70 (m, 3H, $-CH_2-$ und Methin-H), 2.01-2.30 (m, 8H, $-CH_3$ und $-CH_2-$) 2.60-3.00 (m, 5H, $-CH_2-$ und Methin-H), 3.70-3.95 (m, 2H, $OCH_2$), 4.51 (m, 1H, Methin-H), 5.00 (m, 1H, Methin-H), 6.55 und 6.80 (jeweils s, je 1H, arom.H), 6.85-7.10 (m, 4H, arom.H). |
| 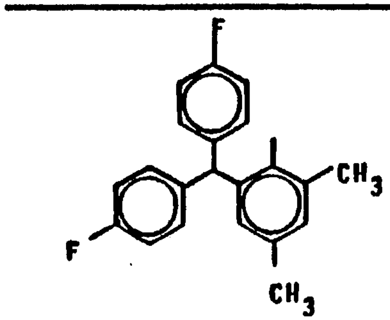 | DC: 0,13 (4) MS: $C_{43}H_{46}F_2O_4Si$ 692, 674, 617, 570 395 | DC: 0.25 (4) MS: $C_{43}H_{44}F_2O_4Si$ 690, 633, 434, 405 | MS: $C_{27}H_{26}F_2O_4$, 452, 434, 321, 227 $^1$H-NMR (270 MHz): 1,60 (s, br., 1H, OH), 1,84-2,10 (m, 2H, Methylen-H), 2,19 (s, 3H, $CH_3$), 2,28 (s, 3H, $CH_3$), 2,57-2,74 (AB von ABX, 2H, Methylen-H), 3,62-3,76 (AB von ABX, 2H, Methylen-H), 4,43 (qui, 1H, Methin-H), 4,80-4,90 (m, 1H, Methin-H), 5,90 (s, 1H, Methin-H), 6,48 (d, 1H, arom.H), 6,89 (d, 1H, arom.H), 6,90-7,17 (m, 8H, arom.H) |

| | | | | |
|---|---|---|---|---|
| r | 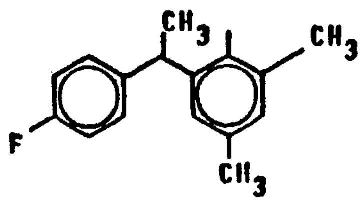 | DC: 0,06 (5)<br>MS: $C_{38}H_{45}FO_4Si$<br>612, 594<br>395, 361 | DC: 0.59 (6)<br>MS: $C_{38}H_{43}FO_4Si$<br>610, 553,<br>437, 405 | MS: $C_{22}H_{25}FO_4$, 372, 354, 339, 241<br>$^1$H-NMR (270 MHz): 1,55(d,3H,$CH_3$), 1,86(s,br.,1H, OH), 1,95-2,20(m,2H,Methylen-H), 2,27(s,6H,2x $CH_3$), 2,60-2,80(AB von ABX,2H,Methylen-H), 3,65-3,92 (AB von ABX,2H,Methylen-H), 4,45-4,56(qua + qui, 2H,Methin-H), 4,90-5,00(m,1H,Methin-H), 6,84-7,00 (m,4H,arom.H), 7,13-7,25(m,2H,arom.H) |
| s | 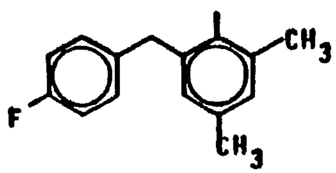 | DC: 0.22 (3) | MS: $C_{37}H_{41}O_4FSi$;<br>596<br>539 | MS: $C_{21}H_{23}FO_4$, 358<br>$^1$H-NMR (270 MHz): 1.96-2.20 (m,2H,Methylen-H), 2,23 und 2,27 (jeweils s, je 3H,-$CH_3$), 2,60-2,80 (ABX,2H, Methylen-H), 3,78-3,92 (ABX,2H,-$OCH_2$-), 3,95 ("s", 2H,-$CH_2$-), 4,48 (m,1H,Methin-H), 4.95 (m,1H,Methin-H), 6.73 und 6.89 (jeweils s, je 1H,arom.H), 6.90-7.20 (m,4H,arom.H) |
| t | 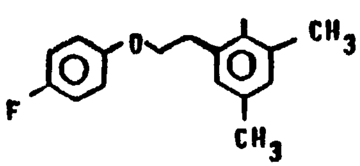 | DC: 0.24 (1)<br>MS: $C_{38}H_{45}FO_5Si$<br>628<br>610 | MS: $C_{38}H_{43}FO_5Si$<br>626<br>569 | MS: $C_{22}H_{25}FO_5$, 388, 370<br>$^1$H-NMR (270 MHz): 2.0-2.25 (m,2H,Methylen-H), 2.27 ("d", zus. 6H,-$CH_3$), 2.61-2.80 (ABX,2H,Methylen-H), 2.96-3.15 (m,2H,Methylen-H), 3.90-4.05 (ABX,2H, -$CH_2O$-), 4,13 (t,2H,-$OCH_2$-), 4,50 (m,1H,Methin-H), 5,02 (m,1H,Methin-H), 6,80-7,00 (m,6H,arom.H) |
| u | 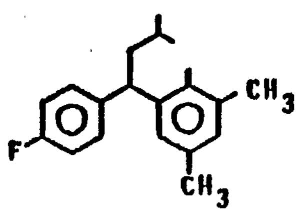 | DC: 0.34 (6)<br>MS: $C_{41}H_{51}FO_4Si$<br>654, 636<br>229, 199 | DC: 0.55 (6)<br>MS: $C_{41}H_{49}FO_4Si$<br>652,595,405,<br>339,311,225,<br>199 | MS: $C_{25}H_{31}FO_4$, 414, 357, 229<br>$^1$H-NMR (270 MHz): 0.90 (2xd, 6H, 2x$CH_3$ von i-Bu), 1,43 (sept., 1H, CH von i-Bu), 1,7-1,88 (m, 4H, 3 Methylen-H und OH), 2,02-2,25 (m, 1H, Methylen-H), 2.24 (s, 3H, $CH_3$), 2.28 (s, 3H,$CH_3$), 2.63-2.82 (AB von ABX, 2H, Methylen-H), 3.6-3.9 (m, 2H, Methylen-H), 4.44 (t, 1H, Methin-H), 4.50 (qui,1H, Methin-H), 4,97 (m, 1H, Methin-H), 6.83-6.98 (m, 4H, arom.H), 7,17-7.30 (m, 2H, arom.H) |

| | | | | |
|---|---|---|---|---|
| v | 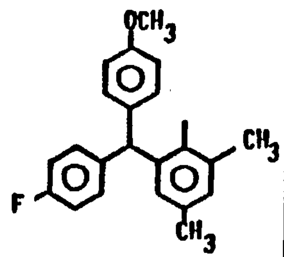 | DC: 0.23 (3)<br>MC: $C_{44}H_{49}FSiO_5$ 686($M^+$-$H_2O$), 629, 334 | DC: 0,27 (3)<br>MS: $C_{44}H_{47}FSiO_5$ 702, 645 ($M^+$-$C_4H_9$), 537, 417 | MS: $C_{28}H_{29}FO_5$, 464, 446, 334, 303<br>$^1$H-NMR (270 MHz): 1.80-2.10 (m, 3H, Methylen-H und OH), 2.18 (s, 3H, $CH_3$), 2.27 (s, 3H, $CH_3$), 2.57-2.75 (AB von ABX, 2H, Methylen -H), 3.60-3.75 (AB von ABX, 2H, Methylen-H), 3.79 (s, 3H, $OCH_3$), 4.42 (qui, 1H, Methin-H), 4.84 (m, 1H, Methin-H), 5.87 (s, 1H, Methin-H), 6.50 (d, 1H, arom. H), 6.77-7.07 (m, 9H, arom. H) |
| w | 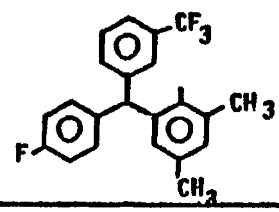 | DC: 0.30 (3)<br>MS: $C_{44}H_{46}F_4SiO_4$ 742, 725 667, 619 | DC: 0.33 (3)<br>MS: $C_{44}H_{44}F_4SiO_4$ 740, 683 ($M^+$ -$C_4H_9$), 405 | MS: $C_{28}H_{26}F_4O_4$, 502, 372, 371, 303<br>$^1$H-NMR (270 MHz): 1.67 (s, br., 1H, OH), 1.85-2.08 (m, 2H, Methylen-H), 2.20 (s, 3H, $CH_3$), 2.28 (s, 3H, $CH_3$), 2,67 (m, 2H, Methylen-H), 3.64-3,78 (m, 2H, Methylen-H), 4,43 (qui, 1H, Methin-H), 4.82-4.92 (m, 1H, Methin-H), 5.98 (2 x s, 1H, Methin-H, 2 Diast.), 6.46 (d, 1H, arom. H), 6.90-7.10 (m, 5H, arom. H), 7.24 - 7.50 (m, 4H, arom. H) |
| x | 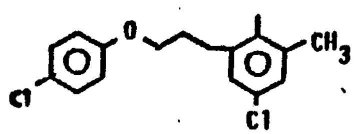 | DC: 0.34 (3)<br>MS:$C_{38}H_{44}Cl_2O_5Si$ 422/424 ($M^+$-tert.Bu-di-Ph-SiOH), 199 | DC: 0.38 (1)<br>MS:$C_{38}H_{42}Cl_2O_5Si$ 676, 619 ($M^+$-tert.-Bu), 491, 309 | MS: $C_{22}H_{24}Cl_2O_5$, 438, 420, 310, 293<br>$^1$H-NMR (270 MHz):1.82 (s, br., 1H, OH), 2.03-2.24 (m, 4H, Methylen-H), 2.27 (s, 3H, $CH_3$), 2.69-2.81 (m, 4H, Methylen-H), 3.87 -4.00 (m, 4H, Methylen-H), 4.50 (qui, 1H, Methin-H), 5.00 (m, 1H, Methin-H), 6.82 (AB, 2H, arom. H), 7.02 (s, 2H, arom. H), 7.22 (AB, 2H, arom. H) |
| y | 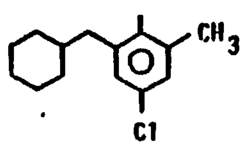 | DC: 0.07 (4) | DC: 0.42 (1)<br>$^1$H-NMR (60MHz): 1.1 (s,9H,tert. Bu), 1.2-2.6 (m, 17H), 2.2 (s,3H, $CH_3$), 3.8 (d, 2H, Methylen-H), 4.4 (qui,1H, Methin-H), 5.1 (m, 1H, Methin-H), 6.9-7.8 (m,12H,arom.H) | MS : $C_{20}H_{27}O_4Cl$, 366, 284, 238<br>$^1$H-NMR (60 MHz): 0.8-2.0 (m,12H, Cyclohexyl-H und OH), 2.0-2.2 (d,2H,Methylen-H), 2.3 (s,3H,$CH_3$), 2.4-2.6 (d, 2H, Methylen-H), 2.75 (d, 2H, Methylen-H), 3.9 (d, 2H, Methylen-H), 4.55 (qui, 1H, Methin-H), 5.0 (m, 1H, Methin-H), 6.95 (s, 2H, arom. H) |

| | | | |
|---|---|---|---|
| 2 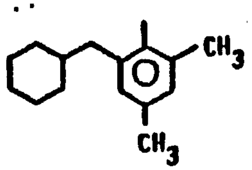 | DC: 0.34 (3) | DC: 0.38 (1) $^1$H-NMR (270MHz): 1.08 (s,9H,tert. Bu), 0.8-1.2 und 1.5-1.7 (m,11H, Cyclohexyl), 1.95-2.02 (m,2H, Methylen-H),2.22 und 2.25 (2xs,6H, 2x $CH_3$), 2.33-2.46 (m,2H, Methylen-H), 2.55 (AB von ABX,2H, Methylen-H),3.88 (AB von ABX,2H, Methylen-H),4.42 (qui,1H, Methin -H), 5.10 (m,1H, Methin-H), 6.77 (s,1H.arom.H), 6.82 (s,1H,arom. H), 7.35-7.50 (m,6H,arom.H), 7.62-7.75 (m,4H, arom. H) | MS: $C_{21}H_{30}O_4$, 346, 328, 264, 218 $^1$H-NMR (270 MHz): 0.85-1.26 (m, 5H, Cyclohexyl-H), 150-1.75 (m, 7 H, Cyclohexyl-H und OH), 2.10-2.30 (m, 2H, Methylen-H), 2.26 und 2.25 (2 x s, 6H, 2 x $CH_3$), 2.43 (AB von ABX, 2H, Methylen-H), 2.75 (AB von ABX, 2H, Methylen-H), 3.93 (AB von ABX, 2 H, Methylen-H), 4.56 (qui, 1H,Methin-H), 5.00 (m, 1H, Methin-H), 6.78 (s, 1H, arom. H), 6.78 (s, 1H, arom. H), 6.82 (s, 1H, arom. H) |

| | | | | |
|---|---|---|---|---|
| aa | 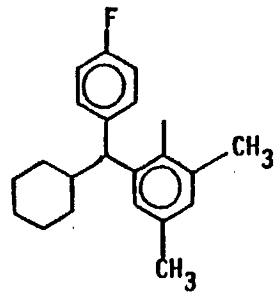 | DC: 0,24 (4)<br>MS: $C_{43}H_{53}FO_4Si$<br>680, 662, 424,<br>341, 310, 229 | DC: 0,35 (4)<br>MS: $C_{43}H_{51}FO_4Si$<br>678, 621, 595, 311,<br>$^1$H-NMR (60MHz): 1,1 (s, 9H, tert. Bu); 1,0-2,2 (m, 13H, 11 Cyclohexyl-H u. 2 Methylen-H); 2,1 (s, 3H, $CH_3$); 2,3 (s, 3H, $CH_3$); 2,6 (m, 2H, Methlyen-H); 3,8 (t, 2H, Methylen-H); 4,0 (1H-Methin-H); 4,4 (qui, 1H, Methin-H); 5,1 (m, 1H, Methin -H); 6,7-7,7 (m, 16H, arom. H) | DC: 0,20 (7)<br>MS: $C_{27}H_{33}FO_4$ 440, 422, 357, 339, 229, 227, 135, 109<br>$^1$H-NMR (270 MHz): 0,7-2,0 (m, 1H, Cyclohexyl-H); 2,1-2,35 (m, 2H, Methylen-H); 2,20 (2 x s, 3H, $CH_3$ 2 Diastereomere); 2,28 (s, 3H, $CH_3$); 2,78 (AB von ABX, 2H, Methylen-H); 3,80 (AB von ABX, 2H, Methylen-H); 3,93 und 3,98 (2 x d, 2 x J = 6Hz, 1H, Methin-H, 2 Diastereomere); 4,57 (qui, J = 3,5Hz, 1H, Methin-H) 5.05 (m, 1H, Methin-H); 6,8 (s, 1H, arom. H); 6,9 (AB, 2H, arom.-H); 7,07 (s, 1H, arom.-H); 7,2-7,33 (m, 2H, arom.-H) |
| bb | 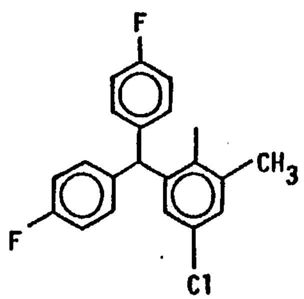 | DC: 0,17 (3)<br>MS: $C_{42}H_{43}ClF_2O_4Si$<br>(712), 637 ($M^+$-$H_2$O-t-Bu), 438 ($M^+$-$H_2$O-t-BuSiP$h_2$OH), 342, 247, 199<br>$^1$H-NMR (60 MHz): 1,1 (s, 9H, tert. Bu); 1,3-2.0 (m, 5H, 2 x Methylen-H und OH); 2,3 (s, 3H, $CH_2$); 3,4-3,6 (m, 2H, Methylen-H); 4,2 - 4,8 (m, 2H, 2 x Methin-H); 5,3 (m, 1H, Methin-H); 5,9 (s, 1H, Methin-H); 6,6-7,8 (m, 20H, arom.-H) , | DC: 0,21 (4)<br>MS: $C_{42}H_{41}ClF_2O_4Si$<br>710, 653, 454, 405<br>$^1$H-NMR (60 MHz): 1,1 (s, 9H, tert.Bu); 1,5-1,9 (m, 2H, Methylen-H); 2,3 (s, 3H, $CH_3$); 2,4-2,6 (m, 2H, Methylen-H); 3,5-3,7 (m, 2H, Methylen-H); 4,3-4,5 (qui, 1H, Methin-H); 4,7-5,2 (m, 1H, Methin-H); 5,8 (s, 1H, Methin-H), 6,6 (d, 1H, arom.-H); 6,8-7,7 (m, 19H, arom.-H) | DC: 0,10 (7)<br>MS: $C_{26}H_{23}ClF_2O_4$ 472, 454, 341, 247<br>$^1$H-NMR (270 MHz): 1,5-1,7 (s, breit, 1H, OH); 1,87 (dt, $J_{gem.}$ = 14Hz, $J_t$ ~ 3,5Hz, 1H, Methylen-H); 2,00 (ddd, $J_{gem.}$ = 14Hz, $J_d$ ~ 11,5 und 3Hz, 1H, Methylen-H); 2,29 (s, 3H, $CH_3$); 2,67 (AB von ABX, 2H, Methylen-H); 3,68 (AB von ABX, $J_{gem.}$ ~ 11Hz, $J_{AX}$ ~ 3Hz, $J_{BX}$ ~ 4Hz, 2H, Methylen-H); 4,45 (qui, 1H, Methin-H), 4,86 (m, 1H, Methin-H); 5,9 (s, 1H, Methin-H), 6,66 (s, J 2,5Hz, 1H, arom.-H); 6,9-7,1 (m, 9H, arom.-H) |

| | | | |
|---|---|---|---|
| 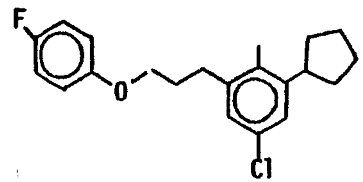 cc | DC: 0,26 (3) $^1$H-NMR (270 MHz): 1,08/1,10 (2 x s, 9H, t-Bu, 2 Anomere); 1,3-2,15 (m, 15H) 2,7-2,85 (m, 2H, Methylen-H); 3,2 - 3,4 (m, 1H, Methin-H); 3,65-4,00 (m, 4H, 2 x Methylen-H); 4,35/4,39 (2 x qui 1H, Methin-H, 2 Anomere); 4,50/4,69 (2 x m, 1H, Methin-H, 2 Anomere), 5,30/5,40 (2 x m, 1H, Methin-H, 2 Anomere); 6,75-6,85 (m, 1H, arom.-H); 6,90-7,00 (m, 3H, arom.-H); 7,00/7,08 (2 x t, 1H, arom.-H); 7,32 - 7,50 (m, 6H, arom.-H); 7,60-7,75 (m, 4H, arom.-H) | DC: 0,28 (3) MS: $C_{42}H_{48}ClFO_5Si$ 714, 657, 458, 437, 225, 199 $^1$H-NMR (60 MHz): 1,1 (s, 9H, t-Bu); ~1,3-2,3 (m, 12H), 2,3-3,0 (m, 4H); 3,0-3,4 (m, 1H, Methin-H); 3,8-4,1 (m, 4H, Methylen-H); 4,3-4,5 (qui, 1H, Methin-H); 4,9-5,2 (m, 1H, Methin-H); 6,8 - 7,1 (m, 6H, arom.-H); 7,4-7,8 (m, 10H, arom.-H) | DC: 0,18 (7) MS: $C_{26}H_{30}ClFO_5$ 476, 458, 348, 236 $^1$H-NMR (270 MHz); 1,4-2,2 (m, 13H); 2,6-2,82 (m, 4H, Methylen-H); 3,30 (qui, 1H, Methin-H); 3,88-4,00 (m, 4H, Methylen-H); 4,50 (qui, 1H, Methin-H); 4,96-5.04 (m, 1H, Methin-H); 6,8-6,9 (m, 2H, arom.-H); 6,93-7,00 (m, 2H, arom.-H); 7,03 (d, 1H, arom.-H); 7,10 (d, 1H, arom.-H) |
| 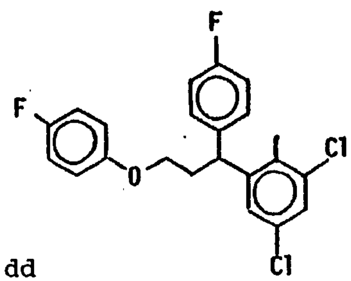 dd | DC: 0,33 (3) MS: $C_{43}H_{44}Cl_2F_2SiO_5$ , 776 | DC: 0,39 (3) MS: $C_{43}H_{42}Cl_2F_2SiO_5$ (774), 717 ($M^+$-t-Bu), 518, 407, 225, 199 $^1$H-NMR (270 MHz); 1,08 (s, 9H, t-Bu); 1,8-1,96 (m, 2H, Methylen-H); 2,33-2,48 (m, 3H, Methylen-H); 2,62 (d, breit, J = 18Hz, 1H, Methylen-H); 3,8 - 4,05 (m, 4H, Methylen-H); 4,38 (qui, 1H, Methin-H); 4,70 (dt, 1H, Methin-H); 5,10 (m, 1H, Methin-H); 6,68-6,80 (m, 2H, arom.-H); 6,86-7,00 (m, 4H, arom.-H); 7,15-7,28 (m, 2H, arom.-H); 7,35-7,48 (m, 6H, arom.-H); 7,64 (m, 4H, arom.-H); | DC: 0,19 (7) MS: $C_{27}H_{24}Cl_2F_2O_5$ 536, 518, 407 295 $^1$H-NMR (60 MHz): 1,65 (s, 1H, OH); 1,9-2,8 (m, 6H), 3,75 - 4,1 (m, 4H); 4,4-5,3 (m, 3H); 6,7-7,4 (m, 10H, arom.-H) |

| | | | | |
|---|---|---|---|---|
| ee | 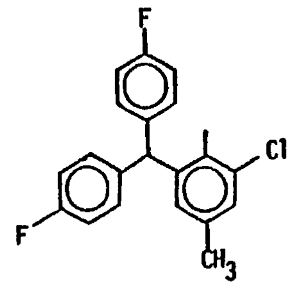 | DC: 0,18 (3)<br>MS: $C_{42}H_{43}ClF_2O_4Si$ (712), 637 ($M^+$-$H_2O$-t-Bu), 438, 199<br>$^1$H-NMR (60 MHz): 1,1 (s, 9H, t-Bu); 1,2-2,1 (m, 5H, Methylen-H und OH); 2,3 (s, 3H, $CH_3$); 3,4-3,6 (m, 2H, Methylen-H); 4,2-4,8 (m, 2H, 2 x Methin-H); 5,3 (m, 1H, Methin-H); 5,9 (s, 1H, Methin-H); 6,6-7,7 (m, 20H, arom.-H) | DC: 0,22 (4)<br>MS: $C_{42}H_{41}ClF_2O_4Si$ 710, 653<br>$^1$H-NMR (60 MHz): 1,1 (s, 9H, t-Bu); 1,4-2,0 (m, 2H, Methylen-H); 2,3 (s, 3H, $CH_3$); 2,4-2.6 (m, 2H, Methylen-H); 3,5-3,7 (m, 2H, Methylen-H); 4,3-4,5 (qui, 1H, Methin-H); 4,7-5,2 (m, 1H, Methin-H); 5,8 (s, 1H, Methin-H); 6,6 (d, 1H, arom.-H); 6,8-7,7 (m, 19H, arom.-H) | DC: 0,12 (7)<br>MS: $C_{26}H_{23}ClF_2O_4$ 472, 454<br>$^1$H-NMR (60 MHz): 1,65 (s, 1H, OH); 1,8-2,2 (m, 2H, Methylen-H); 2,3 (s, 3H, $CH_3$); 2,6-2,8 (m, 2H, Methylen-H) 3,6-3,8 (m, 2H, Methylen-H); 4,4-4,5 (qui, 1H, Methin-H); 4,8-5,0 (m, 1H, Methin-H); 5,9 (s, 1H, Methin-H); 6,7 (d, 1H, arom.-H); 6,85-7,15 (m, 9H, arom.-H) |
| ff | 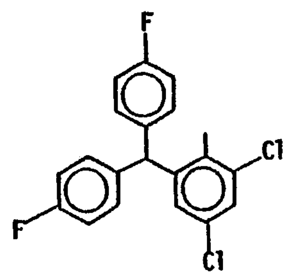 | DC: 0,63 (2)<br>MS: $C_{41}H_{40}Cl_2F_2O_4Si$ (732); 689 ($M^+$-i-Pr), 675 ($M^+$-t-Bu), 657, 631, 553, 485, 363, 267, 199<br>$^1$H-NMR (270 MHz): 1,10/1,12 (2 x s, ca. 1 : 2, 9H, t-Bu, 2 Anomere); 1,2-1,5 (m, 2H, Methylen-H); 1,62 (s, 1H, OH); 1,7 (dt, 1H, Methylen-H); 1,98 (d, 1H, Methylen-H); 3,8 (m, 2H, Methylen-H); 4,28-4,40 (2 x qui, 1H, Methin-H, 2 Anomere); 4,65 (m, 1H, Methin-H); 5,3 (dd, 1H, Methin-H) 5,58/5,63 (2 x s, 1H, Methin-H; 2 Anomere), 6,03/6,11 (2 x s, 1H, arom.-H, 2 Anomere); 6,70/6,73 (2 x d, 1H, arom.-H, 2 Anomere); 6,9-7,05 (m, 8H, arom.-H); 7,34-7,50 (m, 6H, arom.-H); 7,6-7,7 (m, 4H, arom.-H) | DC: 0,69 (2)<br>MS: $C_{41}H_{38}Cl_2F_2O_4Si$ 730, 673 ($M^+$-t-Bu), 631, 405, 363, 267, 225, 199<br>$^1$H-NMR (60 MHz): 1,1 (s, 9H, t-Bu); 1,6-1,9 (m, 2H, Methylen-H); 2,50 (m, 2H, Methylen-H); 3,80 (d, J = 4Hz, 2H, Methylen-H); 4,40 (qui, 1H, Methin-H); 5,00 (m, 1H, Methin-H); 5,90 (s, 1H, Methin-H); 6,60 (d, J = 2,5 Hz, 1H, arom.-H); 6,80 (d, J = 3Hz, 4H, arom.-H); 7,03 (d, J = 3Hz 4H, arom.-H); 7,3-7,7 (m, 11H, arom.-H) | DC: 0,20 (7)<br>MS: $C_{25}H_{20}Cl_2F_2O_4$ 492, 361, 267, 129<br>$^1$H-NMR (270 MHz): 1,8 (s, breit, 1H, OH); 1,9-2,1 (m, 2H, Methylen-H); 2,65 [d(sehr enges AB), 2H, Methylen-H]; 3,86 (dd, $J_{gem}$ = 10 Hz, $J_{vic}$ = 3Hz, 1H, Methylen-H; 3,97 (dd, $J_{gem}$ = 10Ht $J_{vic}$ = 4Hz, 1H, Methylen-H); 4,44 (qui, 1H, Methin-H); 4,89 (m, 1H, Methin-H); 5,95 (s, 1H, Methin-H); 6,75 (d, J = 3Hz, 1H, arom.-H); 6,95-7,10 (m, 8H, arom.-H); 7,30 (d, J = 3Hz, 1H, arom.-H) |

| | | | |
|---|---|---|---|
| qq 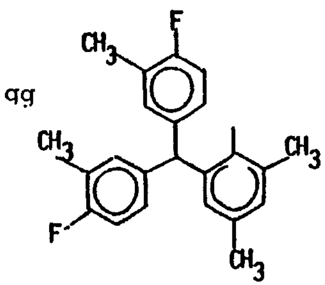 | DC: 0,14 (4)<br>MS: $C_{45}H_{50}F_2O_4Si$ 720, 702, 645 | DC: 0,25 (4)<br>MS: $C_{45}H_{48}F_2O_4Si$ 718, 661 | DC: 0,11 (7)<br>MS: $C_{29}H_{30}F_2O_4$ 480, 462<br>$^1$H-NMR (60 MHz): 1,6 (s, br., 1H, OH); 1,8-2,1 (m, 2H, Methylen-H); 2,15-2,3 (1 x d + 2 x s, $J_{HF}$~2Hz, 12H, 4 x $CH_3$); 2,5-2,8 (m, 2H, Methylen-H); 3,6-3,8 (m, 2H, Methylen-H); 4,45 (qui, 1H, Methin-H); 4,8-4,9 (m, 1H, Methin-H); 5,9 (s, 1H, Methin-H); 6,6 (d, 1H, arom.-H); 6,9-7,2 (m, 7H, arom.-H) |
| hh 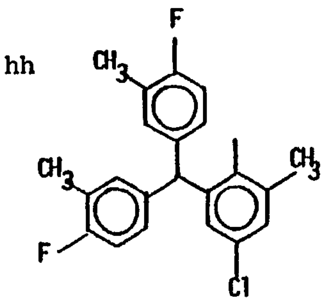 | DC: 0,16 (3)<br>MS: $C_{44}H_{47}ClF_2O_4Si$ (740), 665 ($M^+$-$H_2O$-t-BuSiPh$_2$OH)<br>$^1$H-NMR (60 MHz): 1,1 (s, 9H, t-Bu); 1,3-2,1 (m, 5H, 2 x Methylen-H und OH); 2,2-2,3 (1 x d + 1 x s, 9H, 3 x $CH_3$); 3,4-3,6 (m, 2H, Methylen-H); 4,2-4,8 (m, 2H, 2 x Methin-H); 5,2-5,4 (m, 1H, Methin-H); 5,9 (s, 1H, Methin-H); 6,6-7,8 (m, 18H, arom.-H) | DC: 0,20 (4)<br>MS: $C_{44}H_{45}ClF_2O_4Si$ 738, 681<br>$^1$H-NMR (60 MHz): 1,1 (s, 9H, t-Bu); 1,5-1,9 (m, 2H, Methylen-H); 2,2-2,3 (1 x d + 1 x s, 9H, 3 x $CH_3$); 2,4-2,6 (m, 2H, Methylen-H); 3,5-3,7 (m, 2H, Methylen-H); 4,3-4,5 (qui, 1H, Methin-H); 4,7-5,2 (m, 1H, Methin-H); 5,7 (s, 1H, Methin-H); 6,7 (d, 1H, arom.-H); 6,8-7,7 (m, 17H, arom.-H) | DC: 0,11 (7)<br>MS: $C_{28}H_{27}ClF_2O_4$ 500, 482<br>$^1$H-NMR (60 MHz): 1,6 (s, br., 1H, OH); 1,8-2,1 (m, 2H, Methylen-H); 2,15-2,3 (1 x d, 1 x s, $J_{HF}$~2Hz, 9H, 3 x $CH_3$); 2,5-2,8 (m, 2H, Methylen-H); 3,6-3,8 (m, 2H, Methylen-H); 4,45 (qui, 1H, Methin-H); 4,8-4,9 (m, 1H, Methin-H); 5,9 (s, 1H, Methin-H); 6,6 (d, 1H, arom.-H), 6,9-7,2 (m, 7H, arom.-H) |
| ii 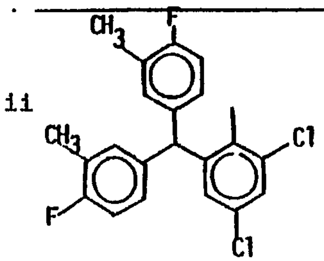 | DC: 0,60 (2)<br>MS: $C_{43}H_{44}Cl_2F_2O_4Si$ (760), 703 ($M^+$-t-Bu) | DC: 0,65 (2)<br>MS: $C_{43}H_{42}Cl_2F_2O_4Si$ 758, 701 | DC: 0,15 (7)<br>MS: $C_{27}H_{24}Cl_2F_2O_4$ 520<br>$^1$H-NMR (270 MHz): 1,8 (s, breit, 1H, OH); 1,9-2,1 (m, 2H, Methylen-H); 2,25 (d, $J_{HF}$~2Hz, 6H, 2 x $CH_3$); 2,6- |

| | | | |
|---|---|---|---|
| | | | 2,7 (AB von ABX, 2H, Methylen-H); 3,8-4,0 (AB von ABX, 2H, Methylen-H); 4,45 (qui, 1H, Methin-H); 4,9 (m, 1H, Methin-H); 5,85 (s, 1H, Methin-H), 6,75 (d, J = 3Hz, 1H, arom.-H); 6,9-7,3 (m, 7H, arom.-H) |
| jj 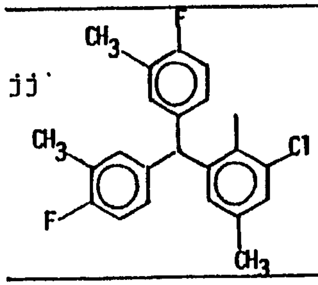 | DC: 0,15 (3)<br>MS: $C_{44}H_{47}ClF_2O_4Si$ (740), 665 | DC: 0,21 (4)<br>MS: $C_{44}H_{45}ClF_2O_4Si$ 738, 681 | DC: 0,12 (7)<br>MS: $C_{28}H_{27}ClF_2O_4$ 500, 482 |
| kk 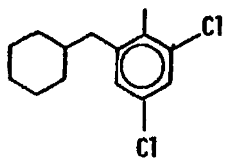 | DC: 0,39 (1)<br>MS: $C_{35}H_{44}Cl_2O_4Si$ (626), 569 ($M^+$-t-Bu), 525, 491, 370, 300, 258, 199<br>$^1$H-NMR (270MHz): 0,8-1,2 (m, 4H); 1,11/1,13 (2 x s, 9H, t-Bu, 2 Anomere); 1,35 - 1,80 (m, 10H); 1,97 (t, 1H, Methin-H) 2,40 - 2,57 (AB von ABX, 2H, Methylen-H); 2,87 (d, J = 6Hz, 0,5H, OH von Anomer 1); 3,8-4,03 (m, 2H, Methylen-H); 4,36/4,39 (2 x qui, 1H, Methin-H, 2 Anomere); 4,53/4,70 (2 x m, 1H, Methin-H, 2 Anomere); 5,30/5,40 (2 x m, 1H, Methin-H); 5,60 (d, J = 11Hz, 0,5H, OH von Anomer 2),; 7,0 (t, 1H, arom.-H); 7,2 (dd, 1H, arom.-H); 7,3-7,5 (m, 6H, arom.-H); 7,6-7,73 (m, 4H, arom.-H) | DC: 0,51 (1)<br>MS: $C_{35}H_{42}Cl_2O_4Si$ (624), 609 ($M^+$-$CH_3$), 567 ($M^+$-t-Bu), 309/311, 225, 199<br>$^1$H-NMR (60MHz); 1,1 (s, 9H, t-Bu); 1,1-2,1 (m, 13H); 2,4-2,6 (m, 4H, 2 x Methylen-H); 4.05 (d, J = 4Hz, 2H, Methylen-H); 4,4 (qui, 1H, Methin-H); 4,8-5,3 (m, 1H, Methin-H); 7,0-7,7 (m, 12H, arom.-H) | DC: 0,16 (7)<br>MS: $C_{19}H_{24}Cl_2O_4$ 386, 256/258/260, 176, 111<br>$^1$H-NMR (60MHz): 0,7-2,6 (m, 15H); 2,7 (d, J = 4Hz, 2H, Methylen-H); 4,1 (d, J = 4Hz, 2H, Methylen-H); 4,6 (qui, 1H, Methin-H); 4,85-5,20 (m, 1H, Methin-H); 7,05 (d, J = 2Hz, 1H, arom.-H); 7,3 (d, J = 2Hz, 1H, arom.-H) |

| | | | |
|---|---|---|---|
| 11<br>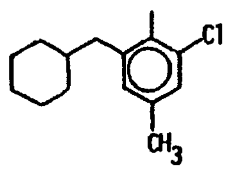 | DC: 0,08 (4)<br>MS: $C_{36}H_{47}ClO_4Si$ (606), 549 ($M^+$-t-Bu) | DC: 0,43 (1)<br>MS: $C_{36}H_{45}ClO_4Si$ (604), 547 ($M^+$-t-Bu)<br>$^1$H-NMR (60 MHz): 1,1 (s, 9H, t-Bu); 1,1-2,65 (m, 17H); 2,2 (s, 3H, $CH_3$); 3,85 (d, 2H, Methylen-H), 4,35 (qui, 1H, Methin-H); 6,95-7,75 (m, 12H, arom.-H) | DC: 0,17 (7)<br>MS: $C_{20}H_{27}O_4Cl$, 366, 284, 238<br>$^1$H-NMR (60 MHz): 0,8-2,0 (m, 12H, Cyclohexyl-H und OH); 2,0-2,2 (d, 2H, Methylen-H); 2,3 (s, 3H, $CH_3$); 2,4-2,6 (d, 2H, Methylen-H); 2,75 (d, 2H, Methylen-H); 3,9 (d, 2H, Methylen-H); 4,50 (qui, 1H, Methin-H), 4,8-5,2 (m, 1H, Methin-H), 6,9-7,1 (m, 2H, arom.-H) |
| mm<br>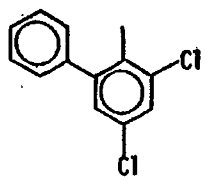 | DC: 0,16 (3)<br>MS: $C_{34}H_{36}Cl_2O_4Si$ (606), 563 ($M^+$-i-Pr), 549 ($M^+$-t-Bu); 505, 471, 445, 289, 238, 199<br>$^1$H-NMR (60 MHz): 1,1 (s, 9H, t-Bu); 1,2 - 2,2 (m, 5H, 2 x Methylen-H und OH); 3,6 (m, 2H, Methylen-H); 4,0-4,6 (m, 2H, Methin-H), 4,9-5,5 (m, 1H, Methin-H); 7,2-7,8 (m, 17H, arom.-H) | DC: 0,30 (3)<br>MS: $C_{34}H_{34}Cl_2O_4Si$ (604), 547/549 ($M^+$-t-Bu), 505/507, 225, 199<br>$^1$H-NMR (60 MHz): 1,05 (s, 9H, t-Bu); 1,5-1,8 (m, 2H, Methylen-H), 2,3-2,5 (m, 2H, Methylen-H); 3,75 (d, 2H, Methylen-H); 4,3 (qui, 1H, Methin-H); 4,6-5,0 (m, 1H, Methin-H); 7,2-7,8 (m, 17H, arom.-H) | DC: 0,11 (7)<br>MS: $C_{18}H_{16}O_4Cl_2$ 366/368, 348/350 ($M^+$-$H_2O$), 238/240, 202/204, 111<br>$^1$H-NMR (270 MHz): 1,70 (d, J = 2,5 Hz, 1H, OH); 1,8-1,98 (m, 2H, Methylen-H) 2,58 (d, J = 4 Hz, 2H, Methylen-H); 3,8 (AB von ABX, 2H, Methylen-H); 4,32 (qui, 1H, Methin-H); 4,67 (m, 1H, Methin-H); 7,15-7,55 (m, 7H, arom.-H) |

| | | | |
|---|---|---|---|
| | 5,3 (m, 1H, Methin-H; 6,7 (s, arom.-H); 6,9 - 7,8 (m, 15H, arom.-H) | 5,1 (m überlagert von s, 3H, Methin-H + Methylen-H); 6,7 (s, 1H, arom.-H); 6,9-7,8 (m, 15H, arom.-H) | 4,9 - 5,2 (m + s, 3H, Methin-H + Methylen-H); 6,6 (s, 1H, arom.-H); 6,95 - 7,55 (m, 5H, arom.-H) |
| nn 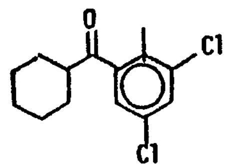 | DC: 0,14 (4)<br>MS: $C_{35}H_{42}Cl_2O_5Si$ (638), 581/583/585 ($M^+$-t-Bu), 565/567 ($M^+$-t-Bu-$H_2O$), 199<br>$^1$H-NMR (60 MHz): 1,05 (s, 9H, t-Bu); 1,1-2,1 (m, 15H); 3,55 (d, J=4Hz, 1H, Methylen-H); 3,90 (d, J=4Hz, 1H, Methylen-H); 4,00 - 4,45 (m, 2H, 2 x Methin-H); 5,0 - 5,5 (m, 1H, Methin-H); 7,2 - 7,8 (m, 12 H, arom.-H) | DC: 0,33 (1)<br>MS: $C_{35}H_{40}Cl_2O_5Si$ 639 ($MH^+$) 581 ($M^+$-t-Bu), 225, 199, 183<br>$^1$H-NMR (270 MHz): 1,08 (s, 9H, t-Bu); 1,25 - 1,40 (m, 5H); 1,60 - 1,93 (m, 7H); 2,45 (dd, J=18Hz und 4Hz, 1H, Methylen-H); 2,60 (dm, J = 18Hz, 1H, Methylen-H); 3,01 (m, 1H, Methin-H); 4,07 (d, J=4Hz, 2H, Methylen-H); 4,38 (qui, 1H, Methin-H); 5,1 (sept., 1H, Methin-H); 7,24 (d, J = 2,5Hz, 1H, arom.-H); 7,37 - 7,50 (m, 7H, arom.-H); 7,60 - 7,70 (m, 4H, arom.-H) | DC: 0,16 (7)<br>MS: $C_{19}H_{22}Cl_2O_5$ 401/403 ($M+H^+$), 299/301, 270/272, 189/191<br>IR ($CHCl_3$): 3450 (breit, OH); 1720 (breit, C=O), 1235 $cm^{-1}$<br>$^1$H-NMR (270 MHz): 1,12 (d, J=6Hz, 1H, OH); 1,2 - 1,45 (m, 5H); 1,6-1,95 (m, 5H); 2,08 - 2,20 (m, 2H, Methylen-H); 2,70 (AB von ABX, $J_{gem}$= 18Hz, $J_{vic}$~4Hz, 2H, Methylen-H); 3,03 (m, 1H, Methin-H); 4,12 (d, J=4Hz, 2H, Methylen-H); 4,50 (qui, 1H, Methin-H); 5,00 (m, 1H, Methin-H); 7,25 (d, J~3Hz, 1H, arom.-H); 7,48 (d, J~3Hz, 1H, arom.-H) |

DC-Laufmittel:
1: Cyclohexan / Essigester = 4 : 1
2: Cyclohexan / Essigester = 3 : 1
3: Cyclohexan / Essigester = 5 : 1
4: Cyclohexan / Essigester = 9 : 1
5: 100 % Toluol
6: Toluol / Essigester = 9 : 1
7: Cyclohexan / Essigester = 1 : 1

Beispiel 45a:

3(R),5(S)-Dihydroxy-6-{4,6-dimethyl-2-[3-(4-fluorphenoxy)propyl]phenoxy}hexansäure Natriumsalz

200 mg Lakton 44a wurden in 15 ml Tetrahydrofuran mit 4,6 ml 0,1 n wäßriger Natronlauge (0,46 mmol) versetzt. Nach 1 h bei Raumtemperatur wurde das Lösungsmittel im Vakuum abgezogen und der Rückstand mehrmals mit Toluol versetzt und einrotiert. Kristallisation mit Diisopropylether ergab 175 mg Natriumsalz 45a.

In Analogie zu Beispiel 45a wurden die entsprechenden Natriumsalze aus den Hydroxylaktonen 44b–44nn (Tabelle 5) hergestellt. In einigen Fällen wurde an Stelle von Tetrahydrofuran Methanol als Lösungsmittel verwendet.

**Patentansprüche für die Vertragsstaaten: BE, CH, DE, FR, GB, IT, LI, LU, NL, SE**

1. 6-Phenoxymethyl-4-hydroxytetrahydropyran-2-one der allgemeinen Formel I

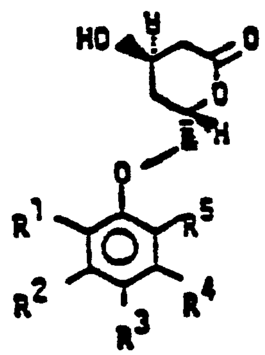

I

worin

$R^1$ und $R^5$ gleich oder verschieden sind und a) Halogen, b) Cycloalkyl mit 4–8 C-Atomen oder einen Phenylrest, der im Kern 1- bis 3-fach substituiert sein kann mit Halogen, Trifluormethyl und/oder Alkyl oder Alkoxy mit je 1–4 C-Atomen oder c) einen geradkettigen oder verzweigten Alkylrest mit 1 bis 18 Kohlenstoffatomen oder einen geradkettigen oder verzweigten Alkylrest mit 2 bis 18 Kohlenstoffatomen bedeuten, wobei die Alkyl- bzw. Alkenylreste ihrerseits 1–3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,

β) Halogen, Hydroxy, Cycloalkyl mit 3–7 C-Atomen, unsubstituierten Phenyl-, α- oder β-Thienylresten oder Phenyl-, α- oder β-Thienylresten, welche ihrerseits im Kern 1- bis 3-fach substituiert sind mit Halogen, Trifluormethyl und/oder Alkyl oder Alkoxy mit 1–4 C-Atomen,

γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,

δ) der Gruppe

$$-O-\overset{O}{\overset{\|}{C}}-R^6,$$

wobei $R^6$ bedeutet: einen geradkettigen oder verzweigten Alkyl- oder Alkenylrest mit bis zu 8 Kohlenstoffatomen, oder einen Cycloalkyl- oder Cycloalkenylrest mit jeweils 3–8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1–4 C-Atomen, oder einen 3-Pyridylrest,

$R^2$ und $R^4$ Wasserstoff bedeuten, und

$R^3$ Alkyl oder Alkenyl mit bis zu 4 C-Atomen, Halogen oder Alkoxy mit 1–4 C-Atomen ist,

sowie die entsprechenden offenkettigen Dihydroxycarbonsäuren, deren pharmakologisch verträglichen Salze mit Basen und deren pharmakologisch verträgliches Ester.

2. Verbindungen gemäß Anspruch 1, dadurch gekennzeichnet, daß in Formel I

$R^1$ und $R^5$ beide gleich oder verschieden sind und

a) Halogen

b) Cycloalkyl mit 5 bis 6 C-Atomen, Phenyl, das im Kern 1–3-fach substituiert sein kann mit Halogen, Trifluormethyl und/oder Alkyl oder Alkoxy mit je 1–4 C-Atomen, oder

c) 1. geradkettiges oder verzweigtes Alkyl oder Alkenyl mit bis zu 12 Kohlenstoffatomen, wobei der Alkyl- bzw. Alkenylrest seinerseits 1–2-fach substituiert sein kann mit Phenylresten, die ihrerseits im Kern 1- bis 3-fach substituiert sein können mit Halogen, Trifluormethyl und/oder Alkyl oder Alkoxy mit 1–4 C-Atomen,

2. geradkettiges, mit O–COR$^6$ substituiertes Alkyl der Formel

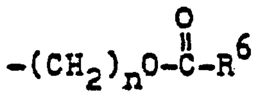

worin n = 1 bis 3 und R$^6$ einen geradkettigen oder verzweigten Alkyl- oder Alkenylrest mit bis zu 8 C-Atomen, einen Cycloalkylrest mit 5 bis 6 C-Atomen oder einen Phenylrest, welcher seinerseits im Kern 1–3-fach substituiert sein kann mit Halogen, Trifluormethyl und/oder Alkyl oder Alkoxy mit 1 bis 4 C-Atomen, bedeutet,

3. geradkettiges mit OR$^7$ substituiertes Alkyl der Formel

$$-(CH_2)_nOR^7,$$

worin n = 1 bis 3 und R$^7$ Wasserstoff oder einen geradkettigen oder verzweigten Alkyl- oder Alkenylrest mit bis zu 8 C-Atomen, einen Cycloalkylrest mit 5 bis 6 C-Atomen oder einen Phenylrest oder Benzylrest, welche ihrerseits im aromatischen Kern 1–3-fach substituiert sein können mit Halogen, Trifluormethyl und/oder Alkyl oder Alkoxy mit 1 bis 4 C-Atomen, bedeutet, darstellen,

R$^2$ und R$^4$ Wasserstoff und

R$^3$ Methyl, Ethyl, Propyl, 1-Propenyl, Allyl, Fluor oder Chlor bedeuten.

3. Verbindungen gemäß Anspruch 1, dadurch gekennzeichnet, daß in Formel I

R$^1$ und R$^5$ gleich oder verschieden sind und

1) Methyl, Ethyl, Propyl, Allyl, 1-Propenyl, Isopropenyl, Isopropyl, Cyclopentyl, Cyclohexyl, oder

2) die Gruppe

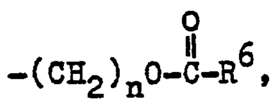

mit n = 1–3, wobei R$^6$ bedeutet: Methyl, Ethyl, Propyl, i-Propyl, n-Butyl, i-Butyl, t-Butyl, Phenyl oder im Kern 1- bis 3-fach substituiertes Phenyl mit Halogen, Methyl oder Methoxy, oder

3) die Gruppe $-(CH_2)_nOR^7$, mit n = 1 bis 3, wobei R$^7$ bedeutet: Wasserstoff, Methyl, geradkettiges oder verzweigtes Alkyl oder Alkenyl mit 3–5 Kohlenstoffatomen, Cyclopentyl, Cyclohexyl, Phenyl oder Benzyl, wobei die aromatischen Kerne 1–3-fach substituiert sein können mit Fluor, Chlor, Methyl, Methoxy, oder

4) eine Alkylgruppe der Formel

$$-(CH_2)_nCHR^8R^9$$

worin n = 0 bis 2 ist und R$^8$ und R$^9$ gleich oder verschieden sind und Wasserstoff, Methyl, Ethyl, Propyl, Allyl, i-Propyl, n-Butyl, i-Butyl, t-Butyl, Cyclohexyl, Cyclopentyl, Benzyl oder Phenyl, wobei die aromatischen Kerne 1- bis 3-fach substituiert sein können mit Fluor, Chlor, Methyl oder Methoxy, bedeuten, darstellen,

R$^2$ und R$^4$ Wasserstoff und

R$^3$ Methyl, Chlor, Fluor bedeuten.

4. Verfahren zur Herstellung der Verbindungen gemäß Anspruch 1, dadurch gekennzeichnet, daß man

a) entsprechend substituierte Phenole der Formel II,

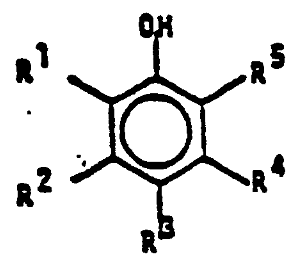

II

worin R$^1$, R$^2$, R$^3$, R$^4$ und R$^5$ die angegebenen Bedeutungen haben, mit dem chiralen Jodid der Formel III

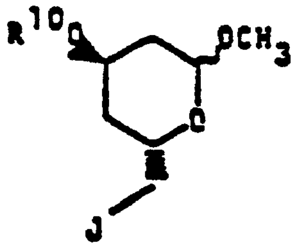

III

worin $R^{10}$ eine gegen Basen und schwache Säuren stabile Schutzgruppe bedeutet, in die Ether der Formel IV

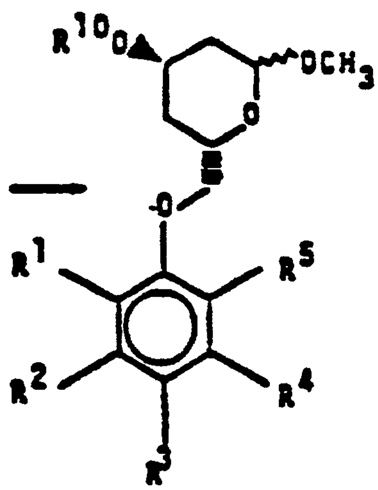

IV

worin $R^1$ bis $R^5$ die zu Formel I und $R^{10}$ die zu Formel III angegebenen Bedeutungen haben, überführt,
b) die Ether der Formel IV zu den entsprechenden Halbacetalen der Formel V

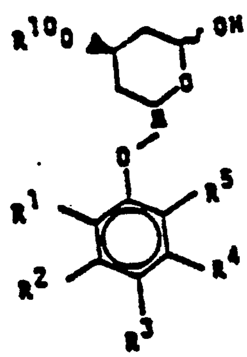

V

worin $R^1$ bis $R^5$ und $R^{10}$ die zu Formel I bzw. III angegebenen Bedeutungen haben, hydrolysiert,
c) die Halbacetale der Formel V zu den entsprechenden Lactonen der Formel VI

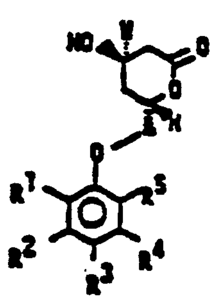

(I)

worin $R^1$ bis $R^5$ die zu Formel I und $R^{10}$ die zu Formel III angegebenen Bedeutungen haben, oxidiert und d) die geschützten Hydroxylaktone der Formel VI in die 6-Phenoxymethyl-4-hydroxytetrahydropyran-2-one der Formel I

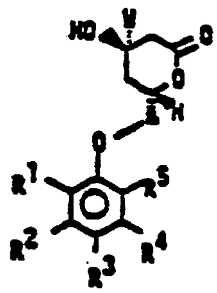

I

überführt, gegebenenfalls die erhaltenen Verbindungen der Formel I in die entsprechenden offenkettigen Dihydroxycarbonsäuren der Formel VII

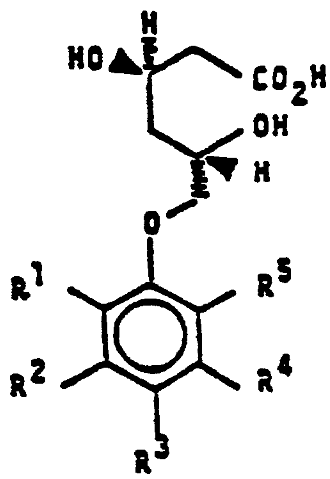

VII

deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

5. Pharmazeutische Präparate enthaltend eine Verbindung gemäß Anspruch 1.

6. Verwendung einer Verbindung gemäß Anspruch 1 zur Herstellung eines pharmazeutischen Präparates zur Prophylaxe und Therapie der Hypercholesterinämie.

7. Ether der Formel IV

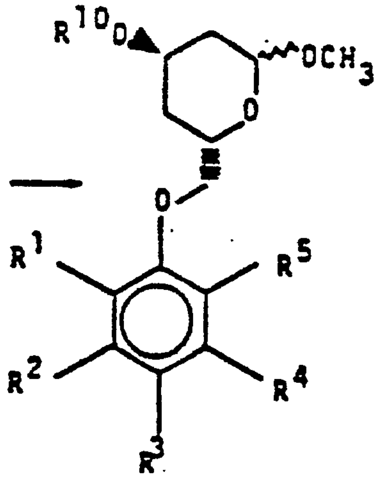

IV

worin $R^1$, $R^2$, $R^3$, $R^4$ und $R^5$ die zu Formel I und $R^{10}$ die zu Formel III angegebenen Bedeutungen haben.

8. Halbacetale der Formel V

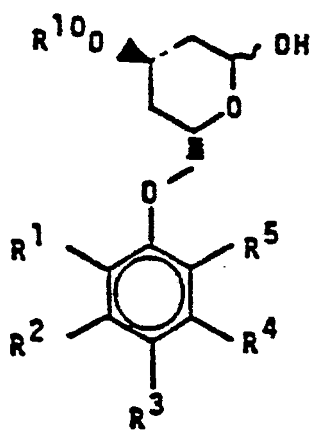

V

worin $R^1$ bis $R^5$ und $R^{10}$ die zu Formel I bzw. III angegebenen Bedeutungen haben.

9. Lactone der Formel VI

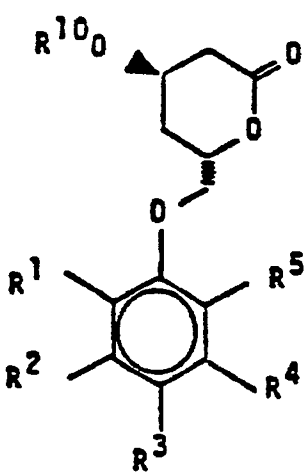

VI

worin $R^1$ bis $R^5$ die zu Formel I und $R^{10}$ die zu Formel III angegebenen Bedeutungen haben.

**Patentansprüche für den Vertragsstaat: AT**

1. Verfahren zur Herstellung von 6-Phenoxymethyl-4-hydroxytetrahydropyran-2-onen der allgemeinen Formel I

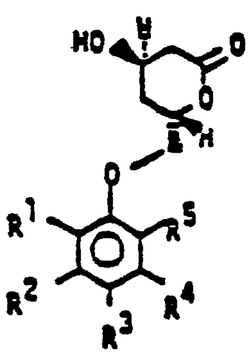

I

worin
$R^1$ und $R^5$ gleich oder verschieden sind und a) Halogen, b) Cycloalkyl mit 4–8 C-Atomen oder einen Phenylrest, der im Kern 1- bis 3-fach substituiert sein kann mit Halogen, Trifluormethyl und/oder Alkyl oder Alkoxy mit je 1–4 C-Atomen oder c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1–3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,

β) Halogen, Hydroxy, Cycloalkyl mit 3–7 C-Atomen, unsubstituierten Phenyl-, α- oder β-Thienylresten oder Phenyl-, α- oder β-Thienylresten, welche ihrerseits im Kern 1- bis 3-fach substituiert sind mit Halogen, Trifluormethyl, und/oder Alkyl oder Alkoxy mit 1–4 C-Atomen,

γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen,

δ) der Gruppe

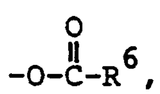

wobei $R^6$ bedeutet:
einen geradkettigen oder verzweigten Alkyl- oder Alkenylrest mit bis zu 8 Kohlenstoffatomen oder einen Cycloalkyl oder Cycloalkenylrest mit jeweils 3–8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1–4 C-Atomen, oder einen 3-Pyridylrest,

$R^2$ und $R^4$ Wasserstoff bedeuten, und
$R^3$ Alkyl oder Alkenyl mit bis zu 4 C-Atomen, Halogen oder Alkoxy mit 1–4 C-Atomen ist,
sowie der entsprechenden offenkettigen Dihydroxycarbonsäuren, deren pharmakologisch verträglichen Salze mit Basen und deren pharmakologisch verträglichen Estern, dadurch gekennzeichnet, daß man

a) entsprechend substituierte Phenolen der Formel II,

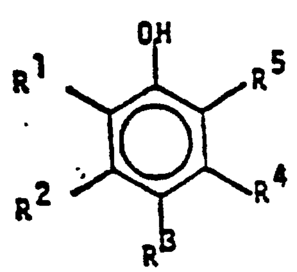

II

worin $R^1$, $R^2$, $R^3$, $R^4$ und $R^5$ die angegebenen Bedeutungen haben, mit dem chiralen Jodid der Formel III

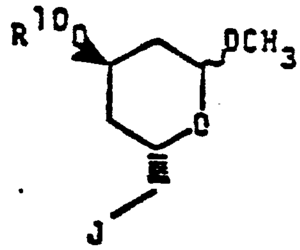

III

worin $R^{10}$ eine gegen Basen und schwache Säuren stabile Schutzgruppe bedeutet, in die Ether der Formel IV

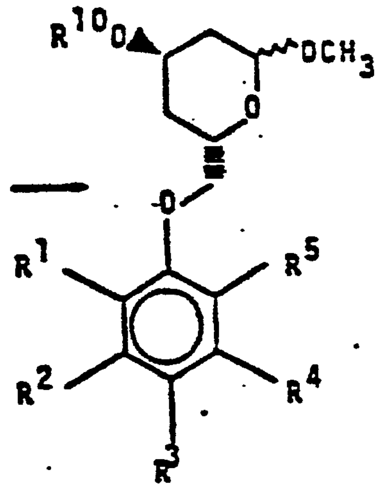

IV

worin $R^1$ bis $R^5$ die zu Formel I und $R^{10}$ die zu Formel III angegebenen Bedeutungen haben, überführt,
b) die Ether der Formel IV zu den entsprechenden Halbacetalen der Formel V

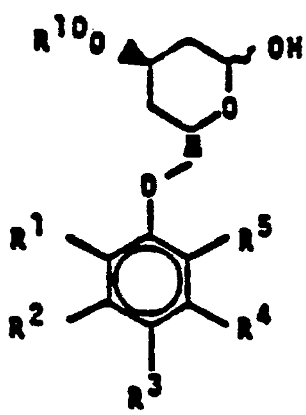

V

worin $R^1$ bis $R^5$ und $R^{10}$ die zu Formel I bzw. III angegebenen Bedeutungen haben, hydrolysiert,
c) die Halbacetale der Formel V zu den entsprechenden Lactonen der Formel VI

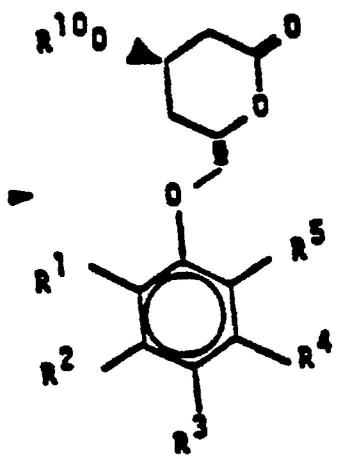

VI

worin $R^1$ bis $R^5$ die zu Formel I und $R^{10}$ die zu Formel III angegebenen Bedeutungen haben, oxidiert und d) die geschützten Hydroxylaktone der Formel VI in die 6-Phenoxymethyl-4-hydroxytetrahydropyran-2-one der Formel I

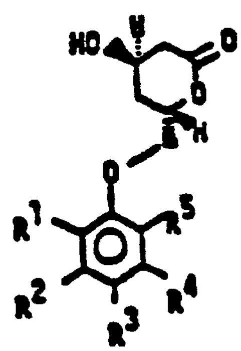

I

überführt, gegebenenfalls die erhaltenen Verbindungen der Formel I in die entsprechenden offenkettigen Dihydroxycarbonsäuren der Formel VII

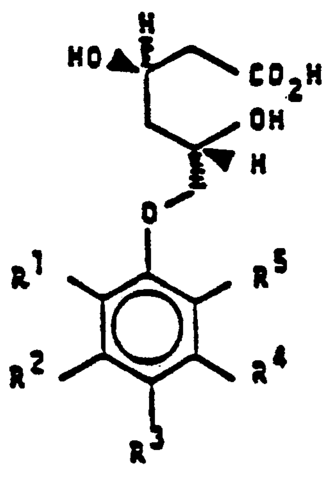

VII

deren Salze oder deren Ester überführt, gegebenenfalls erhaltene Salze oder Ester in die freien Dihydroxycarbonsäuren oder gegebenenfalls die freien Carbonsäuren in die Salze oder Ester überführt.

2. Verfahren gemäß Anspruch 1, dadurch gekennzeichnet, daß man Verbindungen der Formel I, worin $R^1$ und $R^5$ beide gleich oder verschieden sind und

a) Halogen,

b) Cycloalkyl mit 5 bis 6 C-Atomen, Phenyl, das im Kern 1–3-fach substituiert sein kann mit Halogen, Trifluormethyl, und/oder Alkyl oder Alkoxy mit je 1–4 C-Atomen, oder

c) 1. geradkettiges oder verzweigtes Alkyl oder Alkenyl mit bis zu 12 Kohlenstoffatomen, wobei der Alkyl- bzw. Alkenylrest seinerseits 1–2-fach substituiert sein kann mit Phenylresten, die ihrerseits im Kern 1- bis 3-fach substituiert sein können mit Halogen, Trifluormethyl und/oder Alkyl oder Alkoxy mit 1–4 C-Atomen,

2. geradkettiges, mit O–COR⁶ substituiertes Alkyl der Formel

$$-(CH_2)_nO-\overset{\overset{O}{\|}}{C}-R^6$$

worin n = 1 bis 3 und $R^6$ einen geradkettigen oder verzweigten Alkyl- oder Alkenylrest mit bis zu 8 C-Atomen, einen Cycloalkylrest mit 5 bis 6 C-Atomen oder einen Phenylrest, welcher seinerseits im Kern 1–3-fach substituiert sein kann mit Halogen, Trifluormethyl und/oder Alkyl oder Alkoxy mit 1 bis 4 C-Atomen, bedeutet,

3. geradkettiges mit $OR^7$ substituiertes Alkyl der Formel

$$-(CH_2)_nOR^7,$$

worin n = 1 bis 3 und $R^7$ Wasserstoff oder einen geradkettigen oder verzweigten Alkyl- oder Alkenylrest mit bis zu 8 C-Atomen, einen Cycloalkylrest mit 5 bis 6 C-Atomen oder einen Phenylrest oder Benzylrest, welche ihrerseits im aromatischen Kern 1–3-fach substituiert sein können mit Halogen, Trifluormethyl und/oder Alkyl oder Alkoxy mit 1 bis 4 C-Atomen, bedeutet, darstellen,

$R^2$ und $R^4$ Wasserstoff und

$R^3$ Methyl, Ethyl, Propyl, 1-Propenyl, Allyl, Fluor oder Chlor bedeuten, herstellt.

3. Verfahren gemäß Anspruch 1, dadurch gekennzeichnet, daß man Verbindungen der Formel I, worin $R^1$ und $R^5$ gleich oder verschieden sind und

1) Methyl, Ethyl, Propyl, Allyl, 1-Propenyl, Isopropenyl, Isopropyl, Cyclopentyl, Cyclohexyl oder

2) die Gruppe

$$-(CH_2)_nO-\overset{\overset{O}{\|}}{C}-R^6,$$

mit n = 1–3, wobei $R^6$ bedeutet: Methyl, Ethyl, Propyl, i-Propyl, n-Butyl, i-Butyl, t-Butyl, Phenyl oder im Kern 1- bis 3-fach substituiertes Phenyl mit Halogen, Methyl oder Methoxy, oder

3) die Gruppe $-(CH_2)_nOR^7$, mit n = 1 bis 3, wobei $R^7$ bedeutet: Wasserstoff, Methyl, geradkettiges oder verzweigtes Alkyl oder Alkenyl mit 3–5 Kohlenstoffatomen, Cyclopentyl, Cyclohexyl; Phenyl oder Benzyl, wobei die aromatischen Kerne 1–3-fach substituiert sein können mit Fluor, Chlor, Methyl, Methoxy oder

4) eine Alkylgruppe der Formel

$$-(CH_2)_nCHR^8R^9$$

worin n = 0 bis 2 ist und $R^8$ und $R^9$ gleich oder verschieden sind und Wasserstoff, Methyl, Ethyl, Propyl, Allyl, i-Propyl, n-Butyl, i-Butyl, t-Butyl, Cyclohexyl, Cyclopentyl, Benzyl oder Phenyl, wobei die aromatischen Kerne 1- bis 3-fach substituiert sein können mit Fluor, Chlor, Methyl oder Methoxy, bedeuten, darstellen,

$R^2$ und $R^4$ Wasserstoff und

$R^3$ Methyl, Chlor, Fluor bedeuten, herstellt.

4. Verfahren zur Herstellung eines pharmazeutischen Präparates, dadurch gekennzeichnet, daß man eine Verbindung, erhältlich nach Verfahren gemäß Anspruch 1, in eine geeignete Darreichungsform überführt.

5. Verfahren gemäß Anspruch 1, dadurch gekennzeichnet, daß man eine Zwischenstufe der Formel IV

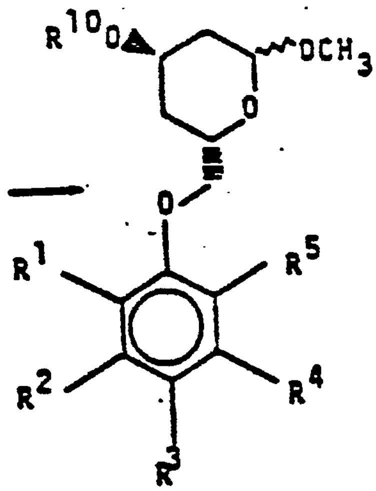

IV

worin $R^1$, $R^2$, $R^3$, $R^4$ und $R^5$ die zu Formel I und $R^{10}$ die zu Formel III angegebenen Bedeutungen haben, isoliert.

6. Verfahren gemäß Anspruch 1, dadurch gekennzeichnet, daß man die Zwischenstufe der Formel V

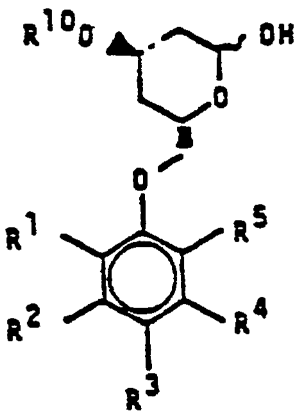

V

worin $R^1$ bis $R^5$ und $R^{10}$ die zu Formel I bzw. III angegebenen Bedeutungen haben, isoliert.

7. Verfahren gemäß Anspruch 1, dadurch gekennzeichnet, daß man die Zwischenstufe der Formel VI

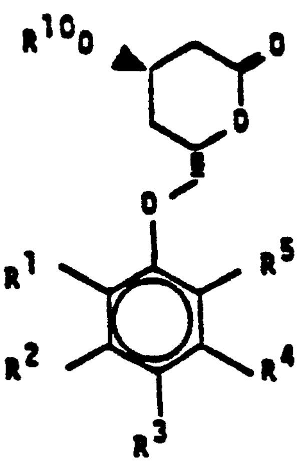

VI

worin $R^1$ bis $R^5$ die zu Formel I und $R^{10}$ die zu Formel III angegebenen Bedeutungen haben, isoliert.

**Claims for the Contracting States: BE, CH, DE, FR, GB, IT, LI, LU, NL, SE**

1. A 6-phenoxymethyl-4-hydroxytetrahydropyran-2-one of the general formula I

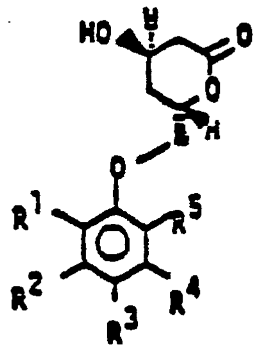

I

in which
$R^1$ and $R^5$ are identical or different and denote
a) halogen, b) cycloalkyl having 4–8 carbon atoms, or a phenyl radical which can be substituted in the nucleus once to 3 times by halogen, trifluoromethyl, and/or alkyl or alkoxy each having 1–4 carbon atoms, or c) a straight-chain or branched alkyl radical having 1 to 18 carbon atoms, or a straight-chain or branched alkenyl radical having 2 to 18 carbon atoms, it being possible for the alkyl and alkenyl radicals in turn to be substituted 1–3 times by

α) straight-chain or branched alkoxy radicals having up to 10 carbon atoms, or cycloalkyl radicals having 3 to 7 carbon atoms, or straight-chain or branched alkenyloxy or alkynyloxy radicals having 3 to 6 carbon atoms,

β) halogen, hydroxyl, cycloalkyl having 3–7 carbon atoms, unsubstituted phenyl or α- or β-thienyl radicals, or phenyl or α- or β-thienyl radicals which in turn are substituted in the nucleus once to 3 times by halogen, trifluoromethyl and/or alkyl or alkoxy having 1–4 carbon atoms,

γ) unsubstituted phenoxy, benzyloxy, α- or β-thienyloxy radicals, or phenoxy, benzyloxy, α- or β-thienyloxy radicals which in turn are substituted in the nucleus once to 3 times by halogen, trifluoromethyl and/or alkyl or alkoxy having 1 to 4 carbon atoms,

δ) the group

$$-O-\overset{\overset{O}{\|}}{C}-R^6,$$

$R^6$ denoting: a straight-chain or branched alkyl or alkenyl radical having up to 8 carbon atoms, or a cycloalkyl or cycloalkenyl radical each having 3–8 carbon atoms, or an unsubstituted phenyl radical, or a phenyl radical which in turn is substituted in the nucleus once to 3 times by halogen, trifluoromethyl and/or alkyl or alkoxy having 1–4 carbon atoms, or a 3-pyridyl radical,

$R^2$ and $R^4$ denote hydrogen, and
$R^3$ is alkyl or alkenyl having up to 4 carbon atoms, halogen or alkoxy having 1–4 carbon atoms, and the corresponding open-chain dihydroxy carboxylic acid, their pharmacologically tolerated salts with bases, and their pharmacologically tolerated esters.

2. A compound as claimed in claim 1, wherein in formula I $R^1$ and $R^5$ are both the same or are different and represent

a) halogen,

b) cycloalkyl having 5 to 6 carbon atoms, phenyl which can be substituted in the nucleus once to 3 times by halogen, trifluoromethyl and/or alkyl or alkoxy each having 1–4 carbon atoms, or

c) 1. straight-chain or branched alkyl or alkenyl having up to 12 carbon atoms, it being possible for the alkyl or alkenyl radical in turn to be substituted 1–2 times by phenyl radicals which in turn can be substituted in the nucleus 1 to 3 times by halogen, trifluoromethyl and/or alkyl or alkoxy having 1–4 carbon atoms,

2. straight-chain alkyl substituted by O–COR$^6$, of the formula

$$-(CH_2)_nO-\overset{\overset{O}{\|}}{C}-R^6$$

in which n denotes 1 to 3, and $R^6$ denotes a straight-chain or branched alkyl or alkenyl radical having up to 8 carbon atoms, a cycloalkyl radical having 5 to 6 carbon atoms, or a phenyl radical which

in turn can be substituted in the nucleus 1–3 times by halogen, trifluoromethyl and/or alkyl or alkoxy having 1 to 4 carbon atoms,

3. straight-chain alkyl substituted by $OR^7$, of the formula

$$-(CH_2)_nOR^7$$

in which n denotes 1 to 3, and $R^7$ denotes hydrogen or a straight-chain or branched alkyl or alkenyl radical having up to 8 carbon atoms, a cycloalkyl radical having 5 to 6 carbon atoms, or a phenyl radical or benzyl radical which in turn can each be substituted in the aromatic nucleus 1–3 times by halogen, trifluoromethyl and/or alkyl or alkoxy having 1 to 4 carbon atoms,

$R^2$ and $R^4$ denote hydrogen, and

$R^3$ denotes methyl, ethyl, propyl, 1-propenyl, allyl, fluorine or chlorine.

3. A compound as claimed in claim 1, wherein in formula I $R^1$ and $R^5$ are identical or different and represent

1) methyl, ethyl, propyl, allyl, 1-propenyl, isopropenyl, isopropyl, cyclopentyl or cyclohexyl, or

2) the group

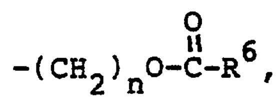

with n = 1–3, $R^6$ denoting: methyl, ethyl, propyl, i-propyl, n-butyl, i-butyl, t-butyl, phenyl, or phenyl which is substituted in the nucleus once to 3 times by halogen, methyl or methoxy, or

3) the group $-(CH_2)_nOR^7$, with n = 1 to 3, $R^7$ denoting: hydrogen, methyl, straight-chain or branched alkyl or alkenyl having 3–5 carbon atoms, cyclopentyl, cyclohexyl; phenyl or benzyl, it being possible for the aromatic nuclei to be substituted 1–3 times by fluorine, chlorine, methyl or methoxy, or

4) an alkyl group of the formula

$$-(CH_2)_nCHR^8R^9$$

in which n is 0 to 2 and $R^8$ and $R^9$ are identical or different and denote hydrogen, methyl, ethyl, propyl, allyl, i-propyl, n-butyl, i-butyl, t-butyl, cyclohexyl, cyclopentyl, benzyl or phenyl, it being possible for the aromatic nuclei to be substituted once to 3 times by fluorine, chlorine, methyl or methoxy,

$R^2$ and $R^4$ denote hydrogen, and

$R^3$ denotes methyl, chlorine or fluorine.

4. Process for the preparation of a compound as claimed in claim 1, which comprises

a) conversion of an appropriately substituted phenol of the formula II

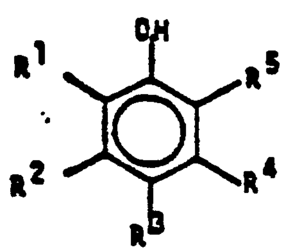

II

in which $R^1$, $R^2$, $R^3$, $R^4$ and $R^5$ have the indicated meanings, with the chiral iodide of the formula III

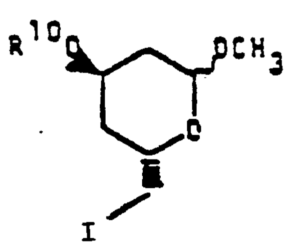

III

in which $R^{10}$ denotes a protective group which is stable to bases and weak acids, into an ether of the formula IV

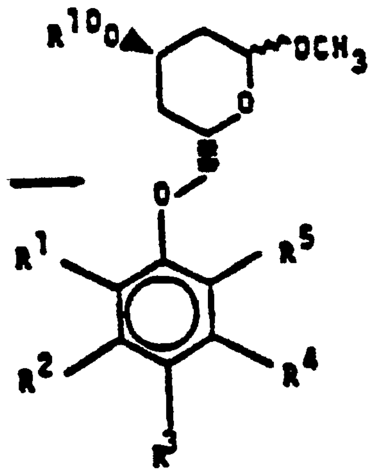

IV

in which $R^1$ to $R^5$ have the meanings indicated for formula I and $R^{10}$ has the meanings indicated for formula III,

b) hydrolysis of the ether of the formula IV to give the corresponding hemiacetal of the formula V

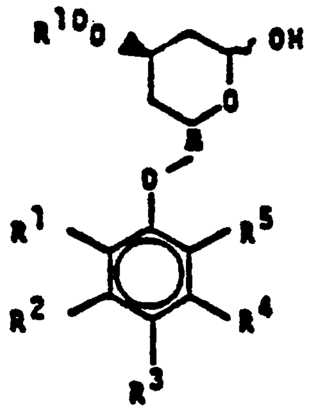

V

in which $R^1$ to $R^5$ and $R^{10}$ have the meanings indicated for formula I or III,

c) oxidation of the hemiacetal of the formula V to give the corresponding lactone of the formula VI

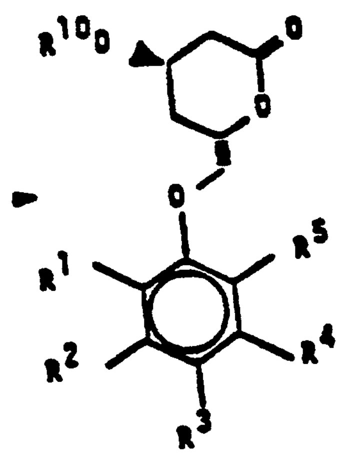

VI

in which $R^1$ to $R^5$ have the meanings indicated for formula I and $R^{10}$ has the meanings indicated for formula III, and

d) conversion of the protected hydroxylactone of the formula VI into the 6-phenoxymethyl-4-hydroxytetrahydropyran-2-one of the formula I

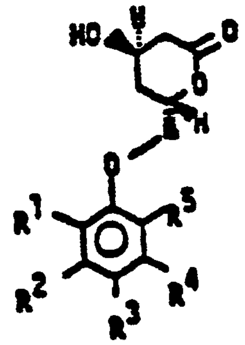

I

where appropriate the compound of the formula I which has been obtained being converted into the corresponding open-chain dihydroxy carboxylic acid of the formula VII

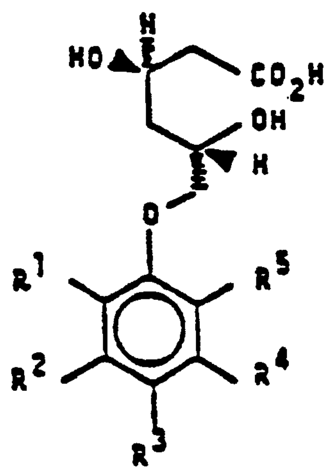

VII

its salt or its ester, where appropriate a salt or ester which has been obtained being converted into the free dihydroxy carboxylic acid or, where appropriate, the free carboxylic acid being converted into the salt or ester.

5. A pharmaceutical product containing a compound as claimed in claim 1.

6. The use of a compound as claimed in claim 1 for the preparation of a pharmaceutical product for the prophylaxis and therapy of hypercholesterolemia.

7. An ether of the formula IV

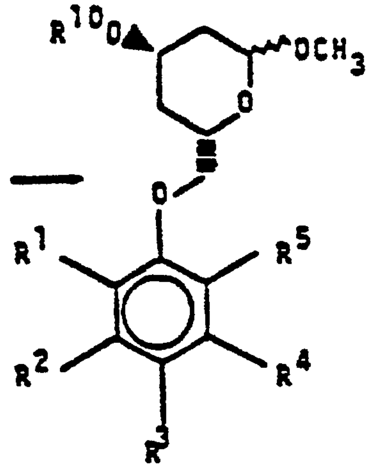

IV

in which $R^1$, $R^2$, $R^3$, $R^4$ and $R^5$ have the meanings indicated for formula I, and $R^{10}$ has the meanings indicated for formula III.

8. A hemiacetal of the formula V

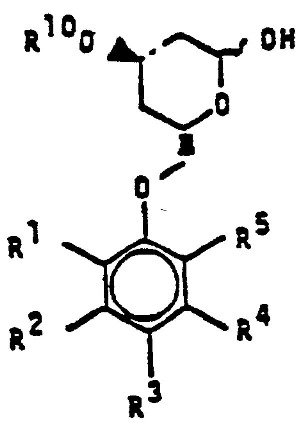

V

in which $R^1$ to $R^5$ and $R^{10}$ have the meanings indicated for formula I or III.

9. A lactone of the formula VI

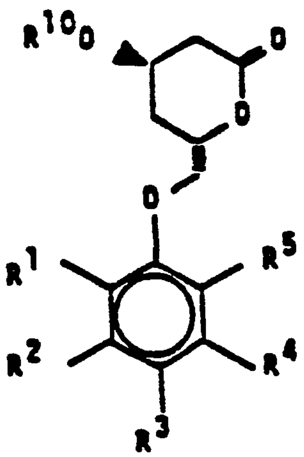

VI

in which $R^1$ to $R^5$ have the meanings indicated for formula I, and $R^{10}$ has the meanings indicated for formula III.

**Claims for the Contracting State: AT**

1. A process for the preparation of a 6-phenoxymethyl-4-hydroxytetrahydropyran-2-one of the general formula I

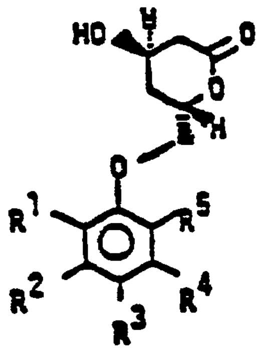

I

in which
$R^1$ and $R^5$ are identical or different and denote
a) halogen, b) cycloalkyl having 4–8 carbon atoms, or a phenyl radical which can be substituted in the nucleus once to 3 times by halogen, trifluoromethyl and/or alkyl or alkoxy each having 1–4 carbon atoms, or c) a straight-chain or branched alkyl radical having 1 to 18 carbon atoms, or a straight-chain or branched alkenyl radical having 2 to 18 carbon atoms, it being possible for the alkyl and alkenyl radicals in turn to be substituted 2–3 times by

α) straight-chain or branched alkoxy radicals having up to 10 carbon atoms, or cycloalkoxy radicals having 3 to 7 carbon atoms, or straight-chain or branched alkenyloxy or alkynyloxy radicals having 3 to 6 carbon atoms,

β) halogen, hydroxyl, cycloalkyl having 3–7 carbon atoms, unsubstituted phenyl or α- or β-thienyl radicals, or phenyl or α- or β-thienyl radicals which in turn are substituted in the nucleus once to 3 times by halogen, trifluoromethyl and/or alkyl or alkoxy having 1–4 carbon atoms,

γ) unsubstituted phenoxy, benzyloxy, α- or β-thienyloxy radicals, or phenoxy, benzyloxy, α- or β-thienyloxy radicals which in turn are substituted in the nucleus once to 3 times by halogen, trifluoromethyl and/or alkyl or alkoxy having 1 to 4 carbon atoms,

δ) the group

$$-O-\overset{O}{\overset{\|}{C}}-R^6,$$

$R^6$ denoting: a straight-chain or branched alkyl or alkenyl having up to 8 carbon atoms, or a cycloalkyl or cycloalkenyl radical each having 3–8 carbon atoms, or an unsubstituted phenyl radical, or a phenyl radical which in turn is substituted in the nucleus once to 3 times by halogen, trifluoromethyl and/or alkyl or alkoxy having 1–4 carbon atoms, or a 3-pyridyl radical,

$R^2$ and $R^4$ denote hydrogen, and
$R^3$ is alkyl or alkenyl having up to 4 carbon atoms, halogen or alkoxy having 1–4 carbon atoms, and the corresponding open-chain dihydroxy carboxylic acid, their pharmacologically tolerated salts with bases, and their pharmacologically tolerated esters, which comprises

a) conversion of an appropriately substituted phenol of the formula II

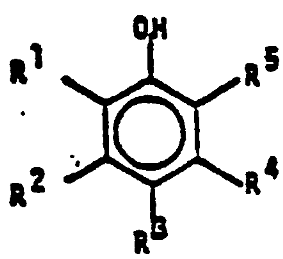

II

in which $R^1$, $R^2$, $R^3$, $R^4$ and $R^5$ have the indicated meanings, with the chiral iodide of the formula III

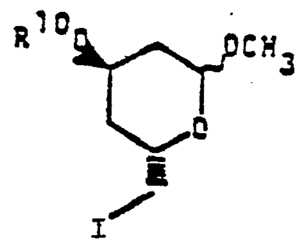

III

in which $R^{10}$ denotes a protective group which is stable to bases and weak acids, into an ether of the formula IV

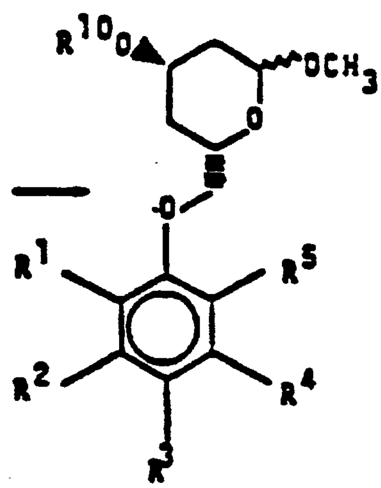

IV

in which $R^1$ to $R^5$ have the meanings indicated for formula I and $R^{10}$ has the meanings indicated for formula III,

b) hydrolysis of the ether of the formula IV to give the corresponding hemiacetal of the formula V

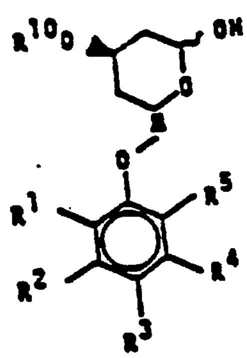

V

in which $R^1$ to $R^5$ and $R^{10}$ have the meanings indicated for formula I or III,

c) oxidation of the hemiacetal of the formula V to give the corresponding lactone of the formula VI

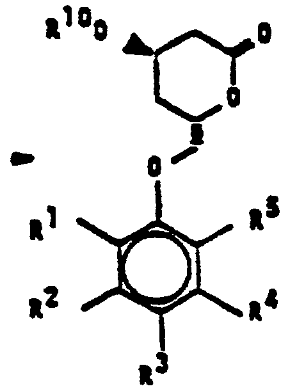

VI

in which $R^1$ to $R^5$ have he meanings indicated for formula I and $R^{10}$ has the meanings indicated for formula III, and

d) conversion of the protected hydroxylactone of the formula VI into the 6-phenoxymethyl-4-hydroxytetrahydropyran-2-one of the formula I

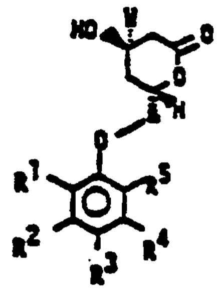

I

where appropriate the compound of the formula I which has been obtained being converted into the corresponding open-chain dihydroxy carboxylic acid of the formula VII

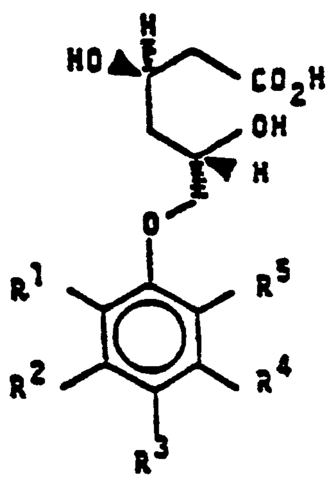

VII

its salt or its ester, where appropriate a salt or ester which has been obtained being converted into the free dihydroxy carboxylic acid or, where appropriate, the free carboxylic acid being converted into the salt or ester.

2. The process as claimed in claim 1, in which is prepared a compound of the formula I in which $R^1$ and $R^5$ are both the same or are different and represent

a) halogen,

b) cycloalkyl having 5 to 6 carbon atoms, phenyl which can be substituted in the nucleus once to 3 times by halogen, trifluoromethyl and/or alkyl or alkoxy each having 1–4 carbon atoms, or

c) 1. straight-chain or branched alkyl or alkenyl having up to 12 carbon atoms, it being possible for the alkyl or alkenyl radical in turn to be substituted 1–2 times by phenyl radicals which in turn can be substituted in the nucleus 1 to 3 times by halogen, trifluoromethyl and/or alkyl or alkoxy having 1–4 carbon atoms,
2. straight-chain alkyl substituted by O–COR$^6$, of the formula

$$-(CH_2)_nO-\overset{\overset{\displaystyle O}{\|}}{C}-R^6$$

in which n denotes 1 to 3, and R$^6$ denotes a straight-chain or branched alkyl or alkenyl radical having up to 8 carbon atoms, a cycloalkyl radical having 5 to 6 carbon atoms, or a phenyl radical which in turn can be substituted in the nucleus 1–3 times by halogen, trifluoromethyl and/or alkyl or alkoxy having 1 to 4 carbon atoms,
3. straight-chain alkyl substituted by OR$^7$, of the formula

$$-(CH_2)_nOR^7$$

in which n denotes 1 to 3, and R$^7$ denotes hydrogen or a straight-chain or branched alkyl or alkenyl radical having up to 8 carbon atoms, a cycloalkyl radical having 5 to 6 carbon atoms, or a phenyl radical or benzyl radical which in turn can each be substituted in the aromatic nucleus 1–3 times by halogen, trifluoromethyl and/or alkyl or alkoxy having 1 to 4 carbon atoms,
R$^2$ and R$^4$ denote hydrogen, and
R$^3$ denotes methyl, ethyl, propyl, 1-propenyl, allyl, fluorine or chlorine.

3. The process as claimed in claim 1, in which is prepared a compound of the formula I in which R$^1$ and R$^5$ are identical or different and represent
1) methyl, ethyl, propyl, allyl, l-propenyl, isopropenyl, isopropyl, cyclopentyl or cyclohexyl, or
2) the group

$$-(CH_2)_nO-\overset{\overset{\displaystyle O}{\|}}{C}-R^6,$$

with n = 1–3, R$^6$ denoting: methyl, ethyl, propyl, i-propyl, n-butyl, i-butyl, t-butyl, phenyl, or phenyl which is substituted in the nucleus once to 3 times by halogen, methyl or methoxy, or
3) the group $-(CH_2)_nOR^7$, with n = 1 to 3, R$^7$ denoting: hydrogen, methyl, straight-chain or branched alkyl or alkenyl having 3–5 carbon atoms, cyclopentyl, cyclohexyl; phenyl or benzyl, it being possible for the aromatic nuclei to be substituted 1–3 times by fluorine, chlorine, methyl or methoxy, or
4) an alkyl group of the formula

$$-(CH_2)_nCHR^8R^9$$

in which n is 0 to 2 and R$^8$ and R$^9$ are identical or different and denote hydrogen, methyl, ethyl, propyl, allyl, i-propyl, n-butyl, i-butyl, t-butyl, cyclohexyl, cyclopentyl, benzyl or phenyl, it being possible for the aromatic nuclei to be substituted once to 3 times by fluorine, chlorine, methyl or methoxy,
R$^2$ and R$^4$ denote hydrogen, and
R$^3$ denotes methyl, chlorine or fluorine.

4. A process for the preparation of a pharmaceutical product, which comprises converting a compound obtainable by the process as claimed in claim 1 into a suitable form for administration.

5. The process as claimed in claim 1, in which is isolated an intermediate of the formula (IV)

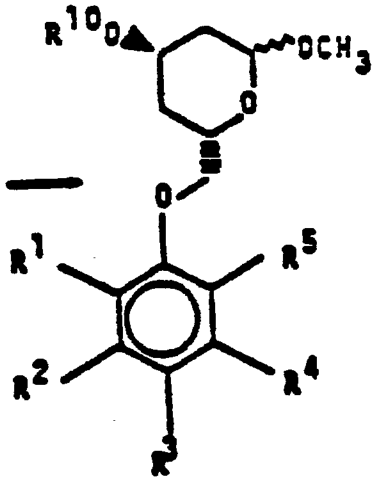

IV

in which $R^1$, $R^2$, $R^3$, $R^4$ and $R^5$ have the meanings indicated for formula I, and $R^{10}$ has the meanings indicated for formula III.

6. The process as claimed in claim 1, in which is isolated the intermediate of the formula V

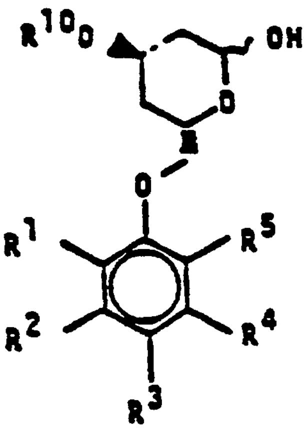

V

in which $R^1$ to $R^5$ and $R^{10}$ have the meanings indicated for formula I or III.

7. The process as claimed in claim 1, in which is isolated the intermediate of the formula VI

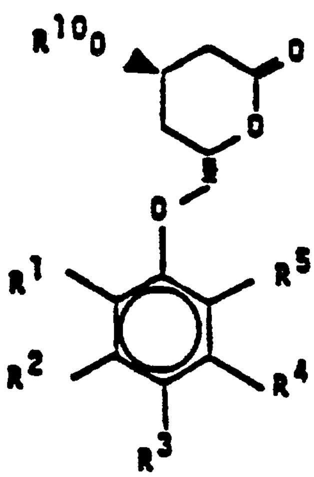

VI

in which $R^1$ to $R^5$ have the meanings indicated for formula I, and $R^{10}$ has the meanings indicated for formula III.

**Revendications pour les Etats contractants: BE, CH, DE, FR, GB, IT, LI, LU, NL, SE**

1. 6-phénoxyméthyl-4-hydroxytétrahydropyranne-2-ones de formule générale I

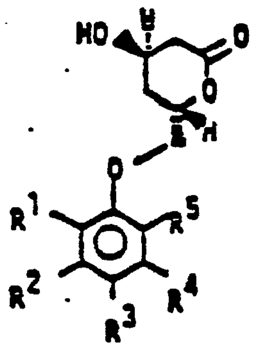

(I)

dans laquelle

$R^1$ et $R^5$ sont identiques ou différents et représentent a) un halogène, b) un radical cycloalkyle ayant de 4 à 8 atomes de carbone ou un radical phényle qui peut être substitué de 1 à 3 fois au noyau par un halogène ou par un groupe trifluorométhyle et/ou alkyle ou alcoxy ayant chacun de 1 à 4 atomes de carbone, ou c) un radical alkyle à chaîne droite ou ramifiée ayant de 1 à 18 atomes de carbone, ou un radical alcényle à chaîne droite ou ramifiée ayant de 2 à 18 atomes de carbone, les radicaux alkyle ou alcényle pouvant pour leur part être 1 à 3 fois substitués par

α) des restes alcoxy à chaînes droites ou ramifiées ayant jusqu'à 10 atomes de carbone ou des restes cycloalcoxy ayant de 3 à 7 atomes de carbone, ou des restes alcényloxy ou alcinyloxy à chaînes droites ou ramifiées ayant de 3 à 6 atomes de carbone;

β) des halogènes, des restes hydroxy, cycloalkyle ayant de 3 à 7 atomes de carbone, des restes phényle, α- ou β-thiényle non substitués, ou des restes phényle, α- ou β-thiényle qui sont pour leur part de 1 à 3 fois substitués au noyau par des halogènes ou par le groupe trifluorométhyle et/ou des groupes alkyle ou alcoxy ayant de 1 à 4 atomes de carbone;

γ) des restes phénoxy, benzyloxy, α- ou β-thiényloxy non substitués, ou des restes phénoxy, benzyloxy, α- ou β-thiényloxy qui sont pour leur part de 1 à 3 fois substitués au noyau par des halogènes, le groupe trifluorométhyle et/ou des groupes alkyle ou alcoxy ayant de 1 à 4 atomes de carbone;

δ) le groupe

$$-O-\overset{\overset{O}{\|}}{C}-R^6,$$

$R^6$ représentant:

un reste alkyle ou alcényle à chaîne droite ou ramifiée ayant jusqu'à 8 atomes de carbone, ou un reste cycloalkyle ou cycloalcényle ayant chacun de 3 à 8 atomes de carbone, ou un reste phényle non substitué ou un reste phényle qui pour sa part est de 1 à 3 fois substitué au noyau par des halogènes, le groupe trifluorométhyle et/ou des groupes alkyle ou alcoxy ayant de 1 à 4 atomes de carbone, ou le reste 3-pyridyle;

$R^2$ et $R^4$ représentent des atomes d'hydrogène; et

$R^3$ représente un groupe alkyle ou alcényle ayant jusqu'à 4 atomes de carbone, un atome d'halogène ou un groupe alcoxy ayant de 1 à 4 atomes de carbone, ainsi que les acides dihydroxycarboxyliques à chaînes ouvertes correspondants, leurs sels pharmacologiquement acceptables avec des bases et leurs esters pharmacologiquement acceptables.

2. Composés selon la revendication 1, caractérisés en ce que, dans la formule I,

$R^1$ et $R^5$ sont identiques ou différents, et représentent

a) un atome d'halogène,

b) un radical cycloalkyle ayant 5 ou 6 atomes de carbone, phényle, qui peut être de 1 à 3 fois substitué au noyau par des atomes d'halogène, le groupe trifluorométhyle et/ou des groupes alkyle ou alcoxy ayant chacun de 1 à 4 atomes de carbone, ou

c) 1. un radical alkyle ou alcényle à chaîne droite ou ramifiée ayant jusqu'à 12 atomes de carbone, le radical alkyle ou alcényle pouvant être, pour sa part, 1 ou 2 fois substitué par des restes phényle qui, pour leur part, peuvent être de 1 à 3 fois substitués au noyau par des atomes d'halogène, le groupe trifluorométhyle et/ou des groupes alkyle ou alcoxy ayant de 1 à 4 atomes de carbone,

2. un groupe alkyle à chaîne droite substitué par O–COR⁶, de formule

$$-(CH_2)_nO-\overset{\overset{O}{\|}}{C}-R^6$$

dans laquelle n = 1 à 3 et $R^6$ représente un reste alkyle ou alcényle à chaîne droite ou ramifiée ayant jusqu'à 8 atomes de carbone, un reste cycloalkyle ayant 5 ou 6 atomes de carbone, ou un reste phényle qui, pour sa part, peut être de 1 à 3 fois substitué au noyau par des atomes d'halogène, le groupe trifluorométhyle et/ou des groupes alkyle ou alcoxy ayant jusqu'à 4 atomes de carbone;

3. un groupe alkyle à chaîne droite, substitué par $OR^7$, de formule

$$-(CH_2)_nOR^7,$$

dans laquelle n = 1 à 3 et $R^7$ représente un atome d'hydrogène ou un reste alkyle ou alcényle à chaîne droite ou ramifiée ayant jusqu'à 8 atomes de carbone, un reste cycloalkyle ayant 5 ou 6 atomes de carbone, ou un reste phényle ou benzyle, qui pour leur part peuvent être de 1 à 3 fois substitués sur le noyau aromatique par des atomes d'halogène, le groupe trifluorométhyle et/ou des groupes alkyle ou alcoxy ayant de 1 à 4 atomes de carbone;

$R^2$ et $R^4$ représentent des atomes d'hydrogène;

$R^3$ représente le groupe méthyle, éthyle, propyle, 1-propényle ou allyle ou un atome de fluor ou de chlore.

3. Composé selon la revendication 1, caractérisés en ce que, dans la formule I,

$R^1$ et $R^5$ sont identiques ou différents, et représentent:

1) le groupe méthyle, éthyle, propyle, allyle, l-propényle, isopropényle, isopropyle, cyclopentyle, cyclohexyle, ou

2) un groupe

$$-(CH_2)_nO-\overset{\overset{O}{\|}}{C}-R^6,$$

avec n = 1–3, $R^6$ représentant le groupe méthyle, éthyle, propyle, isopropyle, n-butyle, isobutyle, tert-butyle, phényle ou un groupe phényle de 1 à 3 fois substitué au noyau par des halogènes ou par le groupe méthyle ou méthoxy, ou

3) un groupe $-(CH_2)_nOR^7$, avec n = 1–3, $R^7$ représentant un atome d'hydrogène, le groupe méthyle ou un groupe alkyle ou alcényle à chaîne droite ou ramifiée ayant de 3 à 5 atomes de carbone, le groupe cyclopentyle, cyclohexyle, phényle ou benzyle, les noyaux aromatiques pouvant être de 1 à 3 fois substitués par des atomes de fluor ou de chlore ou par le groupe méthyle ou méthoxy, ou

4) un groupe alkyle de formule

$$-(CH_2)_nCHR^8R^9,$$

dans laquelle n = 0–2 et $R^8$ et $R^9$ sont identiques ou différents et représentent un atome d'hydrogène ou le groupe méthyle, éthyle, propyle, allyle, isopropyle, n-butyle, isobutyle, tert-butyle, cyclohexyle, cyclopentyle, benzyle ou phényle, les noyaux aromatiques pouvant être de 1 à 3 fois substitués par des atomes de fluor ou de chlore ou par le groupe méthyle ou méthoxy;

$R^2$ et $R^4$ représentent des atomes d'hydrogène;

$R^3$ représente un atome de fluor ou de chlore ou le groupe méthyle.

4. Procédé pour la préparation des composés selon la revendication 1, caractérisé en ce que

a) on convertit des phénols convenablement substitués, de formule II

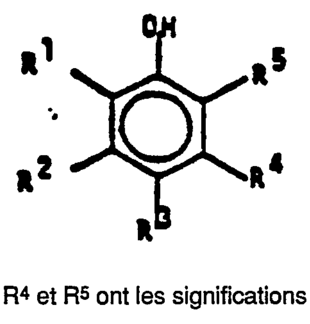

(II)

dans laquelle $R^1$, $R^2$, $R^3$, $R^4$ et $R^5$ ont les significations indiquées, avec l'iodure chiral de formule III

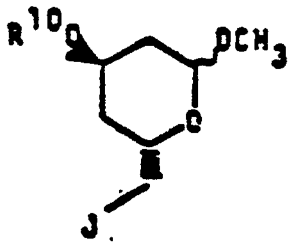

(III)

dans laquelle $R^{10}$ représente un groupe protecteur stable vis-à-vis d'acides faibles et de bases, en les éthers de formule IV

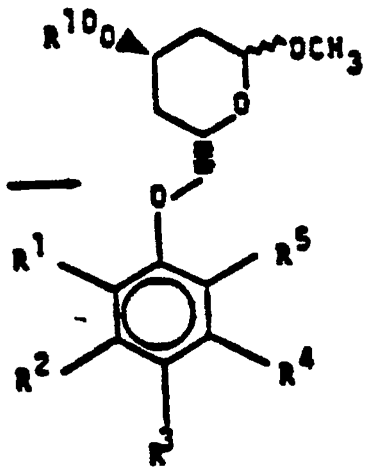

(IV)

dans laquelle $R^1$ à $R^5$ ont les significations données à propos de la formule I et $R^{10}$ a la signification donnée à propos de la formule III;

b) on hydrolyse les éthers de formule IV en les hémi-acétals de formule V correspondants

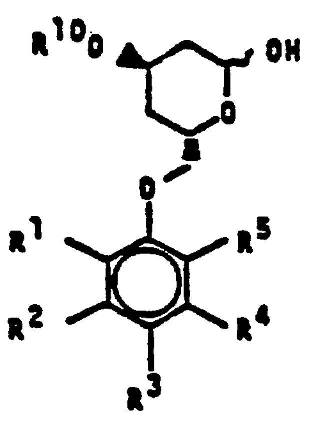

(V)

dans laquelle $R^1$ à $R^5$ et $R^{10}$ ont les significations données à propos de la formule I ou III, respectivement,

c) on oxyde les hémi-acétals de formule V en les lactones correspondantes de formule VI

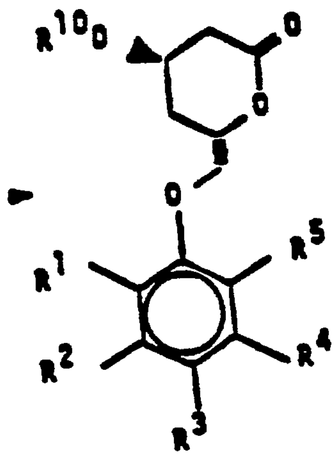

(VI)

dans laquelle $R^1$ à $R^5$ ont les significations données à propos de la formule I et $R^{10}$ a la signification donnée à propos de la formule III, et
d) on convertit les hydroxylactones protégées de formule VI en les 6-phénoxyméthyl-4-hydroxytétrahydropyranne-2-ones de formule I

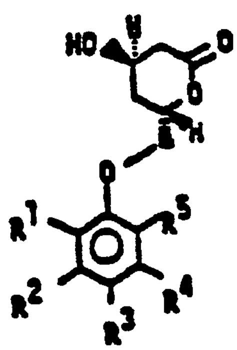

(I)

éventuellement, on convertit les composés de formule I obtenus en les acides dihydroxycarboxyliques à chaînes ouvertes correspondants de formule VII

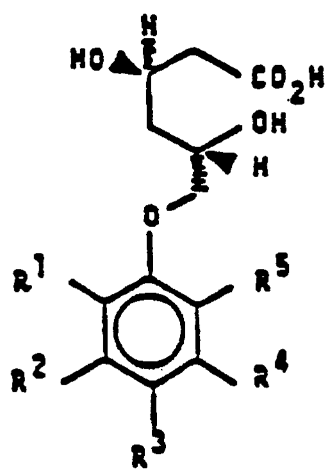

(VII)

en leurs sels ou en leurs esters, éventuellement on convertit les sels ou les esters obtenus en les acides dihydroxycarboxyliques libres, ou éventuellement on convertit les acides carboxyliques libres en les sels ou esters.

5. Compositions pharmaceutiquement contenant un composé selon la revendication 1.

6. Utilisation d'un composé selon la revendication 1, pour la préparation d'une composition pharmaceutique pour la prophylaxie et le traitement de l'hypercholestérolémie.

7. Ethers de formule IV

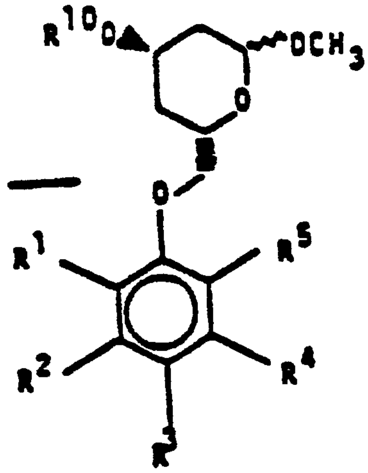

dans laquelle $R^1$, $R^2$, $R^3$, $R^4$ et $R^5$ ont les significations données à propos de la formule I et $R^{10}$ a la signification donnée à propos de la formule III.

8. Hémi-acétals de formule V

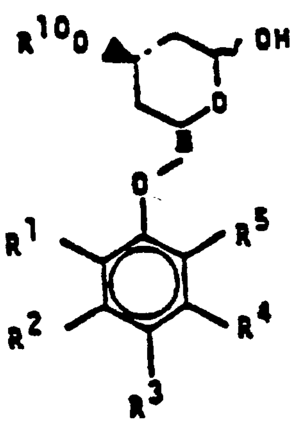

(V)

dans laquelle $R^1$ à $R^5$ et $R^{10}$ ont les significations données à propos de la formule I ou respectivement III.

9. Lactones de formule VI

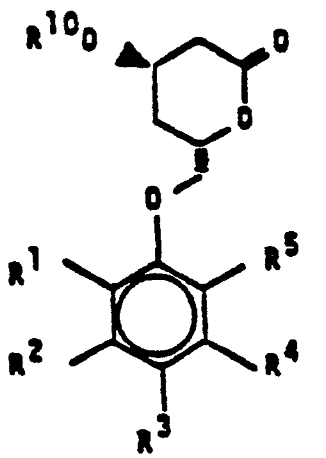

(VI)

dans laquelle $R^1$ à $R^5$ ont les significations données à propos de la formule I et $R^{10}$ a la signification donnée à propos de la formule III.

**Revendications pour l'Etat contractant: AT**

1. Procédé pour la préparation de 6-phénoxyméthyl-4-hydroxytétrahydropyranne-2-ones de formule générale I

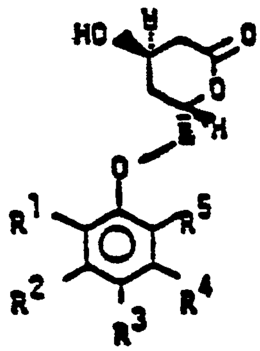

(I)

dans laquelle
$R^1$ et $R^5$ sont identiques ou différents et représentent a) un halogène, b) un radical cycloalkyle ayant de 4 à 8 atomes de carbone ou un radical phényle qui peut être substitué de 1 à 3 fois au noyau par un halogène ou par un groupe trifluorométhyle et/ou alkyle ou alcoxy ayant chacun de 1 à 4 atomes de carbone, ou c) un radical alkyle à chaîne droite ou ramifiée ayant de 1 à 18 atomes de carbone, ou un radical alcényle à chaîne droite ou ramifiée ayant de 2 à 18 atomes de carbone, les radicaux alkyle ou alcényle pouvant pour leur part être 1 à 3 fois substitués par

α) des restes alcoxy à chaînes droites ou ramifiées ayant jusqu'à 10 atomes de carbone ou des restes cycloalcoxy ayant de 3 à 7 atomes de carbone, ou des restes alcényloxy ou alcinyloxy à chaînes droites ou ramifiées ayant de 3 à 6 atomes de carbone;
β) des halogènes, des restes hydroxy, cycloalkyle ayant de 3 à 7 atomes de carbone, des restes phényle, α- ou β-thiényle non substitués, ou des restes phényle, α- ou β-thiényle qui sont pour leur part de 1 à 3 fois substitués au noyau par des halogènes ou par le groupe trifluorométhyle et/ou des groupes alkyle ou alcoxy ayant de 1 à 4 atomes de carbone;
γ) des restes phénoxy, benzyloxy, α- ou β-thiényloxy non substitués, ou des restes phénoxy, benzyloxy, α- ou β-thiényloxy qui sont pour leur part de 1 à 3 fois substitués au noyau par des halogènes, le groupe trifluorométhyle et/ou des groupes alkyle ou alcoxy ayant de 1 à 4 atomes de carbone;
δ) le groupe

$$-O-\overset{O}{\overset{\|}{C}}-R^6,$$

$R^6$ représentant: un reste alkyle ou alcényle à chaîne droite ou ramifiée ayant jusqu'à 8 atomes de carbone, ou un reste cycloalkyle ou cycloalcényle ayant chacun de 3 à 8 atomes de carbone, ou un reste phényle non substitué ou un reste phényle qui pour sa part est de 1 à 3 fois substitué au noyau par des halogènes, le groupe trifluorométhyle et/ou des groupes alkyle ou alcoxy ayant de 1 à 4 atomes de carbone, ou le reste 3-pyridyle;

$R^2$ et $R^4$ représentent des atomes d'hydrogène; et
$R^3$ représente un groupe alkyle ou alcényle ayant jusqu'à 4 atomes de carbone, un atome d'halogène ou un groupe alcoxy ayant de 1 à 4 atomes de carbone,
ainsi que des acides dihydroxycarboxyliques à chaînes ouvertes correspondants, de leurs sels pharmacologiquement acceptables avec des bases et de leurs esters pharmacologiquement acceptables, caractérisé en ce que

a) on convertit des phénols convenablement substitués, de formule II

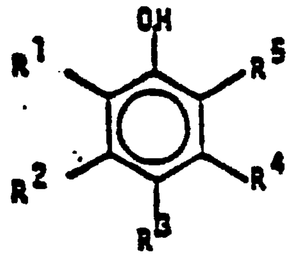

(II)

dans laquelle $R^1$, $R^2$, $R^3$, $R^4$ et $R^5$ ont les significations indiquées, avec l'iodure chiral de formule III

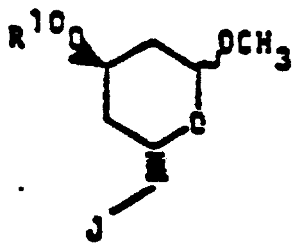

(III)

dans laquelle $R^{10}$ représente un groupe protecteur stable vis-à-vis d'acides faibles et de bases, en les éthers de formule IV

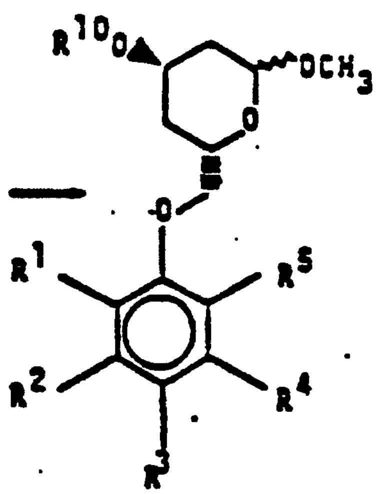

(IV)

dans laquelle $R^1$ à $R^5$ ont les significations données à propos de la formule I et $R^{10}$ a la signification donnée à propos de la formule III;
b) on hydrolyse les éthers de formule IV en les hémi-acétals de formule V correspondants

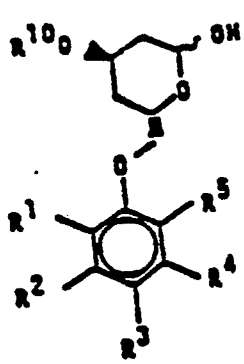

(V)

dans laquelle $R^1$ à $R^5$ et $R^{10}$ ont les significations données à propos de la formule I ou III, respectivement,

c) on oxyde les hémi-acétals de formule V en les lactones correspondantes de formule VI

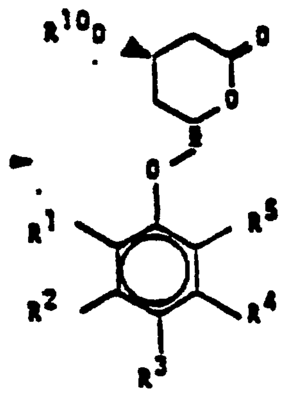

(VI)

dans laquelle $R^1$ à $R^5$ ont les significations données à propos de la formule I et $R^{10}$ a la signification donnée à propos de la formule III, et

d) on convertit les hydroxylactones protégées de formule VI en les 6-phénoxyméthyl-4-hydroxy-tétrahydropyranne-2-ones de formule I

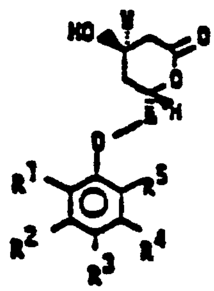

(I)

éventuellement, on convertit les composés de formule I obtenus en les acides dihydroxycarboxyliques à chaînes ouvertes correspondants de formule VII

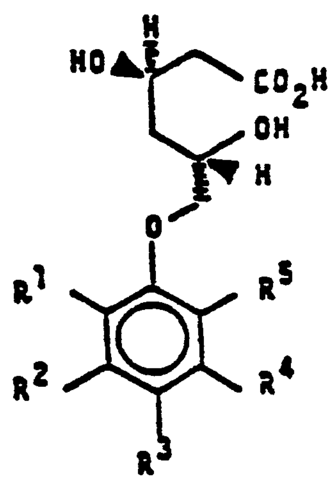

(VII)

en leurs sels ou en leurs esters, éventuellement on convertit les sels ou esters obtenus en les acides dihydroxycarboxyliques libres, ou éventuellement on convertit les acides carboxyliques libres en les sels ou esters.

2. Procédé selon la revendication 1, caractérisé en ce que l'on prépare des composés de formule I dans lesquels

$R^1$ et $R^5$ sont identiques ou différents, et représentent

a) un atome d'halogène,

b) un radical cycloalkyle ayant 5 à 6 atomes de carbone, phényle, qui peut être de 1 à 3 fois substitué au noyau par des atomes d'halogène, le groupe trifluorométhyle et/ou des groupes alkyle ou alcoxy ayant chacun de 1 à 4 atomes de carbone, ou

c) 1. un radical alkyle ou alcényle à chaîne droite ou ramifiée ayant jusqu'à 12 atomes de carbone, le radical alkyle ou alcényle pouvant être, pour sa part, 1 ou 2 fois substitué par des restes phényle qui, pour leur part, peuvent être de 1 à 3 fois substitués au noyau par des atomes d'halogène, le groupe trifluorométhyle et/ou des groupes alkyle ou alcoxy ayant de 1 à 4 atomes de carbone,

2. un groupe alkyle à chaîne droite substitué par $O{-}COR^6$, de formule

$$-(CH_2)_nO-\overset{\overset{O}{\|}}{C}-R^6$$

dans laquelle n = 1 à 3 et $R^6$ représente un reste alkyle ou alcényle à chaîne droite ou ramifiée ayant jusqu'à 8 atomes de carbone, un reste cycloalkyle ayant 5 ou 6 atomes de carbone, ou un reste phényle qui, pour sa part, peut être de 1 à 3 fois substitué au noyau par des atomes d'halogène, le groupe trifluorométhyle et/ou des groupes alkyle ou alcoxy ayant jusqu'à 4 atomes de carbone;

3. un groupe alkyle à chaîne droite, substitué par $OR^7$, de formule

$$-(CH_2)_nOR^7,$$

dans laquelle n = 1 à 3 et $R^7$ représente un atome d'hydrogène ou un reste alkyle ou alcényle à chaîne droite ou ramifiée ayant jusqu'à 8 atomes de carbone, un reste cycloalkyle ayant 5 à 6 atomes de carbone, ou un reste phényle ou benzyle, qui pour leur part peuvent être de 1 à 3 fois substitués sur le noyau aromatique par des atomes d'halogène, le groupe trifluorométhyle et/ou des groupes alkyle ou alcoxy ayant de 1 à 4 atomes de carbone;

$R^2$ et $R^4$ représentent des atomes d'hydrogène;

$R^3$ représente le groupe méthyle, éthyle, propyle, 1-propényle ou allyle ou un atome de fluor ou de chlore.

3. Procédé selon la revendication 1, caractérisé en ce que l'on prépare des composés de formule I dans lesquels $R^1$ et $R^5$ sont identiques ou différents, et représentent:

1) le groupe méthyle, éthyle, propyle, allyle, 1-propényle, isopropényle, isopropyle, cyclopentyle, cyclohexyle, ou

2) un groupe

$$-(CH_2)_nO-\overset{\overset{O}{\|}}{C}-R^6,$$

avec n = 1–3, $R^6$ représentant le groupe méthyle, éthyle, propyle, isopropyle, n-butyle, isobutyle, tert-butyle, phényle ou un groupe phényle de 1 à 3 fois substitué au noyau par des halogènes ou par le groupe méthyle ou méthoxy, ou

3) un groupe $-(CH_2)_nOR^7$, avec n = 1–3, $R^7$ représentant un atome d'hydrogène, le groupe méthyle ou un groupe alkyle ou alcényle à chaîne droite ou ramifiée ayant de 3 à 5 atomes de carbone, le groupe cyclopentyle, cyclohexyle, phényle ou benzyle, les noyaux aromatiques pouvant être de 1 à 3 fois substitués par des atomes de fluor ou de chlore par le groupe méthyle ou méthoxy, ou

4) un groupe alkyle de formule

$$-(CH_2)_nCHR^8R^9,$$

dans laquelle n = 0–2 et $R^8$ et $R^9$ sont identiques ou différents et représentent un atome d'hydrogène ou le groupe méthyle, éthyle, propyle, allyle, isopropyle, n-butyle, isobutyle, tert-butyle, cyclohexyle, cyclopentyle, benzyle ou phényle, les noyaux aromatiques pouvant être de 1 à 3 fois substitués par des atomes de fluor ou de chlore ou par le groupe méthyle ou méthoxy;

$R^2$ et $R^4$ représentent des atomes d'hydrogène;

$R^3$ représente un atome de fluor ou de chlore ou le groupe méthyle.

4. Procédé pour la préparation d'une composition pharmaceutique, caractérisé en ce que l'on convertit en une forme pharmaceutique appropriée un composé pouvant être obtenu par le procédé selon la revendication 1.

5. Procédé selon la revendication 1, caractérisé en ce que l'on isole un produit intermédiaire de formule IV

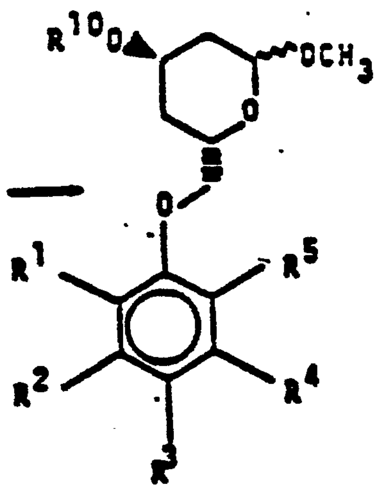

(IV)

dans laquelle $R^1$, $R^2$, $R^3$, $R^4$ et $R^5$ ont les significations données à propos de la formule I et $R^{10}$ a la signification donnée à propos de la formule III.

6. Procédé selon la revendication 1, caractérisé en ce que l'on isole un produit intermédiaire de formule V

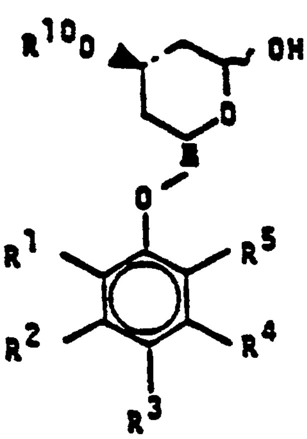

(V)

dans laquelle $R^1$ à $R^5$ et $R^{10}$ ont les significations données à propos de la formule I ou III, respectivement.

7. Procédé selon la revendication 1, caractérisé en ce que l'on isole un produit intermédiaire de formule VI

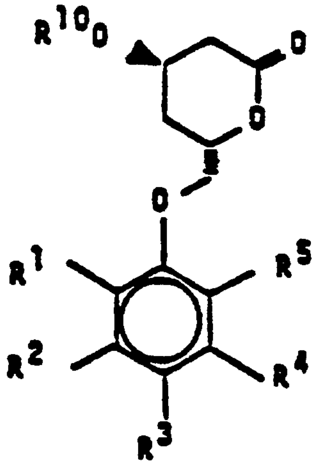

(VI)

dans laquelle $R^1$ à $R^5$ ont les significations données à propos de la formule I et $R^{10}$ a la signification donnée à propos de la formule III.